(19) Europäisches Patentamt
European Patent Office
Office européen des brevets

(11) EP 4 759 930 A1

(12) EUROPEAN PATENT APPLICATION
published in accordance with Art. 153(4) EPC

(43) Date of publication:
**17.06.2026 Bulletin 2026/25**

(21) Application number: **24851089.3**

(22) Date of filing: **08.08.2024**

(51) International Patent Classification (IPC):
***C12N 15/113*** (2010.01) ***A61K 31/7088*** (2006.01)
***A61P 1/16*** (2006.01) ***A61P 3/00*** (2006.01)

(52) Cooperative Patent Classification (CPC):
**A61K 31/7088; A61K 31/713; A61P 1/16; A61P 3/00; A61P 3/04; A61P 3/06; C12N 15/113**

(86) International application number:
**PCT/CN2024/110631**

(87) International publication number:
**WO 2025/031444 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.08.2023 CN 202310998379**
**04.02.2024 CN 202410160134**

(71) Applicant: **BEIJING FOYOU PHARMA CO., LTD**
**Beijing 101113 (CN)**

(72) Inventors:
- **LIN, Guoliang**
  **Beijing 101113 (CN)**
- **WANG, Shucheng**
  **Beijing 101113 (CN)**
- **HUANG, He**
  **Beijing 101113 (CN)**
- **WANG, Yan**
  **Beijing 101113 (CN)**
- **WANG, Xiaojun**
  **Beijing 101113 (CN)**
- **CHAN, Yunxia**
  **Beijing 101113 (CN)**
- **GENG, Yuxian**
  **Beijing 101113 (CN)**
- **CHE, Lingyu**
  **Beijing 101113 (CN)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

# (54) SIRNA FOR INHIBITING DGAT2 GENE EXPRESSION, SIRNA CONJUGATE OR PRODRUG AND PHARMACEUTICAL COMPOSITION THEREOF, AND USE THEREOF

(57) Provided are an siRNA for inhibiting DGAT2 gene expression, an siRNA conjugate and a prodrug thereof, a pharmaceutical composition comprising same, and the use thereof. Each nucleotide in the siRNA is independently a modified or unmodified nucleotide, and the siRNA comprises a sense strand and an antisense strand. The siRNA, the conjugate and the prodrug and pharmaceutical composition thereof can effectively treat and/or prevent diseases associated with DGAT2 gene overexpression.

EP 4 759 930 A1

## Description

### TECHNICAL FIELD

The present disclosure relates to an siRNA for inhibiting DGAT2 gene expression, an siRNA conjugate or a prodrug thereof, a pharmaceutical composition comprising same, a preparation method therefor, and use thereof.

### BACKGROUND

Diacylglycerol acyltransferase (or O-acyltransferase) DGAT catalyzes the formation of triglyceride from diacylglycerol and acyl-coenzyme A (CoA). The reaction catalyzed by DGAT is considered to be the final and only key step in triglyceride synthesis. In mammals, the DGAT enzymes have two isozymes: DGAT1 and DGAT2. Studies have shown that although DGAT1 and DGAT2 catalyze the same reaction, they share no gene sequence homology and are structurally unrelated. These two enzymes play different roles in triglyceride metabolism, wherein DGAT2 is involved in steatosis, while DGAT1 is involved in very-low-density lipoprotein (VLDL) synthesis. DGAT1 is mainly expressed in the small intestine, located in absorptive enterocytes of the intestine and duodenum, where it recombines triglycerides that have been broken down by lipolysis during intestinal absorption. DGAT1 recombines triglycerides in a defined step, after which they are packaged together with cholesterol and proteins to form chylomicrons. DGAT2 is mainly located in fat, liver, and skin cells, promoting triglyceride accumulation and fat formation. In humans, DGAT1 mutations are associated with congenital diarrhea. The exact cause of the diarrhea is unclear and is hypothesized to be related to abnormal fat absorption in the intestinal mucosa and accumulation of DGAT1 substrates. DGAT2, on the other hand, is crucial for adipose tissue formation and is closely related to plasma triglyceride levels, skin barrier formation, and fat accumulation in the liver. Since DGAT1 and DGAT2 share no sequence homology and are structurally unrelated, targeting DGAT2 does not affect DGAT1 function inhibition but can effectively reduce blood lipids and fat accumulation in the liver, making DGAT2 a potential target for treating related diseases such as hyperlipidemia, fatty liver, obesity, and hepatic steatosis.

Non-alcoholic fatty liver disease (NAFLD), also known as metabolic (dysfunction)-associated fatty liver disease (MAFLD), refers to excessive fat accumulation in the liver in the absence of other clear causes (such as alcohol consumption). NAFLD is the most common liver disease in the world, affecting about 25% of the world's population. NAFLD can be divided into two types: non-alcoholic fatty liver (NAFL) and non-alcoholic steatohepatitis (NASH). NAFL carries a lower risk than NASH and generally does not progress to NASH or cirrhosis; when NAFL progresses to NASH, injuries such as lobular inflammation, hepatocyte ballooning, and perisinusoidal fibrosis are observed. Compared with simple steatosis, inflammation is a hallmark of NASH. NASH is a progressive disease that increases hepatocyte death through apoptosis or necrosis, can severely impair liver function, and leads to complications such as cirrhosis, liver failure, liver cancer, or cardiovascular diseases, and has now become one of the top three factors for clinical liver transplantation. There are currently no specific treatments for NAFLD; it is primarily managed through dietary changes and exercise for weight loss, with preliminary studies suggesting the potential of pioglitazone and vitamin E for treatment.

Progressing from NAFL (manifesting as simple steatosis) to NASH, these diseases begin with abnormal fat accumulation in the liver (hepatic steatosis), where fat mainly exists in the form of triglycerides; however, the mechanism of triglyceride accumulation and the reason why accumulation leads to liver dysfunction remain unclear.

Studies have shown that overexpression of DGAT2 increases triglyceride synthesis, while reducing DGAT2 expression levels decreases triglyceride formation. In current studies, NEETA B. AMIN et al. used a DGAT2 inhibitor (PF-06427878), which could significantly reduce triglyceride concentrations in the liver and plasma and decrease the expression of lipogenic genes. In an NASH mouse model, PF-06427878 treatment could significantly ameliorate hepatic steatosis and fibrosis; in two phase 1 clinical trials, it could improve liver function markers such as alanine aminotransferase and reduce hepatic steatosis (Amin N B, Carvajal-Gonzalez S, Purkal J, et al., Targeting diacylglycerol acyltransferase 2 for the treatment of nonalcoholic steatohepatitis [J]. Sci. Transl. Med., 2019, 11(520): eaav9701). A drug named IONIS-DGAT2 Rx developed by Ionis Pharmaceuticals for treating NASH works precisely by inhibiting DGAT2 gene expression using antisense oligonucleotide (Prl A, Emb B, Lw B, et al., Novel antisense inhibition of diacylglycerol O-acyltransferase 2 for treatment of non-alcoholic fatty liver disease: a multicentre, double-blind, randomised, placebo-controlled phase 2 trial [J]. 2020.). A study by Batuhan Yenilmez et al. showed that in an NASH mouse model, using GalNAc-conjugated siRNA targeting DGAT2 could prevent and reverse triglyceride accumulation without increasing diacylglycerol accumulation, thereby significantly improving the fatty liver phenotype (Batuhan Yenilmez et al., An RNAi therapeutic targeting hepatic DGAT2 in a genetically obese mouse model of nonalcoholic steatohepatitis, Mol Ther. 2022 Mar 2; 30(3):1329-1342). Xing Xian Yu et al. investigated the role of DGAT2 in glucose and lipid metabolism using antisense oligonucleotides. The results indicated that reducing DGAT2 expression in the liver and fat of obese mice could significantly reduce hepatic triglyceride content in both models, ameliorate hepatic steatosis, alleviate hyperlipidemia, and thereby ameliorate metabolic syndrome (Xing Xian Yu et al., Antisense oligonucleotide reduction of DGAT2 expression improves hepatic steatosis and hyperlipidemia in bbese mice, Hepatology. 2005 Aug; 42(2):362-71). Brandon Pabst et al. used PF-06424439(1) and

2 as DGAT2 inhibitors to investigate the detailed mechanism of DGAT2 inhibition, further demonstrating that DGAT2 is an attractive therapeutic target for treating hyperlipidemia and hepatic steatosis (Brandon Pabst et al., Mechanistic characterization of long residence time inhibitors of diacylglycerol acyltransferase 2 (DGAT2), Biochemistry, 2018, 57, 51, 6997-7010). Tinglu Ning et al. conducted DGAT2 mutation identification in 227 young obese subjects and 219 lean controls; the results showed that the R128* mutation severely impaired the TG biosynthesis capacity of DGAT2, and all other R128* carriers in the pedigree were lean, thereby indicating an association between DGAT2 gene mutation and human obesity (Tinglu Ning et al., Genetic interaction of DGAT2 and FAAH in the development of human obesity, Endocrine, Volume 56, pages 366-378, (2017)). The above studies indicate that DGAT2 is an effective target for treating NAFLD/NASH, obesity, hepatic steatosis, hyperlipidemia, etc., and inhibiting DGAT2 can effectively reduce triglycerides and lower fat accumulation in the liver.

## SUMMARY

The present disclosure aims to provide an siRNA, an siRNA conjugate or a prodrug thereof, and a pharmaceutical composition, which can selectively and effectively inhibit DGAT2 gene expression and achieve the purpose of treating diseases.

In a first aspect, the present disclosure provides an siRNA for inhibiting DGAT2 gene expression, and the siRNA comprises a sense strand and an antisense strand, wherein each nucleotide in the siRNA is independently a modified or unmodified nucleotide; the sense strand comprises a nucleotide sequence I, the antisense strand comprises a nucleotide sequence II, and the nucleotide sequence I is at least partially reversely complementary to the nucleotide sequence II to form a double-stranded region, wherein the nucleotide sequence I and the nucleotide sequence II are selected from the following sequences:

(1) the nucleotide sequence I comprising the nucleotide sequence shown in SEQ ID NO: 346, and the nucleotide sequence II comprising the nucleotide sequence shown in SEQ ID NO: 347:

5’-GACUAUUUGCUUU-3’ (SEQ ID NO: 346)
5’-AAAGCAAAUAGUC-3’ (SEQ ID NO: 347);

(2) the nucleotide sequence I comprising the nucleotide sequence shown in SEQ ID NO: 348, and the nucleotide sequence II comprising the nucleotide sequence shown in SEQ ID NO: 349:

5’-CCAUCCUCAUGUACAUAU-3’ (SEQ ID NO: 348)
5’-AUAUGUACAUGAGGAUGG-3’ (SEQ ID NO: 349);

(3) the nucleotide sequence I comprising the nucleotide sequence shown in SEQ ID NO: 350, and the nucleotide sequence II comprising the nucleotide sequence shown in SEQ ID NO: 351:

5’-CACCAUAGACUAUU-3’ (SEQ ID NO: 350)
5’-AAUAGUCUAUGGUG-3’ (SEQ ID NO: 351);

(4) the nucleotide sequence I comprising the nucleotide sequence shown in SEQ ID NO: 352, and the nucleotide sequence II comprising the nucleotide sequence shown in SEQ ID NO: 353:

5’-UUUGCUUUCAAAGAA-3’ (SEQ ID NO: 352)
5’-UUCUUUGAAAGCAAAUA-3’ (SEQ ID NO: 353);

(5) the nucleotide sequence I comprising the nucleotide sequence shown in SEQ ID NO: 354, and the nucleotide sequence II comprising the nucleotide sequence shown in SEQ ID NO: 355:

5’-CUUUCGAGACUA-3’ (SEQ ID NO: 354)
5’-UAGUCUCGAAAGUA-3’ (SEQ ID NO: 355);

(6) the nucleotide sequence I comprising the nucleotide sequence shown in SEQ ID NO: 356, and the nucleotide sequence II comprising the nucleotide sequence shown in SEQ ID NO: 357:

5’-CUCAUGUACAUA-3’ (SEQ ID NO: 356)
5’-UAUGUACAUGAG-3’ (SEQ ID NO: 357);

(7) the nucleotide sequence I comprising the nucleotide sequence shown in SEQ ID NO: 358, and the nucleotide sequence II comprising the nucleotide sequence shown in SEQ ID NO: 359:

5'-GUGGCGCUACUUUCGAGA-3' (SEQ ID NO: 358)
5'-UCUCGAAAGUAGCGCCAC-3' (SEQ ID NO: 359).

In one embodiment, the nucleotide sequence I is substantially reversely complementary, virtually reversely complementary, or completely reversely complementary to the nucleotide sequence II; the substantially reversely complementary means that there are no more than 3 base mismatches between the two nucleotide sequences; the virtually reversely complementary means that there is no more than 1 base mismatch between the two nucleotide sequences; the completely reversely complementary means that there is no mismatch between the two nucleotide sequences.

In one embodiment, the sense strand further comprises a nucleotide sequence III, and the antisense strand further comprises a nucleotide sequence IV, wherein the nucleotide sequence III and the nucleotide sequence IV each independently have a length of 0-11 nucleotides; the nucleotide sequence III is linked to the 5' end of the nucleotide sequence I, the nucleotide sequence IV is linked to the 3' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences; and/or the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences.

In one embodiment, the siRNA comprises a sense strand and an antisense strand, wherein each nucleotide in the siRNA is independently a modified or unmodified nucleotide; the sense strand comprises nucleotide sequences I and III, the antisense strand comprises nucleotide sequences II and IV, and the nucleotide sequences I and III are at least partially reversely complementary to the nucleotide sequences II and IV to form a double-stranded region, wherein the nucleotide sequences I and III and the nucleotide sequences II and IV are selected from the following sequences:

(1) the nucleotide sequences I and III comprising or consisting of the nucleotide sequence shown in SEQ ID NO: 251, and the nucleotide sequences II and IV comprising or consisting of the nucleotide sequence shown in SEQ ID NO: 252;
(2) the nucleotide sequences I and III comprising or consisting of the nucleotide sequence shown in SEQ ID NO: 253, and the nucleotide sequences II and IV comprising or consisting of the nucleotide sequence shown in SEQ ID NO: 254;
(3) the nucleotide sequences I and III comprising or consisting of the nucleotide sequence shown in SEQ ID NO: 42, and the nucleotide sequences II and IV comprising or consisting of the nucleotide sequence shown in SEQ ID NO: 128;
(4) the nucleotide sequences I and III comprising or consisting of the nucleotide sequence shown in SEQ ID NO: 23, and the nucleotide sequences II and IV comprising or consisting of the nucleotide sequence shown in SEQ ID NO: 24;
(5) the nucleotide sequences I and III comprising or consisting of the nucleotide sequence shown in SEQ ID NO: 31, and the nucleotide sequences II and IV comprising or consisting of the nucleotide sequence shown in SEQ ID NO: 32;
(6) the nucleotide sequences I and III comprising or consisting of the nucleotide sequence shown in SEQ ID NO: 47, and the nucleotide sequences II and IV comprising or consisting of the nucleotide sequence shown in SEQ ID NO: 48;
(7) the nucleotide sequences I and III comprising or consisting of the nucleotide sequence shown in SEQ ID NO: 255, and the nucleotide sequences II and IV comprising or consisting of the nucleotide sequence shown in SEQ ID NO: 256;
(8) the nucleotide sequences I and III comprising or consisting of the nucleotide sequence shown in SEQ ID NO: 49, and the nucleotide sequences II and IV comprising or consisting of the nucleotide sequence shown in SEQ ID NO: 50;
(9) the nucleotide sequences I and III comprising or consisting of the nucleotide sequence shown in SEQ ID NO: 55, and the nucleotide sequences II and IV comprising or consisting of the nucleotide sequence shown in SEQ ID NO: 56;
(10) the nucleotide sequences I and III comprising or consisting of the nucleotide sequence shown in SEQ ID NO: 257, and the nucleotide sequences II and IV comprising or consisting of the nucleotide sequence shown in SEQ ID NO: 258;
(11) the nucleotide sequences I and III comprising or consisting of the nucleotide sequence shown in SEQ ID NO: 57, and the nucleotide sequences II and IV comprising or consisting of the nucleotide sequence shown in SEQ ID NO: 58;

(12) the nucleotide sequences I and III comprising or consisting of the nucleotide sequence shown in SEQ ID NO: 59, and the nucleotide sequences II and IV comprising or consisting of the nucleotide sequence shown in SEQ ID NO: 60;
(13) the nucleotide sequences I and III comprising or consisting of the nucleotide sequence shown in SEQ ID NO: 61, and the nucleotide sequences II and IV comprising or consisting of the nucleotide sequence shown in SEQ ID NO: 62;
(14) the nucleotide sequences I and III comprising or consisting of the nucleotide sequence shown in SEQ ID NO: 259, and the nucleotide sequences II and IV comprising or consisting of the nucleotide sequence shown in SEQ ID NO: 260;
(15) the nucleotide sequences I and III comprising or consisting of the nucleotide sequence shown in SEQ ID NO: 261, and the nucleotide sequences II and IV comprising or consisting of the nucleotide sequence shown in SEQ ID NO: 262;
(16) the nucleotide sequences I and III comprising or consisting of the nucleotide sequence shown in SEQ ID NO: 75, and the nucleotide sequences II and IV comprising or consisting of the nucleotide sequence shown in SEQ ID NO: 76;
(17) the nucleotide sequences I and III comprising or consisting of the nucleotide sequence shown in SEQ ID NO: 263, and the nucleotide sequences II and IV comprising or consisting of the nucleotide sequence shown in SEQ ID NO: 264;
(18) the nucleotide sequences I and III comprising or consisting of the nucleotide sequence shown in SEQ ID NO: 265, and the nucleotide sequences II and IV comprising or consisting of the nucleotide sequence shown in SEQ ID NO: 266;
(19) the nucleotide sequences I and III comprising or consisting of the nucleotide sequence shown in SEQ ID NO: 267, and the nucleotide sequences II and IV comprising or consisting of the nucleotide sequence shown in SEQ ID NO: 268;
(20) the nucleotide sequences I and III comprising or consisting of the nucleotide sequence shown in SEQ ID NO: 269, and the nucleotide sequences II and IV comprising or consisting of the nucleotide sequence shown in SEQ ID NO: 270;
(21) the nucleotide sequences I and III comprising or consisting of the nucleotide sequence shown in SEQ ID NO: 15, and the nucleotide sequences II and IV comprising or consisting of the nucleotide sequence shown in SEQ ID NO: 271;
(22) the nucleotide sequences I and III comprising or consisting of the nucleotide sequence shown in SEQ ID NO: 272, and the nucleotide sequences II and IV comprising or consisting of the nucleotide sequence shown in SEQ ID NO: 273;
(23) the nucleotide sequences I and III comprising or consisting of the nucleotide sequence shown in SEQ ID NO: 131, and the nucleotide sequences II and IV comprising or consisting of the nucleotide sequence shown in SEQ ID NO: 132;
(24) the nucleotide sequences I and III comprising or consisting of the nucleotide sequence shown in SEQ ID NO: 274, and the nucleotide sequences II and IV comprising or consisting of the nucleotide sequence shown in SEQ ID NO: 275;
(25) the nucleotide sequences I and III comprising or consisting of the nucleotide sequence shown in SEQ ID NO: 276, and the nucleotide sequences II and IV comprising or consisting of the nucleotide sequence shown in SEQ ID NO: 277;
(26) the nucleotide sequences I and III comprising or consisting of the nucleotide sequence shown in SEQ ID NO: 163, and the nucleotide sequences II and IV comprising or consisting of the nucleotide sequence shown in SEQ ID NO: 164;
(27) the nucleotide sequences I and III comprising or consisting of the nucleotide sequence shown in SEQ ID NO: 165, and the nucleotide sequences II and IV comprising or consisting of the nucleotide sequence shown in SEQ ID NO: 166; or
(28) the nucleotide sequences I and III comprising or consisting of the nucleotide sequence shown in SEQ ID NO: 278, and the nucleotide sequences II and IV comprising or consisting of the nucleotide sequence shown in SEQ ID NO: 279.

In one embodiment, the sense strand further comprises a nucleotide sequence V, and/or the antisense strand further comprises a nucleotide sequence VI, wherein the nucleotide sequences V and VI have a length of 0-3 nucleotides; the nucleotide sequence V is linked to the 3' end of the sense strand to form the 3' overhang of the sense strand, and/or the nucleotide sequence VI is linked to the 3' end of the antisense strand to form the 3' overhang of the antisense strand. In one preferred embodiment, the nucleotide sequence V or VI has a length of 2 nucleotides. In one preferred embodiment, the nucleotide sequence V or VI is two consecutive thymine deoxyribonucleotides, two consecutive uracil ribonucleotides, or one cytosine deoxyribonucleotide and one adenine deoxyribonucleotide in the 5' to 3' direction. In one preferred embodiment, the nucleotide sequence V or VI is mismatched or complementary to a nucleotide at a corresponding position of a target mRNA.

In one embodiment, the double-stranded region has a length of 15-30 nucleotide pairs; preferably, the double-stranded region has a length of 17-23 nucleotide pairs; more preferably, the double-stranded region has a length of 19-21 nucleotide pairs.

In one embodiment, the sense strand or the antisense strand has 15-30 nucleotides; preferably, the sense strand or the antisense strand has 19-25 nucleotides; more preferably, the sense strand or the antisense strand has 19-23 nucleotides. In one embodiment, at least one nucleotide in the sense strand or the antisense strand is a modified nucleotide, and/or at least one phosphoester group is a phosphoester group with a modification group; preferably, the phosphoester group with a modification group is a phosphorothioate group formed by substituting one oxygen atom in a phosphodiester bond of the phosphoester group with a sulfur atom.

In one embodiment, the siRNA comprises a sense strand without a 3' overhang nucleotide.

In one embodiment, the 5' end nucleotide of the antisense strand is linked to a 5' phosphate group or a 5' phosphate-derived group, or, the 5' end nucleotide of the antisense strand is not linked to a 5' phosphate group or a 5' phosphate-derived group.

In one embodiment, the modified nucleotide is selected from a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, a 2'-substituted amino-modified nucleotide, a nucleotide analog, and a combination of any two or more thereof.

In one embodiment, the modified nucleotide is selected from a 2'-fluoro-modified nucleotide, a 2'-methoxy-modified nucleotide, a 2'-O-$CH_2$-$CH_2$-O-$CH_3$ modified nucleotide, a 2'-O-$CH_2$-CH=$CH_2$ modified nucleotide, a 2'-$CH_2$-$CH_2$-CH=$CH_2$ modified nucleotide, a 2'-deoxynucleotide, a nucleotide analog, and a combination of any two or more thereof.

In one embodiment, each nucleotide in the sense strand and the antisense strand is independently a 2'-fluoro-modified nucleotide or a non-fluoro-modified nucleotide.

In one preferred embodiment, in the sense strand, in the 5' to 3' direction, the 2'-fluoro-modified nucleotides are located at positions 7, 9, 10, and 11, and the remaining positions are the non-fluoro-modified nucleotides; or, the 2'-fluoro-modified nucleotides are located at positions 5, 7, 8, and 9, and the remaining positions are the non-fluoro-modified nucleotides; or, the 2'-fluoro-modified nucleotides are located at positions 9, 10, and 11, and the remaining positions are the non-fluoro-modified nucleotides; or, the 2'-fluoro-modified nucleotides are located at positions 8, 9, and 10, and the remaining positions are the non-fluoro-modified nucleotides; or, the 2'-fluoro-modified nucleotides are located at positions 8, 9, 10, and 11, and the remaining positions are the non-fluoro-modified nucleotides; or, the 2'-fluoro-modified nucleotides are located at positions 7 and 9, and the remaining positions are the non-fluoro-modified nucleotides; or, the 2'-fluoro-modified nucleotides are located at positions 7, 9, and 11, and the remaining positions are the non-fluoro-modified nucleotides; or, the 2'-fluoro-modified nucleotides are located at positions 7, 11, 13, and 15, and the remaining positions are the non-fluoro-modified nucleotides; or, the 2'-fluoro-modified nucleotides are located at positions 8, 9, 10, and 12, and the remaining positions are the non-fluoro-modified nucleotides; or, the 2'-fluoro-modified nucleotides are located at positions 7, 9, 11, 13, and 15, and the remaining positions are the non-fluoro-modified nucleotides; or, the 2'-fluoro-modified nucleotides are located at positions 9, 11, and 13, and the remaining positions are the non-fluoro-modified nucleotides; or, the 2'-fluoro-modified nucleotides are located at positions 7, 9, 11, and 13, and the remaining positions are the non-fluoro-modified nucleotides; or, the 2'-fluoro-modified nucleotides are located at positions 3, 7, 8, and 9, and the remaining positions are the non-fluoro-modified nucleotides; or, the 2'-fluoro-modified nucleotides are located at positions 3, 9, 11, and 13, and the remaining positions are the non-fluoro-modified nucleotides; or, the 2'-fluoro-modified nucleotides are located at positions 3, 7, 9, and 11, and the remaining positions are the non-fluoro-modified nucleotides; or, the 2'-fluoro-modified nucleotides are located at positions 7, 11, and 13, and the remaining positions are the non-fluoro-modified nucleotides; or, the 2'-fluoro-modified nucleotides are located at positions 7, 11, 12, and 13, and the remaining positions are the non-fluoro-modified nucleotides; or, the 2'-fluoro-modified nucleotides are located at positions 3, 7, 11, and 16, and the remaining positions are the non-fluoro-modified nucleotides; or, the 2'-fluoro-modified nucleotides are located at positions 3, 7, 11, and 17, and the remaining positions are the non-fluoro-modified nucleotides; or, the 2'-fluoro-modified nucleotides are located at positions 3, 7, 11, 16, and 17, and the remaining positions are the non-fluoro-modified nucleotides; and/or

in the antisense strand, in the 5' to 3' direction, the 2'-fluoro-modified nucleotides are located at positions 2, 6, 14, and 16, and the remaining positions are the non-fluoro-modified nucleotides; or, the 2'-fluoro-modified nucleotides are located at positions 2, 6, 8, 9, 14, and 16, and the remaining positions are the non-fluoro-modified nucleotides; or, the 2'-fluoro-modified nucleotides are located at positions 2, 14, and 16, and the remaining positions are the non-fluoro-modified nucleotides; or, the 2'-fluoro-modified nucleotides are located at positions 2, 6, and 14, and the remaining positions are the non-fluoro-modified nucleotides; or, the 2'-fluoro-modified nucleotides are located at positions 2, 3, 5, 8, 10, 14, 16, and 18, and the remaining positions are the non-fluoro-modified nucleotides; or, the 2'-fluoro-modified nucleotides are located at positions 2, 3, 4, 5, 7, 10, and 14, and the remaining positions are the non-fluoro-modified nucleotides; or, the 2'-fluoro-modified nucleotides are located at positions 2, 4, 6, 12, 14, 16, 18, and 20, and the remaining positions are the non-fluoro-modified nucleotides; or, the 2'-fluoro-modified nucleotides are located at positions 2 and 14, and the remaining positions are the non-fluoro-modified nucleotides; or, the 2'-fluoro-modified nucleotides are located at positions 2, 4, 5, 6, 8, 10, 12, 14, 16, 18, and 20, and the remaining positions are the non-fluoro-modified nucleotides; or, the 2'-fluoro-modified nucleotides are located at positions 2, 7, 10, and 14, and the remaining positions are the non-fluoro-modified nucleotides; or, the 2'-fluoro-modified nucleotides are located at positions 2, 5, 7, and 14, and the remaining positions are the non-fluoro-modified nucleotides.

In one preferred embodiment, in the 5' to 3' direction, the 2'-fluoro-modified nucleotides are located at positions 7, 9, 10, and 11 of the sense strand, and the remaining positions are the non-fluoro-modified nucleotides; in the 5' to 3' direction, the 2'-fluoro-modified nucleotides are located at positions 2, 6, 14, and 16 of the antisense strand, and the remaining positions are the non-fluoro-modified nucleotides. In one preferred embodiment, in the 5' to 3' direction, the 2'-fluoro-modified nucleotides are located at positions 7, 9, 10, and 11 of the sense strand, and the remaining positions are the non-fluoro-modified nucleotides; in the 5' to 3' direction, the 2'-fluoro-modified nucleotides are located at positions 2, 6, 8, 9, 14, and 16 of the antisense strand, and the remaining positions are the non-fluoro-modified nucleotides. In one preferred embodiment, in the 5' to 3' direction, the 2'-fluoro-modified nucleotides are located at positions 7, 9, 10, and 11 of the sense strand, and the remaining positions are the non-fluoro-modified nucleotides; in the 5' to 3' direction, the 2'-fluoro-modified nucleotides are located at positions 2, 14, and 16 of the antisense strand, and the remaining positions are the non-fluoro-modified nucleotides.

In one embodiment, each of the non-fluoro-modified nucleotides is a 2'-methoxy-modified nucleotide, and the 2'-methoxy-modified nucleotide refers to a nucleotide formed by substituting 2'-hydroxyl of a ribosyl group with methoxy.

In one embodiment, each of the non-fluoro-modified nucleotides is independently selected from one of a nucleotide formed by substituting hydroxyl at the 2' position of a ribosyl group of the nucleotide with a non-fluorine group and a nucleotide analog, and the nucleotide analog is selected from one of isonucleotide, LNA, ENA, cET-BNA, UNA, and GNA.

In one embodiment, any one nucleotide in the sense strand and the antisense strand is independently a 2'-fluoro-modified nucleotide, a 2'-methoxy-modified nucleotide, a GNA-modified nucleotide, or a combination of any two or more thereof.

In one preferred embodiment, in the sense strand, in the 5' to 3' direction, the 2'-fluoro-modified nucleotides are located at positions 7, 9, 10, and 11, and the remaining positions are the 2'-methoxy-modified nucleotides; or, the 2'-fluoro-modified nucleotides are located at positions 5, 7, 8, and 9, and the remaining positions are the 2'-methoxy-modified nucleotides; or, the 2'-fluoro-modified nucleotides are located at positions 9, 10, and 11, and the remaining positions are the 2'-methoxy-modified nucleotides; or, the 2'-fluoro-modified nucleotides are located at positions 8, 9, and 10, and the remaining positions are the 2'-methoxy-modified nucleotides; or, the 2'-fluoro-modified nucleotides are located at positions 8, 9, 10, and 11, and the remaining positions are the 2'-methoxy-modified nucleotides; or, the 2'-fluoro-modified nucleotides are located at positions 7 and 9, position 11 is a 2'-deoxynucleotide, and the remaining positions are the 2'-methoxy-modified nucleotides; or, the 2'-fluoro-modified nucleotides are located at positions 7, 9, and 11, and the remaining positions are the 2'-methoxy-modified nucleotides; or, the 2'-fluoro-modified nucleotides are located at positions 7, 11, 13, and 15, and the remaining positions are the 2'-methoxy-modified nucleotides; or, the 2'-fluoro-modified nucleotides are located at positions 8, 9, 10, and 12, and the remaining positions are the 2'-methoxy-modified nucleotides; or, the 2'-fluoro-modified nucleotides are located at positions 7, 9, 11, 13, and 15, and the remaining positions are the 2'-methoxy-modified nucleotides; or, the 2'-fluoro-modified nucleotides are located at positions 9, 11, and 13, and the remaining positions are the 2'-methoxy-modified nucleotides; or, the 2'-fluoro-modified nucleotides are located at positions 7, 9, 11, and 13, and the remaining positions are the 2'-methoxy-modified nucleotides; or, the 2'-fluoro-modified nucleotides are located at positions 3, 7, 8, and 9, and the remaining positions are the 2'-methoxy-modified nucleotides; or, the 2'-fluoro-modified nucleotides are located at positions 3, 9, 11, and 13, and the remaining positions are the 2'-methoxy-modified nucleotides; or, the 2'-fluoro-modified nucleotides are located at positions 3, 7, 9, and 11, and the remaining positions are the 2'-methoxy-modified nucleotides; or, the 2'-fluoro-modified nucleotides are located at positions 7, 11, and 13, and the remaining positions are the 2'-methoxy-modified nucleotides; or, the 2'-fluoro-modified nucleotides are located at positions 7, 11, 12, and 13, and the remaining positions are the 2'-methoxy-modified nucleotides; or, the 2'-fluoro-modified nucleotides are located at positions 3, 7, 11, and 16, and the remaining positions are the 2'-methoxy-modified nucleotides; or, the 2'-fluoro-modified nucleotides are located at positions 3, 7, 11, and 17, and the remaining positions are the 2'-methoxy-modified nucleotides; or, the 2'-fluoro-modified nucleotides are located at positions 3, 7, 11, 16, and 17, and the remaining positions are the 2'-methoxy-modified nucleotides; and/or

in the antisense strand, in the 5' to 3' direction, the 2'-fluoro-modified nucleotides are located at positions 2, 6, 14, and 16, and the remaining positions are the 2'-methoxy-modified nucleotides; or, the 2'-fluoro-modified nucleotides are located at positions 2, 6, 14, and 16, position 7 is the GNA-modified nucleotide, and the remaining positions are the 2'-methoxy-modified nucleotides; or, the 2'-fluoro-modified nucleotides are located at positions 2, 6, 8, 9, 14, and 16, and the remaining positions are the 2'-methoxy-modified nucleotides; or, the 2'-fluoro-modified nucleotides are located at positions 2, 14, and 16, and the remaining positions are the 2'-methoxy-modified nucleotides; or, the 2'-fluoro-modified nucleotides are located

at positions 2, 14, and 16, position 6 is the GNA-modified nucleotide, and the remaining positions are the 2'-methoxy-modified nucleotides; or, the 2'-fluoro-modified nucleotides are located at positions 2, 6, and 14, and the remaining positions are the 2'-methoxy-modified nucleotides; or, the 2'-fluoro-modified nucleotides are located at positions 2, 3, 5, 8, 10, 14, 16, and 18, and the remaining positions are the 2'-methoxy-modified nucleotides; or, the 2'-fluoro-modified nucleotides are located at positions 2, 3, 4, 5, 7, 10, and 14, and the remaining positions are the 2'-methoxy-modified nucleotides; or, the 2'-fluoro-modified nucleotides are located at positions 2, 3, 4, 5, 7, 10, and 14, position 6 is the GNA-modified nucleotide, and the remaining positions are the 2'-methoxy-modified nucleotides; or, the 2'-fluoro-modified nucleotides are located at positions 2, 4, 6, 12, 14, 16, 18, and 20, and the remaining positions are the 2'-methoxy-modified nucleotides; or, the 2'-fluoro-modified nucleotides are located at positions 2 and 14, and the remaining positions are the 2'-methoxy-modified nucleotides; or, the 2'-fluoro-modified nucleotides are located at positions 2, 4, 5, 6, 8, 10, 12, 14, 16, 18, and 20, and the remaining positions are the 2'-methoxy-modified nucleotides; or, the 2'-fluoro-modified nucleotides are located at positions 2, 7, 10, and 14, and the remaining positions are the 2'-methoxy-modified nucleotides; or, the 2'-fluoro-modified nucleotides are located at positions 2, 5, 7, and 14, and the remaining positions are the 2'-methoxy-modified nucleotides.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides. In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides. In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the GNA-modified nucleotide is located at position 7 of the antisense strand, and the remaining positions are the 2'-methoxy-modified nucleotides. In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 14, and 16 of the antisense strand, the GNA-modified nucleotide is located at position 6 of the antisense strand, and the remaining positions are the 2'-methoxy-modified nucleotides.

In some embodiments, at least one of the following linkages between nucleotides in the siRNA is a phosphorothioate group linkage:

a linkage between the first nucleotide and the second nucleotide from the 5' end of the sense strand;
a linkage between the second nucleotide and the third nucleotide from the 5' end of the sense strand;
a linkage between the first nucleotide and the second nucleotide from the 3' end of the sense strand;
a linkage between the second nucleotide and the third nucleotide from the 3' end of the sense strand;
a linkage between the first nucleotide and the second nucleotide from the 5' end of the antisense strand;
a linkage between the second nucleotide and the third nucleotide from the 5' end of the antisense strand;
a linkage between the first nucleotide and the second nucleotide from the 3' end of the antisense strand;
a linkage between the second nucleotide and the third nucleotide from the 3' end of the antisense strand.

In some embodiments, in the direction from the 5' end to the 3' end of the siRNA, the sense strand comprises phosphorothioate groups located at the positions shown below:

a position between the first nucleotide and the second nucleotide from the 5' end of the sense strand;
a position between the second nucleotide and the third nucleotide from the 5' end of the sense strand;
a position between the first nucleotide and the second nucleotide from the 3' end of the sense strand;
a position between the second nucleotide and the third nucleotide from the 3' end of the sense strand; or,
the sense strand comprises phosphorothioate groups located at the positions shown below:

a position between the first nucleotide and the second nucleotide from the 5' end of the sense strand;
a position between the second nucleotide and the third nucleotide from the 5' end of the sense strand.

In some embodiments, in the direction from the 5' end to the 3' end of the siRNA, the antisense strand comprises phosphorothioate groups located at the positions shown below:

a position between the first nucleotide and the second nucleotide from the 5' end of the antisense strand;
a position between the second nucleotide and the third nucleotide from the 5' end of the antisense strand;

a position between the first nucleotide and the second nucleotide from the 3' end of the antisense strand;
a position between the second nucleotide and the third nucleotide from the 3' end of the antisense strand.

In one embodiment, any one nucleotide in the sense strand and the antisense strand is independently a 2'-fluoro-modified nucleotide, a 2'-methoxy-modified nucleotide, a GNA-modified nucleotide, or a combination of any two or more thereof.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 8, 9, 14, and 16 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the GNA-modified nucleotide is located at position 7 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the GNA-modified nucleotide is located at position 7 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 14, and 16 of the antisense strand, the GNA-modified nucleotide is located at position 6 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 14, and 16 of the antisense strand, the GNA-modified nucleotide is located at position 6 of the antisense strand, and the remaining positions are the 2'-methoxy-modified nucleotides, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 8, 9, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, the remaining positions are the 2'-methoxy-modified nucleotides, and the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, and 14 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 5, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 8, 9, and 10 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 5, 8, 10, 14, 16, and 18 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 8, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 4, 6, 12, 14, 16, 18, and 20 of the antisense strand,

the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2 and 14 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7 and 9 of the sense strand, position 11 is a 2'-deoxynucleotide, and the remaining positions are the 2'-methoxy-modified nucleotides; in the 5' to 3' direction, the 2'-fluoro-modified nucleotides are located at positions 2, 4, 5, 6, 8, 10, 12, 14, 16, 18, and 20 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7 and 9 of the sense strand, position 11 is a 2'-deoxynucleotide, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 11, 13, and 15 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 8, 9, 10, and 12 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 11, 13, and 15 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 11, 13, and 15 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7,

9, 11, and 13 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 8, and 9 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 11, 12, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 11, and 16 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 11, and 17 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 11, 16, and 17 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, position 6 is the GNA-modified nucleotide, the remaining positions are the 2'-methoxy-modified nucleotides, and the 5' end

nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 5, 7, and 14 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 8, and 9 of the sense strand, and the remaining positions are the 2'-methoxy-modified nucleotides, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 9, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, and the 5' end nucleotide of the antisense strand is not linked to a 5' phosphate group or a 5' phosphate-derived group.

In one embodiment, any one nucleotide in the sense strand and the antisense strand is independently a 2'-fluoro-modified nucleotide, a 2'-methoxy-modified nucleotide, a GNA-modified nucleotide, or a combination of any two or more thereof.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 8, 9, 14, and 16 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 8, 9, 14, and 16 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the GNA-modified nucleotide is located at position 7 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the GNA-modified nucleotide is located at position 7 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 14, and 16 of the antisense strand, the GNA-modified nucleotide is located at position 6 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 14, and 16 of the antisense strand, the GNA-modified nucleotide is located at position 6 of the antisense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, with the 5' end nucleotide of the antisense strand linked to a 5' phosphate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate

group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 8, 9, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 8, 9, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 8, 9, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is linked to a 5'-(E)-vinylphosphonate group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 5, 7, 8, and 9 of the sense strand, the remaining positions are the 2'-methoxy-modified nucleotides, and phosphorothioate

group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 8, 9, and 10 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 3, 5, 8, 10, 14, 16, and 18 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 3, 4, 5, 7, 10, and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 8, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 3, 4, 5, 7, 10, and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 4, 6, 12, 14, 16, 18, and 20 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2 and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7 and 9 of the sense strand, position 11 is a 2'-deoxynucleotide, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 4, 5, 6, 8, 10, 12, 14, 16, 18, and 20 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7 and 9 of the sense strand, position 11 is a 2'-deoxynucleotide, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the

second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 11, 13, and 15 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 8, 9, 10, and 12 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 11, 13, and 15 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 11, 13, and 15 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 9, 11, and 13 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 11, and 13 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 11, and 13 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 8, and 9 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 8, and 9 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 9, 11, and 13 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 9, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 9, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the

second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 11, and 13 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 11, 12, and 13 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 11, and 16 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 11, and 17 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 11, 16, and 17 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified nucleotides are located at positions 2, 6, 14, and 16 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end; in the 5' to 3' direction, the 2'-fluoro-modified

nucleotides are located at positions 2, 3, 4, 5, 7, 10, and 14 of the antisense strand, position 6 is the GNA-modified nucleotide, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 7, 9, 10, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 5, 7, and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 8, and 9 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one preferred embodiment, in the 5' to 3' direction, the 2'-fluoro-modified nucleotides are located at positions 3, 7, 9, and 11 of the sense strand, the remaining positions are the 2'-methoxy-modified nucleotides, and phosphorothioate group linkages are present between the first nucleotide and the second nucleotide of the 5' end and between the second nucleotide and the third nucleotide of the 5' end, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, phosphorothioate group linkages are present between the first nucleotide and the second nucleotide of the 5' end, between the second nucleotide and the third nucleotide of the 5' end, between the first nucleotide and the second nucleotide of the 3' end, and between the second nucleotide and the third nucleotide of the 3' end, and the 5' end nucleotide of the antisense strand is not linked to a 5' phosphate group or a 5' phosphate-derived group.

In one specific embodiment, the present disclosure provides an siRNA selected from Table 1; preferably, the siRNA is selected from N-FY001003, N-FY001003M2, N-FY001003D2, N-FY001003M2D2, N-FY001003M3, N-FY001003M4, N-FY001003M5, N-FY001003M6, N-FY001003M6D2, N-FY001003M7, N-FY001003M7D2, N-FY001003M8, N-FY001003M9, N-FY001003M10, N-FY001003M11, N-FY001003M12, N-FY001003M13, N-FY001003M14, N-FY001003M15, N-FY001003M16, N-FY001003M17, N-FY001003M18, N-FY001003M19, N-FY001003M20, N-FY001003M21, N-FY001003M21D2, N-FY001003M22, N-FY001003M23, N-FY001003M24, N-FY001003M24D2, N-FY001003M25, N-FY001003M26, N-FY001003M26D2, N-FY001003M27, N-FY001003M27D2, N-FY001003M28, N-FY001003M29, N-FY001003M29D2, N-FY001003M30, N-FY001003M31, N-FY001003M32, N-FY001003M33, N-FY001003M34, N-FY001003M35, N-FY001003M37, N-FY001003M40, N-FY001003M44, and N-FY001003M45.

In a second aspect, the present disclosure also provides an siRNA conjugate or a prodrug thereof, and the siRNA conjugate comprises the siRNA of the present disclosure and a conjugated group conjugated to the siRNA (as shown in the following figure, the double helix structure represents the siRNA, and the conjugated group is linked to the 3' end of the sense strand of the siRNA):

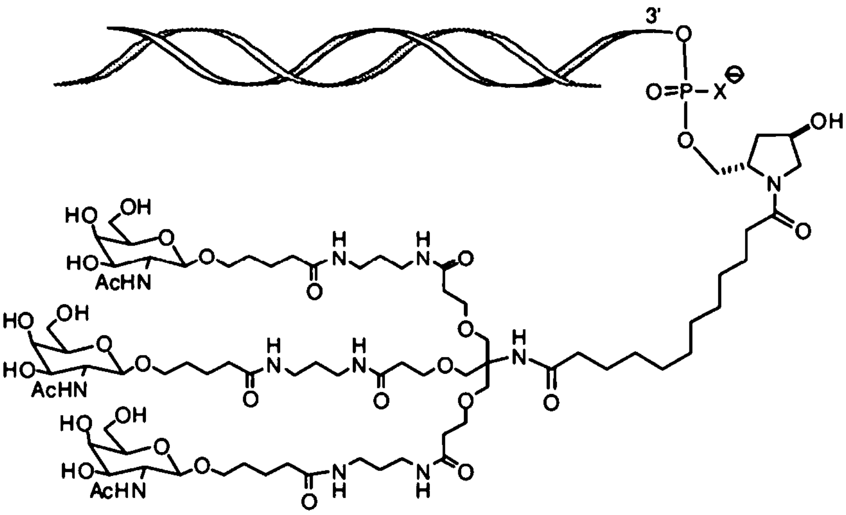

,

in the conjugate structure described above, X may optionally be O or S; in one embodiment, X is O.

In one embodiment, the conjugated group comprises a pharmaceutically acceptable targeting group and a linker, and the siRNA, the linker, and the targeting group are sequentially linked covalently or non-covalently.

preferably, in the siRNA conjugate, the sense strand and the antisense strand of the siRNA are complementary to form a double-stranded region of the siRNA conjugate, the 3' end of the sense strand forms a blunt end, and the 3' end of the antisense strand has 1-3 overhanging nucleotides extending out of the double-stranded region;

or,

in the siRNA conjugate, the sense strand and the antisense strand of the siRNA are complementary to form a double-stranded region of the siRNA conjugate, the 3' end of the sense strand forms a blunt end, and the 3' end of the antisense strand forms a blunt end.

In one embodiment, the conjugated group is selected from the groups consisting of formula (I) to formula (VIII):

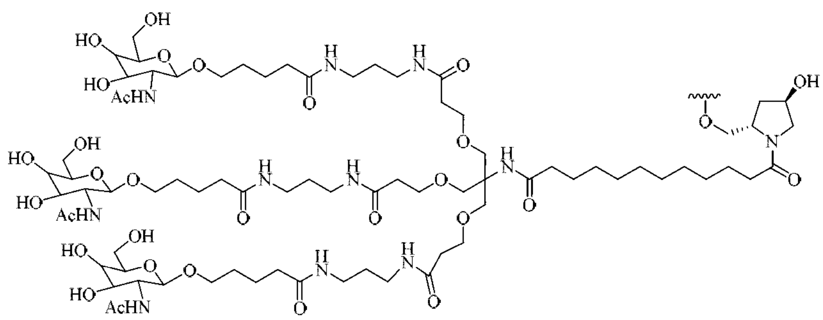

formula (I)

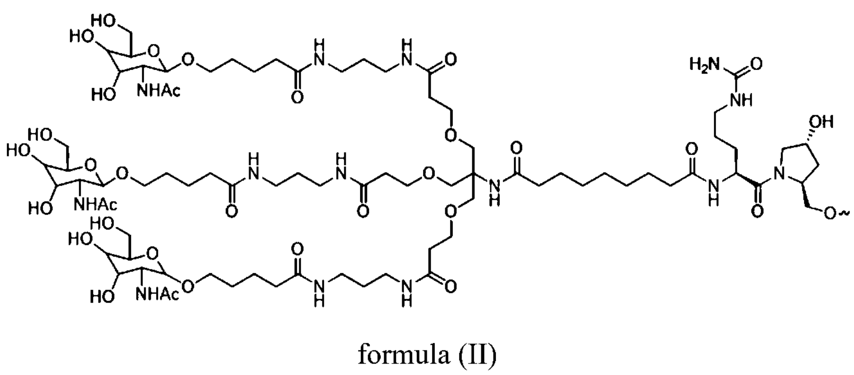

formula (II)

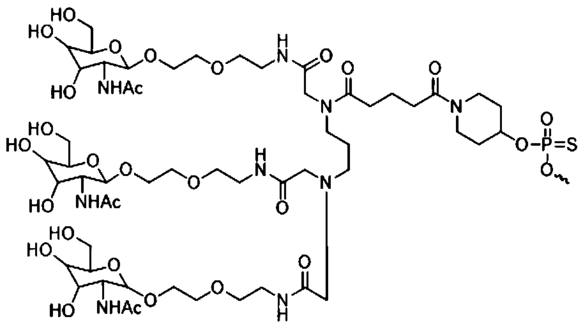

formula (III)

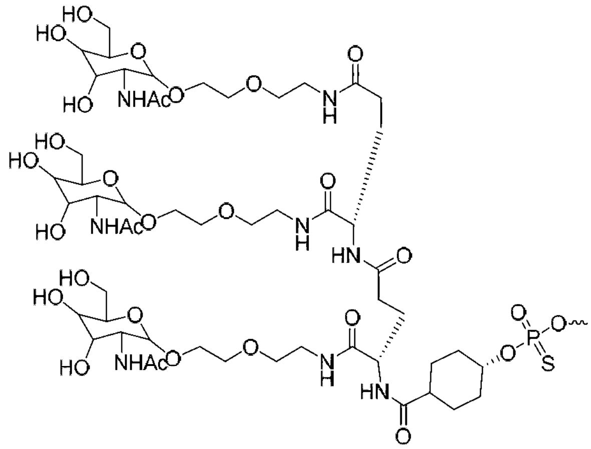

formula (IV)

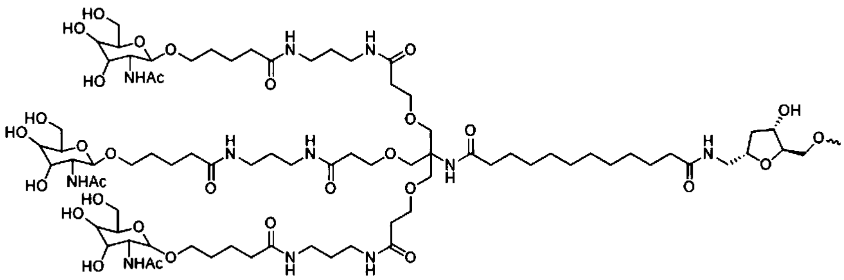

formula (V)

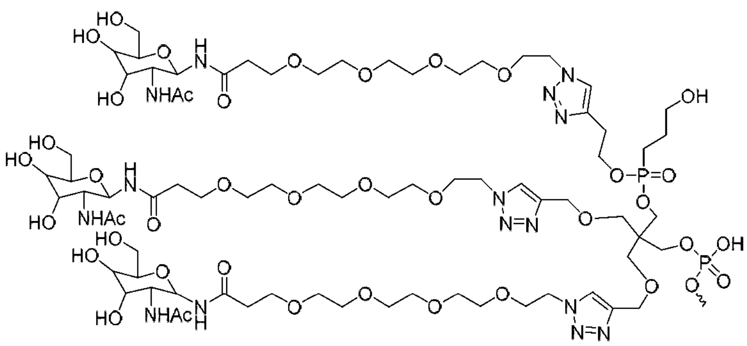

formula (VI)

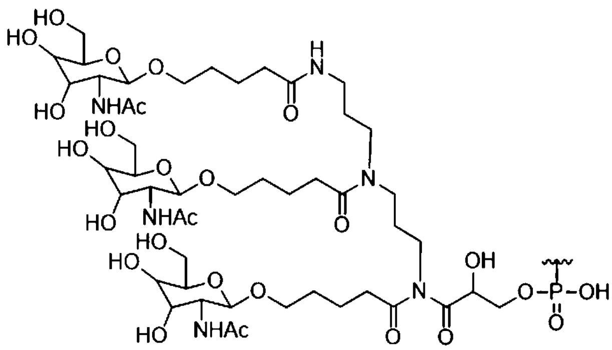

formula (VII)

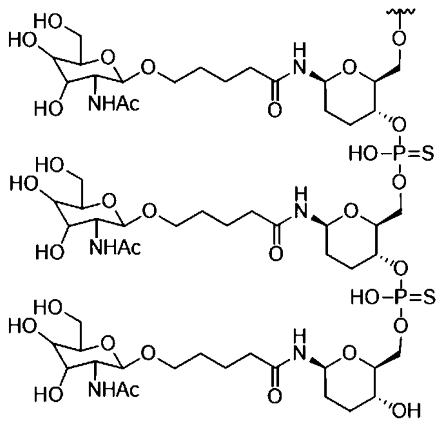

formula (VIII).

In one specific embodiment, the siRNA conjugate is an siRNA conjugate selected from Table 2; preferably, the siRNA conjugate is selected from N-FY001003M2L96, N-FY001003M3L96, N-FY001003M6L96, N-FY001003M7L96, N-FY001003M8L96, N-FY001003M9L96, N-FY001003M10L96, N-FY001003M11L96, N-FY001003M12L96, N-FY001003M13L96, N-FY001003M14L96, N-FY001003M15L96, N-FY001003M16L96, N-FY001003M17L96, N-FY001003M18L96, N-FY001003M19L96, N-FY001003M20L96, N-FY001003M21L96, N-FY001003M22L96, N-FY001003M23L96, N-FY001003M24L96, N-FY001003M25L96, N-FY001003M26L96, N-FY001003M27L96, N-FY001003M28L96, N-FY001003M29L96, N-FY001003M30L96, N-FY001003M31L96, N-FY001003M32L96, N-FY001003M33L96, N-FY001003M34L96, N-FY001003M35L96, N-FY001003M37L96, N-FY001003M40L96, N-FY001003M44L96, N-FY001003M45L96, and N-FY001003M6L96B-1.

In a third aspect, the present disclosure also provides a pharmaceutical composition, which comprises the siRNA of the present disclosure, or the siRNA conjugate or the prodrug thereof of the present disclosure, and a pharmaceutically acceptable carrier.

In a fourth aspect, the present disclosure also provides a kit, which comprises the siRNA of the present disclosure, or the siRNA conjugate or the prodrug thereof of the present disclosure, or the pharmaceutical composition of the present disclosure.

In a fifth aspect, the present disclosure also provides use of the siRNA of the present disclosure, or the siRNA conjugate or the prodrug thereof of the present disclosure, or the pharmaceutical composition of the present disclosure in preparing a medicament for inhibiting DGAT2 gene expression.

In a sixth aspect, the present disclosure also provides use of the siRNA of the present disclosure, or the siRNA conjugate or the prodrug thereof of the present disclosure, or the pharmaceutical composition of the present disclosure in preparing a medicament for preventing and/or treating a disease associated with DGAT2 gene overexpression.

In a specific embodiment, the disease is selected from the group consisting of: non-alcoholic fatty liver disease, non-alcoholic steatohepatitis, alcoholic fatty liver, obesity, hepatic steatosis, fatty liver, and hyperlipidemia.

In a seventh aspect, the present disclosure also provides a method for inhibiting DGAT2 gene expression, which comprises contacting a therapeutically effective amount of the siRNA of the present disclosure, or the siRNA conjugate or the prodrug thereof of the present disclosure, or the pharmaceutical composition of the present disclosure with a cell

expressing DGAT2 or administering to a subject in need a therapeutically effective amount of the siRNA of the present disclosure, or the siRNA conjugate or the prodrug thereof of the present disclosure, or the pharmaceutical composition of the present disclosure.

In an eighth aspect, the present disclosure also provides a method for treating and/or preventing a disease associated with DGAT2 gene overexpression, which comprises administering to a subject in need a therapeutically effective amount of the siRNA of the present disclosure, or the siRNA conjugate or the prodrug thereof of the present disclosure, or the pharmaceutical composition of the present disclosure.

In a specific embodiment, the disease is selected from the group consisting of: non-alcoholic fatty liver disease, non-alcoholic steatohepatitis, alcoholic fatty liver, obesity, hepatic steatosis, fatty liver, and hyperlipidemia.

In a ninth aspect, the present disclosure also provides the siRNA of the present disclosure, or the siRNA conjugate or the prodrug thereof of the present disclosure, or the pharmaceutical composition of the present disclosure for use as a medicament.

In a tenth aspect, the present disclosure also provides the siRNA of the present disclosure, or the siRNA conjugate or the prodrug thereof of the present disclosure, or the pharmaceutical composition of the present disclosure for use in therapy.

Beneficial effects

The siRNA, the siRNA conjugate, and the pharmaceutical composition comprising the same provided in the present disclosure exhibit excellent inhibition activity against DGAT2 gene expression in *in vitro* cell experiments and have good potential for treating diseases associated with DGAT2 gene overexpression. For example, the siRNA and the conjugate thereof provided in the present disclosure can reduce the expression of DGAT2 mRNA in the liver, exhibit low toxic and side effects, good plasma stability, and have promising prospects for clinical application.

The siRNA provided in the present disclosure exhibits good inhibition effects on the DGAT2 gene in HepG2 cells. In some specific embodiments, the siRNA of the present disclosure can significantly inhibit DGAT2 gene expression at both 1 nM and 0.01 nM, with an inhibition rate of up to 95.24 % at 1 nM after 24 h and an inhibition rate of up to 83.65% at 0.01 nM after 24 h.

In some specific embodiments, the siRNA conjugate provided in the present disclosure exhibits relatively high DGAT2 gene inhibition activity in PHH cells. For example, when the siRNA conjugate is introduced into PHH via transfection, the inhibition rate at 5 nM can be up to 97.01%; when the siRNA conjugate is introduced into PHH via free uptake, the inhibition rate at 200 nM can be up to 95.32%.

## DETAILED DESCRIPTION

### Definitions

Throughout the specification, unless otherwise specified, in the art, "G", "C", "A", "T", and "U" generally represent the bases of guanine, cytosine, adenine, thymine, and uracil, respectively, but it is also generally known in the art that "G", "C", "A", "T", and "U" each also generally represent nucleotides that contain guanine, cytosine, adenine, thymine, and uracil, respectively, as bases, which is a common way of representing deoxyribonucleic acid sequences and/or ribonucleic acid sequences. Therefore, in the context of the present disclosure, the meanings represented by "G", "C", "A", "T", and "U" include the various possible cases described above, and "nucleotide" and "ribonucleotide" are used interchangeably herein. The lowercase letters a, u, c, and g represent 2'-methoxy-modified nucleotides; Af, Gf, Cf, and Uf represent 2'-fluoro-modified nucleotides; d indicates that the nucleotide adjacent to the right side of the letter d is a 2'-deoxyribonucleotide; the lowercase letter s indicates that the two nucleotides adjacent to the left and right sides of the letter s are linked by a phosphorothioate group; P1 indicates that the nucleotide adjacent to the right side of P1 is a 5'-phosphate nucleotide; EVP indicates that the nucleotide adjacent to the right side of EVP is a 5'-(E)-vinylphosphonate nucleotide; <u>***A***</u>, <u>***U***</u>, <u>***C***</u>, and <u>***G***</u> (underlined + bold + italic) represent GNA-modified nucleotides.

In the text above and below, the "2'-fluoro-modified nucleotide" refers to a nucleotide formed by substituting hydroxyl at the 2' position of a ribosyl group of the nucleotide with fluorine. "Non-fluoro-modified nucleotide" refers to a nucleotide formed by substituting hydroxyl at the 2' position of a ribosyl group of the nucleotide with a non-fluorine group or a nucleotide analog. In some embodiments, each of the non-fluoro-modified nucleotides is independently selected from one of a nucleotide formed by substituting hydroxyl at the 2' position of a ribosyl group of the nucleotide with a non-fluorine group and a nucleotide analog. Nucleotides in which hydroxyl at the 2' position of a ribosyl group is substituted with a non-fluorine group are well known to those skilled in the art. These nucleotides may be selected from 2'-alkoxy-modified nucleotides, 2'-substituted alkoxy-modified nucleotides, 2'-alkyl-modified nucleotides, 2'-substituted alkyl-modified nucleotides, 2'-amino-modified nucleotides, 2'-substituted amino-modified nucleotides, and 2'-deoxynucleotides.

"Alkyl" includes linear, branched, or cyclic saturated alkyl groups. For example, the alkyl includes, but is not

limited to, methyl, ethyl, propyl, cyclopropyl, n-butyl, isobutyl, sec-butyl, tert-butyl, cyclobutyl, n-pentyl, cyclohexyl, and similar groups. Illustratively, "$C_{1-6}$" in "$C_{1-6}$ alkyl" refers to a group containing 1, 2, 3, 4, 5, or 6 carbon atoms arranged in a linear, branched, or cyclic form.

"Alkoxy" as used herein refers to an alkyl group attached to the rest of the molecule via an oxygen atom (-O-alkyl), wherein the alkyl is as defined herein. Non-limiting examples of the alkoxy include methoxy, ethoxy, trifluoromethoxy, difluoromethoxy, n-propoxy, isopropoxy, n-butoxy, tert-butoxy, n-pentoxy, and the like.

"Nucleotide analog" refers to a group that can replace a nucleotide in a nucleic acid but has a structure different from adenine ribonucleotide, guanine ribonucleotide, cytosine ribonucleotide, uracil ribonucleotide, or thymine deoxyribonucleotide, e.g., an isonucleotide, a bridged nucleic acid (BNA for short), or an acyclic nucleotide.

A BNA refers to a constrained or inaccessible nucleotide. A BNA may contain a five-membered, six-membered, or seven-membered ring bridge structure with a "fixed" C3'-endosugar constriction. The bridge is typically incorporated at the 2'- and 4'-positions of the ribose to provide a 2',4'-BNA nucleotide, such as LNA, ENA, and cET BNA, wherein LNA is as shown in formula (1), ENA is as shown in formula (2), and cET BNA is as shown in formula (3):

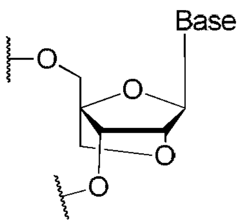
formula (1)

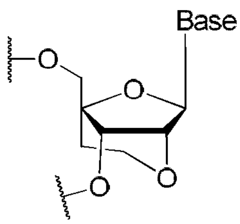
formula (2)

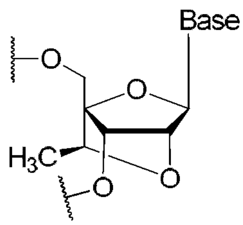
formula (3).

Acyclic nucleotides are a class of nucleotides formed by opening the sugar ring of nucleotides, such as unlocked nucleic acid (UNA) or glycerol nucleic acid (GNA), wherein UNA is as shown in formula (4), and GNA is as shown in formula (5):

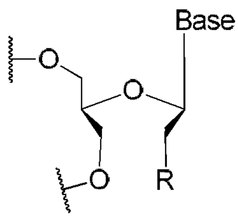
formula (4)

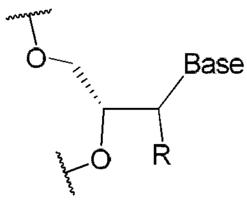
formula (5).

In formula (4) and formula (5), R is selected from H, OH, and alkoxy (O-alkyl).

The isonucleotide refers to a compound formed by changing the position of a base on the ribose ring in a nucleotide, for example, a compound formed by moving the base from the 1'-position to the 2'-position or 3'-position of the ribose ring, as shown in formula (6) or (7):

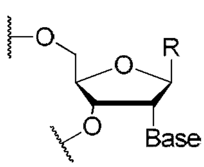
formula (6)

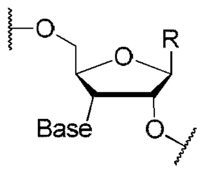
formula (7).

In the compounds of formulas (6) and (7), Base represents a base, such as A, U, G, C, or T; R is selected from H, OH, F, and non-fluorine groups as described above.

In some embodiments, the nucleotide analog is selected from one of isonucleotides, LNA, ENA, cET BNA, UNA, and GNA. In some embodiments, each of the non-fluoro-modified nucleotides is a 2'-methoxy-modified nucleotide, a GNA-modified nucleotide, or a combination of any two or more thereof. In some preferred embodiments, each of the non-fluoro-modified nucleotides is a 2'-methoxy-modified nucleotide, and in the text above and below, the 2'-methoxy-modified nucleotide refers to a nucleotide formed by substituting 2'-hydroxyl of a ribosyl group with methoxy.

The "2'-methoxy-modified nucleotide" refers to a nucleotide formed by substituting 2'-hydroxyl of a ribosyl group with methoxy. The "phosphorothioate group" refers to a phosphorothioate group formed by substituting one oxygen atom in a phosphodiester bond of a phosphoester group with a sulfur atom.

The "phosphorothioate group" refers to the following formula:

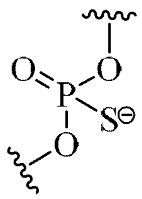

.

The "5'-phosphate nucleotide" refers to a structure of the following formula:

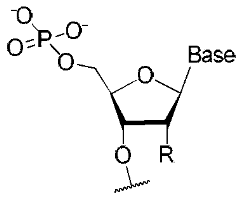

.

In the context of the specification, the expressions "complementary" and "reversely complementary" are used interchangeably and have the meaning well known to those skilled in the art, that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (G) is always paired with the pyrimidine base cytosine (C). Each base pair comprises a purine and a pyrimidine. When adenines of one strand are always paired with thymines (or uracils) of the other strand and guanines are always paired with cytosines, the two strands are considered complementary to each other, and the sequence of one strand can be deduced from the sequence of its complementary strand. Accordingly, "mismatch" in the art means that in a double-stranded nucleic acid, the bases in the corresponding positions are not paired in a complementary manner.

In the text above and below, unless otherwise specified, "substantially reversely complementary" means that there are no more than 3 base mismatches between two nucleotide sequence segments involved; "virtually reversely complementary" means that there is no more than 1 base mismatch between two nucleotide sequence segments; "completely reversely complementary" means that there is no base mismatch between two nucleotide sequence segments.

In the text above and below, a "nucleotide difference" between one nucleotide sequence and another nucleotide sequence refers to a change in the type of nucleotide base at the same position when comparing the former with the latter. For example, if one nucleotide base in the latter is A, while the corresponding nucleotide base at the same position in the former is U, C, G, or T, it is considered that a nucleotide difference exists between the two nucleotide sequences at that position. In some embodiments, the replacement of a nucleotide at its original position with a abasic nucleotide or an equivalent thereof can also be considered to create a nucleotide difference at that position.

In the context, the "overhang" refers to one or more unpaired nucleotides protruding from the duplex structure of an siRNA when the 3' end of one strand of the siRNA extends beyond the 5' end of the other strand, or vice versa. The "blunt end" means that there are no unpaired nucleotides at that end of the siRNA, i.e., no nucleotide overhang. A "blunt-ended" siRNA is an siRNA that is fully double-stranded along its entire length, meaning that there are no nucleotide overhangs at either end of the molecule.

In the text above and below of the specification of the present disclosure, especially when the preparation method for the siRNA, the pharmaceutical composition, or the siRNA conjugate of the present disclosure is described, unless otherwise specified, the nucleoside monomer refers to a modified or unmodified nucleoside phosphoramidite monomer used in solid-phase phosphoramidite synthesis according to the type and sequence of nucleotides in the siRNA or siRNA conjugate to be prepared. Solid-phase phosphoramidite synthesis is a method used in RNA synthesis, well known to those skilled in the art. The nucleoside monomers used in the present disclosure are all commercially available.

In the context of the present disclosure, unless otherwise stated, "conjugation" means that two or more chemical moieties each having a specific function are covalently linked to each other; accordingly, "conjugate" refers to a compound formed by covalent linkage between the various chemical moieties. Further, the "siRNA conjugate" refers to a compound formed by covalently linking one or more chemical moieties with specific functions to an siRNA. The siRNA conjugate should be understood as a general term of multiple siRNA conjugates or an siRNA conjugate represented by a certain

chemical formula according to the context. In the context of the specification of the present disclosure, the "conjugated molecule" should be understood as a specific compound that may be conjugated to an siRNA through a reaction, ultimately forming the siRNA conjugate of the present disclosure.

In the context, the term "metabolite" refers to an active product produced by *in vivo* metabolism of the siRNA conjugate of the present disclosure. Such products may result from, for example, dephosphorylation at the 5' end and/or removal of one nucleotide from the 3' end of the antisense strand of the administered siRNA conjugate. Thus, the present disclosure encompasses metabolites of the siRNA conjugate of the present disclosure, including products produced by a method comprising contacting the siRNA conjugate of the present disclosure with a mammal for a period of time sufficient to obtain the metabolites thereof.

The siRNA conjugate provided in the present disclosure also exists in a prodrug form. Prodrugs of the siRNA conjugates described herein readily undergo chemical changes under physiological conditions to convert into the siRNA conjugates of the present disclosure. Any substance that can be converted *in vivo* to provide a bioactive substance (i.e., the siRNA conjugate provided in the present disclosure) is a prodrug within the scope and spirit of the present disclosure. For example, the M6 conjugate in the present disclosure is a prodrug of the M2 conjugate, and the difference between the modifications of the M2 conjugate and the M6 conjugate lies in whether there is P1 at the 5' end of the antisense strand. The M6 conjugate without P1 undergoes phosphorylation *in vivo* to convert into an M2 conjugate sequence with P1, thereby exerting its effect. Similarly, the same relationship exists between the M7 conjugate and the M3 conjugate. Therefore, prodrugs corresponding to the siRNA conjugates are included herein.

A variety of hydroxyl protecting groups may be used in the present disclosure. In general, protecting groups render a chemical functional group insensitive to specific reaction conditions and may be added to and removed from that functional group in a molecule without substantially damaging the rest of the molecule. In some embodiments, the protecting groups are stable under basic conditions and can be removed under acidic conditions. In some embodiments, non-exclusive examples of hydroxyl protecting groups that can be used in the present disclosure include monomethoxytrityl, 9-phenylxanthine-9-yl (Pixyl), and 9-(p-methoxyphenyl)xanthine-9-yl (Mox). In some embodiments, non-exclusive examples of hydroxyl protecting groups that can be used in the present disclosure include Tr (trityl), MMTr (4-methoxytrityl), DMTr (4,4'-dimethoxytrityl), and TMTr (4,4',4"-trimethoxytrityl).

As used in the specification, "optional" or "optionally" means that the subsequently described event or circumstance may or may not occur, and that the description includes instances where the event or circumstance occurs and instances where it does not.

The term "subject", as used in the specification, refers to any animal, such as a mammal or a marsupial. Subjects of the present disclosure include, but are not limited to, humans, non-human primates (e.g., rhesus or other types of macaques), mice, pigs, horses, donkeys, cows, sheep, rats, rabbits, or any species of poultry.

As used in the specification, "treatment" refers to means of obtaining a beneficial or desired result, including but not limited to a treatment benefit. The "treatment benefit" means eradicating or ameliorating the underlying disorder being treated. Furthermore, treatment benefit is obtained by eradicating or ameliorating one or more physiological symptoms associated with the underlying disorder, whereby improvement is observed in the subject, although the subject may still be afflicted by the underlying disorder.

As used in the specification, "prevention" refers to means of obtaining a beneficial or desired result, including but not limited to prophylactic benefit. In order to obtain "prophylactic benefit", an siRNA, an siRNA conjugate, or a pharmaceutical composition may be administered to a subject at risk for a particular disease, or a subject with one or more physiological symptoms of a reported disease, even though a diagnosis of the disease may not have been made.

## siRNA

The present disclosure relates to an siRNA capable of inhibiting DGAT2 gene expression. The siRNA of the present disclosure contains a nucleotide group as a basic structural unit. It is well known to those skilled in the art that the nucleotide group contains a phosphate group, a ribosyl group, and a base. siRNAs that are generally active, i.e., functional, have a length of about 12-40 nucleotides, and in some embodiments, have a length of about 15-30 nucleotides.

The siRNA of the present disclosure comprises a sense strand and an antisense strand, and each nucleotide in the siRNA is independently a modified or unmodified nucleotide, wherein the sense strand comprises a nucleotide sequence I, the antisense strand comprises a nucleotide sequence II, and the nucleotide sequence I is at least partially reversely complementary to the nucleotide sequence II to form a double-stranded region. In some embodiments, the double-stranded region has a length of 15-30 nucleotide pairs. In other embodiments, the double-stranded region has a length of 17-23 nucleotide pairs. In other embodiments, the double-stranded region has a length of 19-21 nucleotide pairs. In yet other embodiments, the double-stranded region has a length of 19 or 21 nucleotide pairs.

In some embodiments, the sense strand further comprises a nucleotide sequence III, and the antisense strand further comprises a nucleotide sequence IV, wherein the nucleotide sequence III and the nucleotide sequence IV each independently have a length of 0-11 nucleotides; the nucleotide sequence III is linked to the 5' end of the nucleotide

sequence I, the nucleotide sequence IV is linked to the 3' end of the nucleotide sequence II, and the nucleotide sequence III and nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences. In some embodiments, the sense strand further comprises a nucleotide sequence III, and the antisense strand further comprises a nucleotide sequence IV, wherein the nucleotide sequence III and the nucleotide sequence IV each independently have a length of 0-11 nucleotides; the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences. In some embodiments, the sense strand further comprises a nucleotide sequence III, and the antisense strand further comprises a nucleotide sequence IV, wherein the nucleotide sequence III and the nucleotide sequence IV each independently have a length of 0-11 nucleotides; the nucleotide sequence III is linked to the 5' end of the nucleotide sequence I, the nucleotide sequence IV is linked to the 3' end of the nucleotide sequence II, and the nucleotide sequence III and the nucleotide sequence IV are equal in length and virtually reversely complementary or completely reversely complementary; and the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences.

In some embodiments, the sense strand further comprises a nucleotide sequence V, and/or the antisense strand further comprises a nucleotide sequence VI, wherein the nucleotide sequences V and VI have a length of 0 to 3 nucleotides; the nucleotide sequence V is linked to the 3' end of the sense strand to form the 3' overhang of the sense strand, and/or the nucleotide sequence VI is linked to the 3' end of the antisense strand to form the 3' overhang of the antisense strand. In some embodiments, the nucleotide sequence V or VI has a length of 2 nucleotides. In other embodiments, the nucleotide sequence V or VI is two consecutive thymine deoxyribonucleotides or two consecutive uracil ribonucleotides. In other embodiments, the nucleotide sequence V or VI is mismatched or complementary to a nucleotide at a corresponding position of the a mRNA.

The sense strand and the antisense strand provided in the present disclosure are identical or different in length, and in some embodiments, the sense strand or the antisense strand has 15-30 nucleotides. In other embodiments, the sense strand or the antisense strand has 19-25 nucleotides. In other embodiments, the sense strand or the antisense strand has 19-23 nucleotides. The length ratio of the sense strand to the antisense strand of the siRNA provided in the present disclosure may be 15/15, 16/16, 17/17, 18/18, 19/19, 19/20, 19/21, 19/22, 19/23, 20/19, 20/20, 20/21, 20/22, 20/23, 21/19, 21/20, 21/21, 21/22, 21/23, 22/19, 22/20, 22/21, 22/22, 22/23, 23/19, 23/20, 23/21, 23/22, 23/23, 24/24, 25/25, 26/26, 27/27, 28/28, 29/29, 30/30, 22/24, 22/25, 22/26, 23/24, 23/25, 23/26, etc. In some embodiments, the length ratio of the sense strand to the antisense strand of the siRNA is 19/19, 21/21, 19/21, 21/23, or 23/23, and at this time, the siRNA of the present disclosure has better cellular mRNA silencing activity.

It is found that different modification strategies can have distinct effects on indexes such as stability, biological activity, and cytotoxicity of siRNA. For example, CN102140458B investigated various chemical modification strategies for siRNAs and validated 7 effective modification approaches. Compared with unmodified siRNAs, siRNAs obtained by one of the modification approaches not only had enhanced stability in the blood but also maintained inhibition activity largely comparable to that of unmodified siRNAs.

The nucleotides in the siRNA of the present disclosure are each independently a modified or unmodified nucleotide. In some embodiments, each nucleotide in the siRNA of the present disclosure is an unmodified nucleotide; in some embodiments, some or all of the nucleotides in the siRNA of the present disclosure are modified nucleotides, and these modifications on the nucleotide groups do not cause obvious weakening or loss of the function of the siRNA of the present disclosure to inhibit DGAT2 gene expression.

In some embodiments, the siRNA of the present disclosure comprises at least 1 modified nucleotide. In the context of the present disclosure, the term "modified nucleotide" as used refers to a nucleotide formed by substituting hydroxyl at the 2' position of a ribosyl group of the nucleotide with other groups or a nucleotide analog, or a nucleotide with a modified base. The modified nucleotides do not cause obvious weakening or loss of the function of the siRNA to inhibit gene expression. For example, the modified nucleotides disclosed in J.K. Watts, G.F. Deleavey, and M.J. Damha, Chemically modified siRNA: tools and applications. Drug Discov Today, 2008, 13(19-20): 842-55 may be selected.

In some embodiments, at least one nucleotide in the sense strand or the antisense strand of the siRNA provided in the present disclosure is a modified nucleotide, and/or at least one phosphoester group is a phosphoester group with a modification group; in other words, at least a portion of a phosphoester group and/or a ribosyl group in a phosphate-ribose

backbone of at least one single strand of the sense strand and the antisense strand is a phosphoester group with a modification group and/or a ribosyl group with a modification group. In some embodiments, the phosphoester group with a modification group is a phosphorothioate group formed by substituting one oxygen atom in a phosphodiester bond of the phosphoester group with a sulfur atom.

In some embodiments, the siRNA comprises a sense strand without a 3' overhang nucleotide; that is, while the sense strand of the siRNA may have a 3' overhang nucleotide, it can form a blunt end by removing the 3' overhang nucleotide from the sense strand. In some specific embodiments, the sense strand and the antisense strand of the siRNA comprise sequences shown in Table 1-1.

In some embodiments, when the nucleotide sequence of the sense strand and the nucleotide sequence of the antisense strand are complementary to form a double-stranded region, and there is no protruding nucleotide at the 3' end of the sense strand, a nucleotide sequence V is added to the 3' end of the sense strand as the protruding nucleotide. Subsequently, after chemical modification of the nucleotide sequence formed by linking the nucleotide sequence V to the 3' end of the sense strand is completed, the nucleotide sequence V is removed, and correspondingly, the sense strand of the siRNA forms a blunt end.

In some embodiments, when the nucleotide sequence of the sense strand and the nucleotide sequence of the antisense strand are complementary to form a double-stranded region and the 3' end of the sense strand has a protruding nucleotide extending out of the double-stranded region, the protruding nucleotide located at the 3' end of the sense strand is removed to serve as the nucleotide sequence of the sense strand, and correspondingly, the sense strand of the siRNA forms a blunt end.

In some embodiments, the 5' end nucleotide of the antisense strand is linked to a 5' phosphate group or a 5' phosphate-derived group.

An exemplary structure of the 5' phosphate group is:

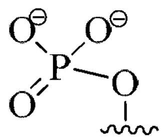;

the structure of the 5' phosphate-derived group includes, but is not limited to:

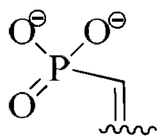 (EVP), 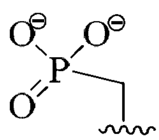,

and the like.

After the 5' end nucleotide located on the antisense strand is linked to a 5' phosphate group or a 5' phosphate-derived group, the following structures are formed:

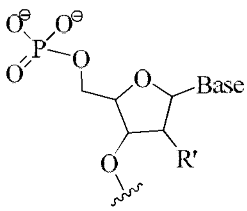, 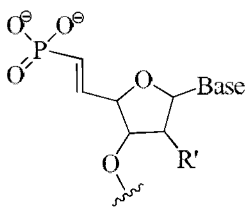, 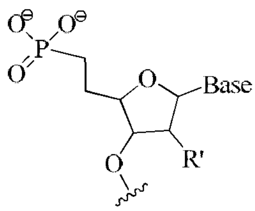,

wherein Base represents a base, e.g., A, U, G, C, or T. R' is hydroxyl or is substituted with various groups known to those skilled in the art; for example, the modified nucleotide after substitution may be a 2'-fluoro (2'-F)-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, or a 2'-deoxynucleotide.

In some embodiments, the 5' end nucleotide of the antisense strand is not linked to a 5' phosphate group or a 5' phosphate-derived group, and has a structure as shown in formula (X):

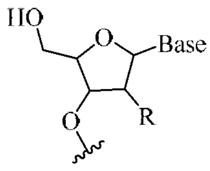

formula (X)

wherein Base represents a base, e.g., A, U, G, C, or T. R is hydroxyl or hydrogen or is substituted with various groups known to those skilled in the art; for example, R may be 2'-fluoro (2'-F), 2'-alkoxy, 2'-substituted alkoxy, 2'-alkyl, 2'-substituted alkyl, 2'-amino, 2'-substituted amino, or 2'-deoxynucleotide.

An exemplary modified nucleotide has the structure shown below:

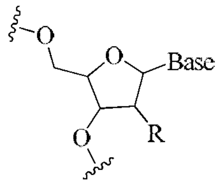

,

wherein Base represents a base, e.g., A, U, G, C, or T. The hydroxyl at the 2' position of the ribosyl group is substituted with R. The hydroxyl at the 2' position of these ribosyl groups may be substituted with various groups known to those skilled in the art; for example, the modified nucleotide after substitution may be a 2'-fluoro (2'-F)-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, or a 2'-deoxynucleotide.

In some embodiments, the 2'-alkoxy-modified nucleotide is a 2'-methoxy (2'-OMe, 2'-O-$CH_3$)-modified nucleotide or the like.

In some embodiments, the 2'-substituted alkoxy-modified nucleotide is a 2'-methoxyethoxy (2'-O-$CH_2$-$CH_2$-O-$CH_3$)-modified nucleotide, a 2'-O-$CH_2$-CH=$CH_2$-modified nucleotide, or the like.

In some embodiments, the 2'-substituted alkyl-modified nucleotide is a 2'-$CH_2$-$CH_2$-CH=$CH_2$-modified nucleotide or the like.

In some embodiments, all of the nucleotides in the sense strand and/or the antisense strand are modified nucleotides. In some embodiments, each nucleotide in the sense strand and the antisense strand of the siRNA provided in the present disclosure is independently a 2'-fluoro-modified nucleotide or a non-fluoro-modified nucleotide. In some embodiments, each non-fluoro-modified nucleotide is a 2'-methoxy-modified nucleotide, a 2'-deoxynucleotide, or a GNA-modified nucleotide, and the 2'-methoxy-modified nucleotide refers to a nucleotide formed by substituting 2'-hydroxyl of a ribosyl group with methoxy.

In some embodiments, at least one of the following linkages between nucleotides in the siRNA is a phosphorothioate group linkage:

a linkage between the first nucleotide and the second nucleotide from the 5' end of the sense strand;
a linkage between the second nucleotide and the third nucleotide from the 5' end of the sense strand;
a linkage between the first nucleotide and the second nucleotide from the 3' end of the sense strand;
a linkage between the second nucleotide and the third nucleotide from the 3' end of the sense strand;
a linkage between the first nucleotide and the second nucleotide from the 5' end of the antisense strand;
a linkage between the second nucleotide and the third nucleotide from the 5' end of the antisense strand;
a linkage between the first nucleotide and the second nucleotide from the 3' end of the antisense strand;
a linkage between the second nucleotide and the third nucleotide from the 3' end of the antisense strand.

In some embodiments, in the direction from the 5' end to the 3' end of the siRNA, the sense strand comprises phosphorothioate groups located at the positions shown below:

a position between the first nucleotide and the second nucleotide from the 5' end of the sense strand;
a position between the second nucleotide and the third nucleotide from the 5' end of the sense strand;
a position between the first nucleotide and the second nucleotide from the 3' end of the sense strand;
a position between the second nucleotide and the third nucleotide from the 3' end of the sense strand; or,
the sense strand comprises phosphorothioate groups located at the positions shown below:

a position between the first nucleotide and the second nucleotide from the 5' end of the sense strand;

a position between the second nucleotide and the third nucleotide from the 5’ end of the sense strand.

In some embodiments, in the direction from the 5’ end to the 3’ end of the siRNA, the antisense strand comprises phosphorothioate groups located at the positions shown below:

a position between the first nucleotide and the second nucleotide from the 5’ end of the antisense strand;
a position between the second nucleotide and the third nucleotide from the 5’ end of the antisense strand;
a position between the first nucleotide and the second nucleotide from the 3’ end of the antisense strand;
a position between the second nucleotide and the third nucleotide from the 3’ end of the antisense strand.

In one embodiment, any one nucleotide in the sense strand and the antisense strand is independently a 2’-fluoro-modified nucleotide, a 2’-methoxy-modified nucleotide, a GNA-modified nucleotide, or a combination of any two or more thereof.

SiRNA conjugate or prodrug thereof

The present disclosure relates to an siRNA conjugate or a prodrug thereof, and the siRNA conjugate contains the siRNA described above and a conjugated group conjugated to the siRNA.

In the present disclosure, the sense strand and the antisense strand of the siRNA conjugate form a double-stranded region of the siRNA conjugate, and a blunt end is formed at the 3’ end of the sense strand of the siRNA conjugate. In some embodiments, the 3’ end of the sense strand of the siRNA conjugate forms a blunt end, and the 3’ end of the antisense strand of the siRNA conjugate has 1-3 protruding nucleotides extending out of the double-stranded region. In other embodiments, the 3’ end of the sense strand of the siRNA conjugate forms a blunt end, and the 3’ end of the antisense strand of the siRNA conjugate forms a blunt end.

In some preferred embodiments, the siRNA conjugate is obtained by conjugating the siRNA to a conjugated group. The sense strand and the antisense strand of the siRNA are complementary to form a double-stranded region of the siRNA, the 3’ end of the sense strand of the siRNA forms a blunt end, and the conjugated group is conjugated to the 3’ end of the sense strand with the blunt end to form the siRNA conjugate.

In some preferred embodiments, the 3’ end of the sense strand of the siRNA has a protruding nucleotide extending out of the double-stranded region, the sequence with a 3’ blunt end formed by removing the protruding nucleotide located at the 3’ end of the sense strand is used as a nucleotide sequence for linking to a conjugated group, and the conjugated group is linked to the 3’ blunt end of the sense strand to form the siRNA conjugate.

In some more preferred embodiments, when the nucleotide sequence of the sense strand and the nucleotide sequence of the antisense strand are complementary to form a double-stranded region, and there is no protruding nucleotide at the 3’ end of the sense strand, a nucleotide sequence V is added to the 3’ end of the sense strand as the protruding nucleotide. The sequence with a 3’ blunt end formed by removing the protruding nucleotide located at the 3’ end of the sense strand is used as a nucleotide sequence for linking to a conjugated group, and the conjugated group is linked to the 3’ blunt end of the sense strand to form the siRNA conjugate.

In some more preferred embodiments, when the nucleotide sequence of the sense strand and the nucleotide sequence of the antisense strand are complementary to form a double-stranded region and the 3’ end of the sense strand has a protruding nucleotide extending out of the double-stranded region, the sequence with a 3’ blunt end formed by removing the protruding nucleotide located at the 3’ end of the sense strand is used as a nucleotide sequence for linking to a conjugated group, and the conjugated group is linked to the 3’ blunt end of the sense strand to form the siRNA conjugate.

Illustratively, for the sequences of the siRNA of N-FY001003M2, the 3’ end of the sense strand of the siRNA has a protruding nucleotide extending out of the double-stranded region, and the blunt-ended sequence ascscauaGfaCfUfAfuuugcuuu formed by removing the protruding -scsa nucleotide located at the 3’ end of the sense strand is used as a nucleotide sequence for linking to an L96 conjugated group. Therefore, the sequences forming the siRNA conjugate are as follows: the sense strand is ascscauaGfaCfUfAfuuugcuuuL96, and the antisense strand is P1asAfsagcAfaauagucUfaUfggusgsu.

Generally, the conjugated group comprises at least one pharmaceutically acceptable targeting group or optionally further comprises a linker, and the siRNA, the linker, and the targeting group are linked sequentially. In some embodiments, the number of the targeting group is 1-6. In some embodiments, the number of the targeting group is 2-4. The siRNA molecule may be non-covalently or covalently conjugated to the conjugated group. For example, the same may be covalently conjugated to the conjugated group. The conjugation site between the siRNA and the conjugated group may be at the 3’ end or 5’ end of the sense strand of the siRNA, or at the 5’ end of the antisense strand, or in the internal sequence of the siRNA. In some embodiments, the conjugation site between the siRNA and the conjugated group is at the 3’ end of the sense strand of the siRNA.

In some embodiments, the conjugated group may be linked to the phosphate group, the 2’-position hydroxyl, or

the base of the nucleotide. In some embodiments, the conjugated group may be linked to the 3'-position hydroxyl, and at this time, the nucleotides are linked via a 2'-5' phosphodiester bond. When the conjugated group is linked to the end of the siRNA chain, the conjugated group is usually linked to the phosphate group of the nucleotide; when the conjugated group is linked to the internal sequence of the siRNA, the conjugated group is usually linked to the ribose sugar ring or the base. For various linkage methods, please refer to the publication: Muthiah Manoharan et.al., siRNA conjugates carrying sequentially assembled trivalent N-acetylgalactosamine linked through nucleosides elicit robust gene silencing in vivo in hepatocytes. ACS Chemical biology, 2015, 10(5): 1181-7.

In some embodiments, the siRNA and the conjugated group may be linked via acid-labile or reducible chemical bonds, which are degradable in the acidic environment of the cell endosome, thereby enabling the siRNA to be in a free state. For non-degradable conjugation methods, the conjugated group can be linked to the sense strand of the siRNA to minimize the effect of conjugation on the activity of the siRNA.

In some embodiments, the pharmaceutically acceptable targeting group may be a ligand conventionally used in the field of siRNA administration, such as the various ligands described in WO2009082607A2, which is incorporated herein by reference in its entirety.

In some embodiments, the pharmaceutically acceptable targeting group may be selected from one or more of ligands formed by the following targeting molecules or derivatives thereof: lipophilic molecules such as cholesterol, bile acids, vitamins (e.g., vitamin E), and lipid molecules of different chain lengths; polymers such as polyethylene glycol; polypeptides such as transmembrane peptides; aptamers; antibodies; quantum dots; sugars such as lactose, polylactose, mannose, galactose, and N-acetylgalactosamine (GalNAc); folate; ligands for receptor expressed by hepatocytes such as asialoglycoprotein, asialosaccharide residues, lipoproteins (e.g., high-density lipoprotein, low-density lipoprotein, etc.), glucagon, neurotransmitters (e.g., epinephrine), growth factors, transferrin, and the like.

In some embodiments, each of the ligands is independently selected from a ligand capable of binding to a cell surface receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human hepatocyte surface receptor. In some embodiments, at least one ligand is a ligand capable of binding to the liver surface asialoglycoprotein receptor (ASGPR). The types of these ligands are well known to those skilled in the art, and their functions are generally to bind to specific receptors on the surface of target cells and mediate the delivery of an siRNA linked to a ligand to the target cells.

In some embodiments, the pharmaceutically acceptable targeting group may be any ligand that binds to the asialoglycoprotein receptor (ASGPR) on the surface of mammalian hepatocytes. In some embodiments, each ligand is independently an asialoglycoprotein, such as asialoorosomucoid (ASOR) or asialofetuin (ASF). In some embodiments, the ligand is a saccharide or a saccharide derivative.

In some embodiments, at least one ligand is a saccharide. In some embodiments, each ligand is a saccharide. In some embodiments, at least one ligand is a monosaccharide, a polysaccharide, a modified monosaccharide, a modified polysaccharide, or a saccharide derivative. In some embodiments, at least one of the ligands may be a monosaccharide, a disaccharide, or a trisaccharide. In some embodiments, at least one ligand is a modified saccharide. In some embodiments, each ligand is a modified saccharide. In some embodiments, each ligand is independently selected from a polysaccharide, a modified polysaccharide, a monosaccharide, a modified monosaccharide, a polysaccharide derivative, and a monosaccharide derivative. In some embodiments, each or at least one ligand is selected from the group consisting of the following saccharide: glucose and derivatives thereof, mannan and derivatives thereof, galactose and derivatives thereof, xylose and derivatives thereof, ribose and derivatives thereof, fucose and derivatives thereof, lactose and derivatives thereof, maltose and derivatives thereof, arabinose and derivatives thereof, fructose and derivatives thereof, and sialic acid.

In some embodiments, each of the ligands can be independently selected from D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, α-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, 2,3,4-tri-O-acetyl-1-thio-6-O-trityl-α-D-glucopyranoside methyl ester, 4-thio-β-D-galactopyranose, 3,4,6,7-tetra-O-acetyl-2-deoxy-1,5-dithio-α-D-glucopyranoside ethyl ester, 2,5-anhydro-D-allose nitrile, ribose, D-ribose, D-4-thioribose, L-ribose, and L-4-thioribose. Other options for the ligand can be found, for example, in CN105378082A, which is incorporated herein by reference in its entirety.

In some embodiments, the pharmaceutically acceptable targeting group in the siRNA conjugate may be galactose or N-acetylgalactosamine, wherein the galactose or N-acetylgalactosamine molecule may be monovalent, divalent, trivalent, or tetravalent. It should be understood that the monovalent, divalent, trivalent, or tetravalent described

herein refers to that after an siRNA molecule forms an siRNA conjugate with a conjugated group containing a galactose or N-acetylgalactosamine molecule as a targeting group, the molar ratio of the siRNA molecule to the galactose or N-acetylgalactosamine molecule in the siRNA conjugate is 1:1, 1:2, 1:3, or 1:4. In some embodiments, the pharmaceutically acceptable targeting group is N-acetylgalactosamine. In some embodiments, when the siRNA described in the present disclosure is conjugated to a conjugated group comprising N-acetylgalactosamine, the N-acetylgalactosamine molecule is trivalent or tetravalent. In some embodiments, when the siRNA described in the present disclosure is conjugated to a conjugated group containing N-acetylgalactosamine, the N-acetylgalactosamine molecule is trivalent.

The targeting group may be linked to the siRNA molecule through a suitable linker, and those skilled in the art may select a suitable linker according to the specific type of the targeting group. The types of these linkers and targeting groups and the linkage methods with the siRNA can be found in the disclosure of WO2015006740A2, which is incorporated herein by reference in its entirety.

Synthesis method for siRNA

Nucleoside monomers were linked one by one in the 3'-5' direction in the order according to the nucleotide arrangement by a conventional solid-phase phosphoramidite method in the art. The linkage of each nucleoside monomer involves four reactions: deprotection, coupling, oxidation or sulfurization, and capping. When a phosphoester linkage is present between two nucleotides, the steps for linking the subsequent nucleoside monomer include deprotection, coupling, oxidation, and capping. When a phosphorothioate linkage is present between two nucleotides, the steps for linking the subsequent nucleoside monomer include deprotection, coupling, sulfurization, and capping. In the present disclosure, nucleotide monomers are selected according to the target sequence to be synthesized, and the selected nucleotide monomers are nucleotide monomers commonly used by those skilled in the art. For example, the nucleotide monomer for synthesizing A may be, but is not limited to, adenosine-3-phosphate. It should be understood that these monomers, when present in the oligonucleotide, are linked to each other via a 5'-3' phosphodiester bond or a 5'-3' phosphorothioate group, and in cases where, for example, the last nucleotide in the 5' to 3' direction has a hydroxyl group at its 3' position, this is achieved according to conventional techniques in the art.

For example, the synthesis conditions of the siRNA of the present disclosure may be as follows.

Deprotection conditions include: a reaction temperature of 25°C, a reaction time of 70 s, a deprotection reagent selected from a solution of dichloroacetic acid in dichloromethane (3% v/v), and a molar ratio of the deprotection reagent to the protecting groups on the solid-phase support of 5:1.

Coupling reaction conditions include: a reaction temperature of 25°C, a reaction time of 600 s, a coupling reagent selected from a 0.25 M solution of 5-ethylthio-1H-tetrazole (ETT) in acetonitrile, and a molar ratio of the nucleic acid sequence linked to the solid-phase support to the nucleoside monomer of 1:10.

Oxidation reaction conditions include: a reaction temperature of 25°C, a reaction time of 15 s, an oxidation reagent selected from a 0.05 M solution of iodine in tetrahydrofuran, and a molar ratio of the oxidation reagent to the nucleic acid sequence linked to the solid-phase support in the coupling step of 30:1.

Sulfurization reaction conditions include: a reaction temperature of 25°C, a reaction time of 300 s, a sulfurization reagent selected from xanthane hydride, and a molar ratio of the sulfurization reagent to the nucleic acid sequence linked to the solid-phase support in the coupling step of 120:1.

Capping reaction conditions include: a reaction temperature of 25°C, a reaction time of 15 s, a capping reagent selected from a mixed solution of CapA (a 10% solution of acetic anhydride in acetonitrile) and CapB (a 10% solution of N-methylimidazole in pyridine/acetonitrile) in a molar ratio of 1:1, and a molar ratio of the capping reagent to the nucleic acid sequence linked to the solid-phase support, the molar ratio being acetic anhydride:N-methylimidazole:nucleic acid sequence attached to the solid-phase support = 1:1:1. After all nucleoside monomers are linked, the nucleic acid sequence linked to the solid-phase support is sequentially cleaved, deprotected, purified, and desalted to obtain the sense strand and the antisense strand of the siRNA, and finally the two strands are heated and annealed to obtain the product.

Methods of cleavage, deprotection, purification, desalting, and annealing are well known in the art. For example, cleavage and deprotection are performed by contacting the nucleotide sequence linked to the solid-phase support with concentrated ammonia water; purification is performed by chromatography; desalting is performed by reversed-phase chromatography; annealing is performed by mixing the sense strand and the antisense strand in an equimolar ratio under different stringency conditions, followed by gradual cooling.

Taking the conjugated group L96 represented by formula (I) as an example, the synthesis method for the siRNA conjugate is as follows:

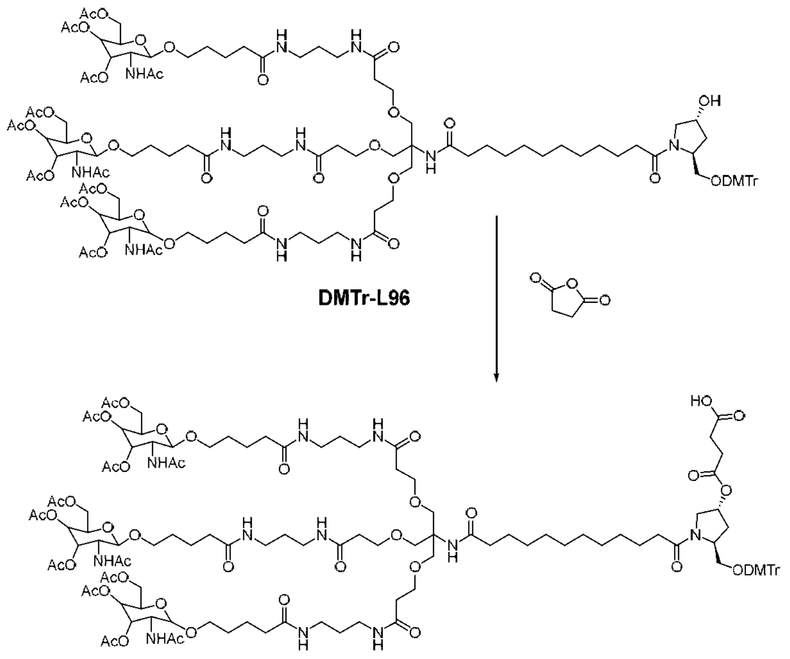

DMTr-L96

L96-A

Step 1: reaction of DMTr-L96 with succinic anhydride to obtain compound L96-A:

Preparation process: DMTr-L96, succinic anhydride, 4-dimethylaminopyridine, and diisopropylethylamine are added to dichloromethane, and the mixture is reacted under stirring at 25°C for 24 h. The reaction solution is then washed with 0.5 M triethylammonium phosphate. The aqueous phase is washed three times with dichloromethane, and the organic phases are combined and concentrated under reduced pressure to obtain a crude product. The crude product is purified by column chromatography to obtain pure L96-A.

Step 2: reaction of L96-A with $NH_2$-SPS to obtain L96-B:

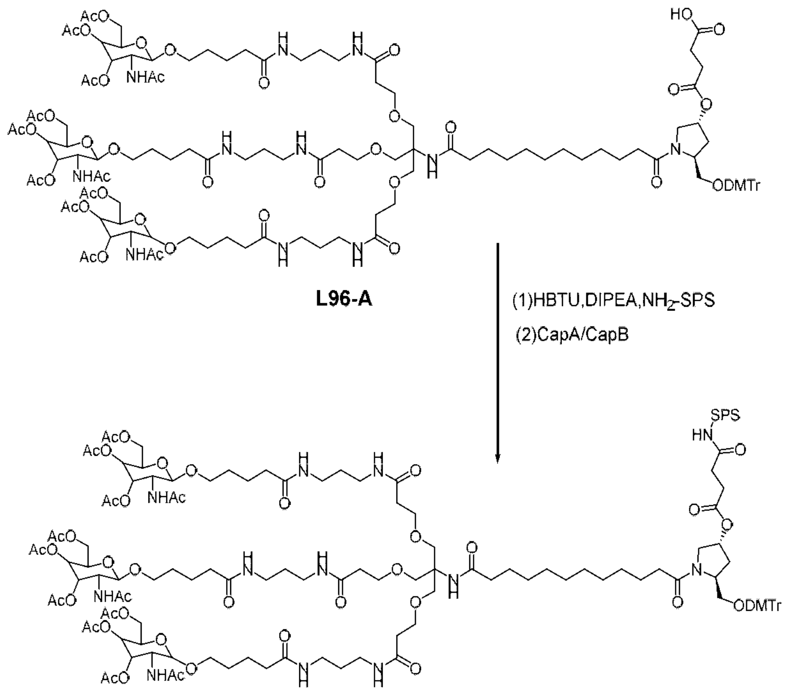


**L96-A**

**L96-B**

Preparation process: L96-A, O-(benzotriazol-1-yl)-N,N,N',N'-tetramethyluronium hexafluorophosphate (HBTU), and diisopropylethylamine (DIPEA) are mixed and dissolved in acetonitrile. The mixture is stirred at room temperature for 5 min to give a homogeneous solution. An aminomethyl resin ($NH_2$-SPS, 100-200 mesh) is added to the reaction solution. The mixture is reacted on a shaker at 25°C for 18 h and then filtered, and the filter cake is washed sequentially with dichloromethane and acetonitrile to obtain a filter cake. The filter cake is subjected to a capping reaction using a CapA/CapB mixed solution to obtain L96-B, which serves as the solid-phase support containing the conjugated molecule. Subsequently, nucleoside monomers are linked to the conjugated molecule under coupling reaction. The siRNA sense strand linked to the conjugate molecule is then synthesized according to the previously described siRNA molecule synthesis method, and the siRNA antisense strand is synthesized using the previously described siRNA molecule synthesis method. Finally, annealing is performed to generate the siRNA conjugate of the present disclosure.

<u>Pharmaceutical composition</u>

The present disclosure provides a pharmaceutical composition, and the pharmaceutical composition contains the siRNA as described above as an active ingredient, and a pharmaceutically acceptable carrier.

The pharmaceutically acceptable carrier may be a carrier commonly used in the field of siRNA administration, for example, but not limited to one or more of lipid nanoparticles (LNPs), magnetic nanoparticles (such as nanoparticles based on $Fe_3O_4$ or $Fe_2O_3$), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid)copolymer (PLGA), poly(2-aminoethyl ethylene phosphate) (PPEEA), and poly(2-dimethylaminoethyl methacrylate) (PDMAEMA), as well as derivatives thereof.

In the pharmaceutical composition, there is no special requirement on the contents of the siRNA and the pharmaceutically acceptable carrier, and the content may be a conventional content of each component.

In some embodiments, the pharmaceutical composition may further comprise other pharmaceutically acceptable excipients, which may be one or more of various formulations or compounds conventionally used in the art. For example, the other pharmaceutically acceptable excipients may include at least one of a pH buffer, a protective agent, and an osmotic pressure regulator.

The pH buffer may be a Tris hydrochloride buffer with a pH value of 7.5-8.5 and/or a phosphate buffer with a pH value of 5.5-8.5, for example, a phosphate buffer with a pH value of 5.5-8.5.

The protective agent may be at least one of inositol, sorbitol, sucrose, trehalose, mannose, maltose, lactose, and glucose. Based on the total weight of the pharmaceutical composition, the content of the protective agent may be 0.01-30 wt%.

The osmotic pressure regulator may be sodium chloride and/or potassium chloride. The content of the osmotic pressure regulator makes the osmotic pressure of the pharmaceutical composition 200-700 milliosmol/kg (mOsm/kg).

According to the desired osmotic pressure, those skilled in the art may easily determine the content of the osmotic pressure regulator.

In some embodiments, the pharmaceutical composition may be a liquid formulation, such as an injection; it may also be a freeze-dried powder injection, which is mixed with a liquid excipient during administration to prepare a liquid formulation. The liquid formulation may be used for, but is not limited to, subcutaneous, intramuscular, or intravenous injection administration, and may also be administered via, but not limited to, spray to the lungs or spray to other organ tissues (e.g., the liver) through the lungs. In some embodiments, the pharmaceutical composition is used for intravenous injection administration.

In some embodiments, the pharmaceutical composition may be in the form of a liposomal formulation. In some embodiments, the pharmaceutically acceptable carrier used in the liposomal formulation comprises an amine-containing transfection compound (hereinafter also referred to as an organic amine), a helper lipid, and/or a PEGylated lipid.

The following examples are intended to further illustrate the present disclosure, but not to limit the present disclosure in any way.

## Examples

Other objects, features, and advantages of the present disclosure will become apparent from the following detailed description. However, it should be understood that the detailed description and the specific examples, while indicating specific embodiments of the present disclosure, are provided for explanatory purposes only. After reading this detailed description, various changes and modifications made within the spirit and scope of the present disclosure will become apparent to those skilled in the art.

The experimental techniques and methods used in the examples, unless otherwise specified, are all conventional techniques and methods. For example, in the following examples, if no specific conditions are indicated, the experimental methods are generally performed under conventional conditions, such as those described in Sambrook et al., Molecular Cloning: A Laboratory Manual (New York: Cold Spring Harbor Laboratory Press, 1989) or those as recommended by the manufacturer. All materials, reagents, and the like used in the examples are commercially available unless otherwise specified.

### Example 1. Preparation of siRNAs

siRNA molecules with the following sequences were synthesized by Shanghai Talen-bio Scientific Co.,Ltd.

Table 1. siRNAs and sequences thereof

| siRNA ID | Sense strand: 5'-3' | SEQ ID NO | Antisense strand: 5'-3' | SEQ ID NO |
|---|---|---|---|---|
| N-FY001001 | GACUAUUUGCUUUCAAAGAAU | 1 | 1<br>UCUUUGAAAGCAAAUAGUCUA | 2 |
| N-FY001001M2 | gsascuauUfuGfCfUfuucaaagasasu | 3 | P1usCfsuuuGfaaagcaaAfuAfgucsusa | 4 |
| N-FY001001M3 | gsascuauUfuGfCfUfuucaaagasasu | 3 | P1usCfsuuuGfaAfAfgcaaAfuAfgucsusa | 5 |
| N-FY001001M4 | gsascuauUfuGfCfUfuucaaagasasu | 3 | P1usCfsuuu***G***aaagcaaAfuAfgucsusa | 6 |
| N-FY001001M5 | gsascuauUfuGfCfUfuucaaagasasu | 3 | P1usCfsuuuGf***A***aagcaaAfuAfgucsusa | 7 |
| N-FY001002 | GCUACUUUCGAGACUACUUUC | 8 | AAGUAGUCUCGAAAGUAGCGC | 9 |
| N-FY001002M2 | gscsuacuUfuCfGfAfgacuacuususc | 10 | P1asAfsguaGfucucgaaAfgUfagcsgsc | 11 |

(continued)

| siRNA ID | Sense strand: 5'-3' | SEQ ID NO | Antisense strand: 5'-3' | SEQ ID NO |
|---|---|---|---|---|
| N-FY001002M3 | gscsuacuUfuCfGfAfgacuacuususc | 10 | P1asAfsguaGfuCfUfcgaaAfgUfagcsgsc | 12 |
| N-FY001002M4 | gscsuacuUfuCfGfAfgacuacuususc | 10 | P1asAfsgua***G***ucucgaaAfgUfagcsgsc | 13 |
| N-FY001002M5 | gscsuacuUfuCfGfAfgacuacuususc | 10 | P1asAfsguaGf***U***cucgaaAfgUfagcsgsc | 14 |
| N-FY001003 | ACCAUAGACUAUUUGCUUUCA | 15 | AAAGCAAAUAGUCUAUGGUGU | 16 |
| N-FY001003M2 | ascscauaGfaCfUfAfuuugcuuuscsa | 17 | P1asAfsagcAfaauagucUfaUfggusgsu | 18 |
| N-FY001003M2D2 | ascscauaGfaCfUfAfuuugcuuu | 19 | P1asAfsagcAfaauagucUfaUfggusgsu | 18 |
| N-FY001003M3 | ascscauaGfaCfUfAfuuugcuuuscsa | 17 | P1asAfsagcAfaAfUfagucUfaUfggusgsu | 20 |
| N-FY001003M4 | ascscauaGfaCfUfAfuuugcuuuscsa | 17 | P1asAfsagc***A***aauagucUfaUfggusgsu | 21 |
| N-FY001003M5 | ascscauaGfaCfUfAfuuugcuuuscsa | 17 | P1asAfsagcAf***A***auagucUfaUfggusgsu | 22 |
| N-FY001004 | CCAUCCUCAUGUACAUAUU | 23 | AAUAUGUACAUGAGGAUGG | 24 |
| N-FY001004M2 | cscsauccUfcAfUfGfuacauauusTsT | 25 | P1asAfsuauGfuacaugaGfgAfuggsTsT | 26 |
| N-FY001004M2D2 | cscsauccUfcAfUfGfuacauauu | 27 | P1asAfsuauGfuacaugaGfgAfuggsTsT | 26 |
| N-FY001004M3 | cscsauccUfcAfUfGfuacauauusTsT | 25 | P1asAfsuauGfuAfCfaugaGfgAfuggsTsT | 28 |
| N-FY001004M4 | cscsauccUfcAfUfGfuacauauusTsT | 25 | P1asAfsuau***G***uacaugaGfgAfuggsTsT | 29 |
| N-FY001004M5 | cscsauccUfcAfUfGfuacauauusTsT | 25 | P1asAfsuauGf***U***acaugaGfgAfuggsTsT | 30 |
| N-FY001005 | CCAUCCUCAUGUACAUAUA | 31 | UAUAUGUACAUGAGGAUGG | 32 |
| N-FY001005M2 | cscsauccUfcAfUfGfuacauauasTsT | 33 | P1usAfsuauGfuacaugaGfgAfuggsTsT | 34 |
| N-FY001005M3 | cscsauccUfcAfUfGfuacauauasTsT | 33 | P1usAfsuauGfuAfCfaugaGfgAfuggsTsT | 35 |

(continued)

| siRNA ID | Sense strand: 5'-3' | SEQ ID NO | Antisense strand: 5'-3' | SEQ ID NO |
|---|---|---|---|---|
| N-FY001005M4 | cscsauccUfcAfUfGfuacauauasTsT | 33 | P1usAfsuau***G***uacaugaGfgAfuggsTsT | 36 |
| N-FY001005M5 | cscsauccUfcAfUfGfuacauauasTsT | 33 | P1usAfsuauGf***U***acaugaGfgAfuggsTsT | 37 |
| N-FY001003M6 | ascscauaGfaCfUfAfuuugcuuuscsa | 17 | asAfsagcAfaauagucUfaUfggusgsu | 38 |
| N-FY001003M7 | ascscauaGfaCfUfAfuuugcuuuscsa | 17 | asAfsagcAfaAfUfagucUfaUfggusgsu | 39 |
| N-FY001003M8 | ascscauaGfaCfUfAfuuugcuuuscsa | 17 | EVPasAfsagcAfaauagucUfaUfggusgsu | 40 |
| N-FY001003M9 | ascscauaGfaCfUfAfuuugcuuuscsa | 17 | EVPasAfsagcAfaAfUfagucUfaUfggusgsu | 41 |
| N-FY001003D2 | ACCAUAGACUAUUUGCUUU | 42 | AAAGCAAAUAGUCUAUGGUGU | 16 |
| N-FY001006 | UGCCAUCCUCAUGUACAUAUU | 43 | UAUGUACAUGAGGAUGGCACU | 44 |
| N-FY001007 | GCCAUCCUCAUGUACAUAUUC | 45 | AUAUGUACAUGAGGAUGGCAC | 46 |
| N-FY001008 | UGCCAUCCUCAUGUACAUA | 47 | UAUGUACAUGAGGAUGGCA | 48 |
| N-FY001010 | CAUCCUCAUGUACAUAUUA | 49 | UAAUAUGUACAUGAGGAUG | 50 |
| N-FY001011 | CCUCAUGUACAUAUUCUGAAC | 51 | UCAGAAUAUGUACAUGAGGAU | 52 |
| N-FY001012 | CUCAUGUACAUAUUCUGCACUGA | 53 | AGUGCAGAAUAUGUACAUGAGGA | 54 |
| N-FY001013 | CCUCAUGUACAUAUUCUGA | 55 | UCAGAAUAUGUACAUGAGG | 56 |
| N-FY001014 | CUCAUGUACAUAUUCUGCA | 57 | UGCAGAAUAUGUACAUGAG | 58 |
| N-FY001015 | UGUGGCGCUACUUUCGAGA | 59 | UCUCGAAAGUAGCGCCACA | 60 |
| N-FY001016 | GUGGCGCUACUUUCGAGAA | 61 | UUCUCGAAAGUAGCGCCAC | 62 |
| N-FY001017 | GCGCUACUUUCGAGACUAAUU | 63 | UUAGUCUCGAAAGUAGCGCCA | 64 |
| N-FY001017M2 | gscsgcuaCfuUfUfCfgagacuaasusu | 65 | P1usUfsaguCfucgaaagUfaGfcgcscsa | 66 |

(continued)

| siRNA ID | Sense strand: 5'-3' | SEQ ID NO | Antisense strand: 5'-3' | SEQ ID NO |
|---|---|---|---|---|
| N-FY001018 | CGCUACUUUCGAGACUACUUU | 67 | AGUAGUCUCGAAAGUAGCGCC | 68 |
| N-FY001019 | CUACUUUCGAGACUACUUUCC | 69 | AAAGUAGUCUCGAAAGUAGCG | 70 |
| N-FY001019M2 | csusacuuUfcGfAfGfacuacuuuscsc | 71 | P1asAfsaguAfgucucgaAfaGfuagscsg | 72 |
| N-FY001020 | UACUUUCGAGACUACUUUACC | 73 | UAAAGUAGUCUCGAAAGUAGC | 74 |
| N-FY001021 | CUACUUUCGAGACUACUUU | 75 | AAAGUAGUCUCGAAAGUAG | 76 |
| N-FY001021M2 | csusacuuUfcGfAfGfacuacuuusTsT | 77 | P1asAfsaguAfgucucgaAfaGfuagsTsT | 78 |
| N-FY001021M4 | csusacuuUfcGfAfGfacuacuuusTsT | 77 | P1asAfsagu***A***gucucgaAfaGfuagsTsT | 79 |
| N-FY001021M5 | csusacuuUfcGfAfGfacuacuuusTsT | 77 | P1asAfsaguAf***G***ucucgaAfaGfuagsTsT | 80 |
| N-FY001022 | CUUUCGAGACUACUUUCCCAUCC | 81 | AUGGGAAAGUAGUCUCGAAAGUA | 82 |
| N-FY001022M2 | csusuucgAfgAfCfUfacuuucccauscsc | 83 | P1asUfsgggAfaaguaguCfuCfgaaagsusa | 84 |
| N-FY001022M4 | csusuucgAfgAfCfUfacuuucccauscsc | 83 | P1asUfsggg***A***aaguaguCfuCfgaaagsusa | 85 |
| N-FY001022M5 | csusuucgAfgAfCfUfacuuucccauscsc | 83 | P1asUfsgggAf***A***aguaguCfuCfgaaagsusa | 86 |
| N-FY001024 | GGGACACCAUAGACUAUUA | 87 | UAAUAGUCUAUGGUGUCCC | 88 |
| N-FY001024M2 | gsgsgacaCfcAfUfAfgacuauuasTsT | 89 | P1usAfsauaGfucuauggUfgUfcccsTsT | 90 |
| N-FY001025 | ACACCAUAGACUAUUUGCUUU | 91 | AGCAAAUAGUCUAUGGUGUCC | 92 |
| N-FY001025M2 | ascsaccaUfaGfAfCfuauuugcususu | 93 | P1asGfscaaAfuagucuaUfgGfuguscsc | 94 |
| N-FY001025M3 | ascsaccaUfaGfAfCfuauuugcususu | 93 | P1asGfscaaAfuAfGfucuaUfgGfuguscsc | 95 |
| N-FY001025M4 | ascsaccaUfaGfAfCfuauuugcususu | 93 | P1asGfscaa***A***uagucuaUfgGfuguscsc | 96 |
| N-FY001025M5 | ascsaccaUfaGfAfCfuauuugcususu | 93 | P1asGfscaaAf***U***agucuaUfgGfuguscsc | 97 |

(continued)

| siRNA ID | Sense strand: 5'-3' | SEQ ID NO | Antisense strand: 5'-3' | SEQ ID NO |
|---|---|---|---|---|
| N-FY001025M6 | ascsaccaUfaGfAfCfuauuugcususu | 93 | asGfscaaAfuagucuaUfgGfuguscsc | 98 |
| N-FY001025M7 | ascsaccaUfaGfAfCfuauuugcususu | 93 | asGfscaaAfuAfGfucuaUfgGfuguscsc | 99 |
| N-FY001025M8 | ascsaccaUfaGfAfCfuauuugcususu | 93 | EVPasGfscaaAfuagucuaUfgGfuguscsc | 100 |
| N-FY001025M9 | ascsaccaUfaGfAfCfuauuugcususu | 93 | EVPasGfscaaAfuAfGfucuaUfgGfuguscsc | 101 |
| N-FY001026 | CACCAUAGACUAUUUGCUUUC | 102 | AAGCAAAUAGUCUAUGGUGUC | 103 |
| N-FY001026M2 | csasccauAfgAfCfUfauuugcuususc | 104 | P1asAfsgcaAfauagucuAfuGfgugsusc | 105 |
| N-FY001026M3 | csasccauAfgAfCfUfauuugcuususc | 104 | P1asAfsgcaAfaUfAfgucuAfuGfgugsusc | 106 |
| N-FY001026M4 | csasccauAfgAfCfUfauuugcuususc | 104 | P1asAfsgca***A***auagucuAfuGfgugsusc | 107 |
| N-FY001026M5 | csasccauAfgAfCfUfauuugcuususc | 104 | P1asAfsgcaAf***A***uagucuAfuGfgugsusc | 108 |
| N-FY001026M6 | csasccauAfgAfCfUfauuugcuususc | 104 | asAfsgcaAfauagucuAfuGfgugsusc | 109 |
| N-FY001026M7 | csasccauAfgAfCfUfauuugcuususc | 104 | asAfsgcaAfaUfAfgucuAfuGfgugsusc | 110 |
| N-FY001026M8 | csasccauAfgAfCfUfauuugcuususc | 104 | EVPasAfsgcaAfauagucuAfuGfgugsusc | 111 |
| N-FY001026M9 | csasccauAfgAfCfUfauuugcuususc | 104 | EVPasAfsgcaAfaUfAfgucuAfuGfgugsusc | 112 |
| N-FY001027 | ACCAUAGACUAUUUGCUUUCAAA | 113 | UGAAAGCAAAUAGUCUAUGGUGU | 114 |
| N-FY001027M2 | ascscauaGfaCfUfAfuuugcuuucasasa | 115 | P1usGfsaaaGfcaaauagUfcUfauggusgsu | 116 |
| N-FY001027M3 | ascscauaGfaCfUfAfuuugcuuucasasa | 115 | P1usGfsaaaGfcAfAfauagUfcUfauggusgsu | 117 |
| N-FY001027M4 | ascscauaGfaCfUfAfuuugcuuucasasa | 115 | P1usGfsaaa***G***caaauagUfcUfauggusgsu | 118 |
| N-FY001027M5 | ascscauaGfaCfUfAfuuugcuuucasasa | 115 | P1usGfsaaaGf***C***aaauagUfcUfauggusgsu | 119 |

(continued)

| siRNA ID | Sense strand: 5'-3' | SEQ ID NO | Antisense strand: 5'-3' | SEQ ID NO |
|---|---|---|---|---|
| N-FY001027M6 | ascscauaGfaCfUfAfuuugcuuucasasa | 115 | usGfsaaaGfcaaauagUfcUfauggusgsu | 120 |
| N-FY001027M7 | ascscauaGfaCfUfAfuuugcuuucasasa | 115 | usGfsaaaGfcAfAfauagUfcUfauggusgsu | 121 |
| N-FY001027M8 | ascscauaGfaCfUfAfuuugcuuucasasa | 115 | EVPusGfsaaaGfcaaauagUfcUfauggusgsu | 122 |
| N-FY001027M9 | ascscauaGfaCfUfAfuuugcuuucasasa | 115 | EVPusGfsaaaGfcAfAfauagUfcUfauggusgsu | 123 |
| N-FY001028 | CCAUAGACUAUUUGCUUUCAAAG | 124 | UUGAAAGCAAAUAGUCUAUGGUG | 125 |
| N-FY001028M2 | cscsauagAfcUfAfUfuugcuuucaasasg | 126 | P1usUfsgaaAfgcaaauaGfuCfuauggsusg | 127 |
| N-FY001029 | ACCAUAGACUAUUUGCUUU | 42 | AAAGCAAAUAGUCUAUGGU | 128 |
| N-FY001029M2 | ascscauaGfaCfUfAfuuugcuuusTsT | 129 | P1asAfsagcAfaauagucUfaUfggusTsT | 130 |
| N-FY001030 | CCAUAGACUAUUUGCUUUA | 131 | UAAAGCAAAUAGUCUAUGG | 132 |
| N-FY001030M2 | cscsauagAfcUfAfUfuugcuuuasTsT | 133 | P1usAfsaagCfaaauaguCfuAfuggsTsT | 134 |
| N-FY001031 | UAGACUAUUUGCUUUCAAAGA | 135 | UUUGAAAGCAAAUAGUCUAUG | 136 |
| N-FY001031M2 | usasgacuAfuUfUfGfcuuucaaasgsa | 137 | P1usUfsugaAfagcaaauAfgUfcuasusg | 138 |
| N-FY001031M3 | usasgacuAfuUfUfGfcuuucaaasgsa | 137 | P1usUfsugaAfaGfCfaaauAfgUfcuasusg | 139 |
| N-FY001031M4 | usasgacuAfuUfUfGfcuuucaaasgsa | 137 | P1usUfsuga***A***agcaaauAfgUfcuasusg | 140 |
| N-FY001031M5 | usasgacuAfuUfUfGfcuuucaaasgsa | 137 | P1usUfsugaAf***A***gcaaauAfgUfcuasusg | 141 |
| N-FY001031M6 | usasgacuAfuUfUfGfcuuucaaasgsa | 137 | usUfsugaAfagcaaauAfgUfcuasusg | 142 |
| N-FY001031M7 | usasgacuAfuUfUfGfcuuucaaasgsa | 137 | usUfsugaAfaGfCfaaauAfgUfcuasusg | 143 |
| N-FY001031M8 | usasgacuAfuUfUfGfcuuucaaasgsa | 137 | EVPusUfsugaAfagcaaauAfgUfcuasusg | 144 |

(continued)

| siRNA ID | Sense strand: 5'-3' | SEQ ID NO | Antisense strand: 5'-3' | SEQ ID NO |
|---|---|---|---|---|
| N-FY001031M9 | usasgacuAfuUfUfGfcuuucaaasgsa | 137 | EVPusUfsugaAfaGfCfaaauAfgUfcuasusg | 145 |
| N-FY001032 | AGACUAUUUGCUUUCAAAAAA | 146 | UUUUGAAAGCAAAUAGUCUAU | 147 |
| N-FY001032M2 | asgsacuaUfuUfGfCfuuucaaaasasa | 148 | P1usUfsuugAfaagcaaaUfaGfucusasu | 149 |
| N-FY001033 | ACUAUUUGCUUUCAAAGAAUG | 150 | UUCUUUGAAAGCAAAUAGUCU | 151 |
| N-FY001033M2 | ascsuauuUfgCfUfUfucaaagaasusg | 152 | P1usUfscuuUfgaaagcaAfaUfaguscsu | 153 |
| N-FY001033M3 | ascsuauuUfgCfUfUfucaaagaasusg | 152 | P1usUfscuuUfgAfAfagcaAfaUfaguscsu | 154 |
| N-FY001033M4 | ascsuauuUfgCfUfUfucaaagaasusg | 152 | P1usUfscuu***U***gaaagcaAfaUfaguscsu | 155 |
| N-FY001033M5 | ascsuauuUfgCfUfUfucaaagaasusg | 152 | P1usUfscuuUf***G***aaagcaAfaUfaguscsu | 156 |
| N-FY001033M6 | ascsuauuUfgCfUfUfucaaagaasusg | 152 | usUfscuuUfgaaagcaAfaUfaguscsu | 157 |
| N-FY001033M7 | ascsuauuUfgCfUfUfucaaagaasusg | 152 | usUfscuuUfgAfAfagcaAfaUfaguscsu | 158 |
| N-FY001033M8 | ascsuauuUfgCfUfUfucaaagaasusg | 152 | EVPusUfscuuUfgaaagcaAfaUfaguscsu | 159 |
| N-FY001033M9 | ascsuauuUfgCfUfUfucaaagaasusg | 152 | EVPusUfscuuUfgAfAfagcaAfaUfaguscsu | 160 |
| N-FY001034 | CUAUUUGCUUUCAAAGAAUGG | 161 | AUUCUUUGAAAGCAAAUAGUC | 162 |
| N-FY001035 | ACUAUUUGCUUUCAAAGAA | 163 | UUCUUUGAAAGCAAAUAGU | 164 |
| N-FY001036 | CUAUUUGCUUUCAAAGAAU | 165 | AUUCUUUGAAAGCAAAUAG | 166 |
| N-FY001037 | UUUGCUUUCAAAGAAUGGGAAUG | 167 | UUCCCAUUCUUUGAAAGCAAAUA | 168 |
| N-FY001003M6D2 | ascscauaGfaCfUfAfuuugcuuu | 19 | asAfsagcAfaauagucUfaUfggusgsu | 38 |
| N-FY001003M7D2 | ascscauaGfaCfUfAfuuugcuuu | 19 | asAfsagcAfaAfUfagucUfaUfggusgsu | 39 |

(continued)

| siRNA ID | Sense strand: 5'-3' | SEQ ID NO | Antisense strand: 5'-3' | SEQ ID NO |
|---|---|---|---|---|
| N-FY001003M10 | ascscauaGfaCfUfAfuuugcuuuscsa | 17 | asAfsagcAfaauagucUfauggusgsu | 169 |
| N-FY001003M11 | ascscaUfaGfAfCfuauuugcuuuscsa | 170 | asAfsagcAfaauagucUfaUfggusgsu | 38 |
| N-FY001003M12 | ascscauagaCfUfAfuuugcuuuscsa | 171 | asAfsagcAfaauagucUfaUfggusgsu | 38 |
| N-FY001003M13 | ascscauagAfCfUfauuugcuuuscsa | 172 | asAfsagcAfaauagucUfaUfggusgsu | 38 |
| N-FY001003M14 | ascscauaGfaCfUfAfuuugcuuuscsa | 17 | asAfsAfgCfaaAfuAfgucUfaUfgGfusgsu | 173 |
| N-FY001003M15 | ascscauaGfaCfUfAfuuugcuuuscsa | 17 | asAfsAfGfCfaAfauAfgucUfauggusgsu | 174 |
| N-FY001003M16 | ascscauagAfCfUfAfuuugcuuuscsa | 175 | asAfsAfGfCfaAfauAfgucUfauggusgsu | 174 |
| N-FY001003M17 | ascscauaGfaCfUfAfuuugcuuuscsa | 17 | asAfsaGfcAfaauagUfcUfaUfgGfusGfsu | 176 |
| N-FY001003M18 | ascscauaGfaCfUfAfuuugcuuuscsa | 17 | asAfsagcaaauagucUfauggusgsu | 177 |
| N-FY001003M19 | ascscauaGfaCfudAuuugcuuuscsa | 178 | asAfsaGfCfAfaAfuAfgUfcUfaUfgGfusGfsu | 179 |
| N-FY001003M20 | ascscauaGfaCfudAuuugcuuuscsa | 178 | asAfsagcAfaauagucUfaUfggusgsu | 38 |
| N-FY001003M21 | ascscauaGfaCfuAfuuugcuuuscsa | 180 | asAfsagcAfaauagucUfaUfggusgsu | 38 |
| N-FY001003M21D2 | ascscauaGfaCfuAfuuugcuuu | 181 | asAfsagcAfaauagucUfaUfggusgsu | 38 |
| N-FY001003M22 | ascscauaGfacuAfuUfuGfcuuuscsa | 182 | asAfsagcAfaauagucUfaUfggusgsu | 38 |
| N-FY001003M23 | ascscauagAfCfUfaUfuugcuuuscsa | 183 | asAfsagcAfaauagucUfaUfggusgsu | 38 |
| N-FY001003M24 | ascscauaGfaCfuAfuUfuGfcuuuscsa | 184 | asAfsagcAfaauagucUfaUfggusgsu | 38 |
| N-FY001003M24D2 | ascscauaGfaCfuAfuUfuGfcuuu | 185 | asAfsagcAfaauagucUfaUfggusgsu | 38 |
| N-FY001003M25 | ascscauagaCfuAfuUfugcuuuscsa | 186 | asAfsagcAfaauagucUfaUfggusgsu | 38 |
| N-FY001003M26 | ascscauaGfaCfuAfuUfugcuuuscsa | 187 | asAfsagcAfaauagucUfaUfggusgsu | 38 |

(continued)

| siRNA ID | Sense strand: 5'-3' | SEQ ID NO | Antisense strand: 5'-3' | SEQ ID NO |
|---|---|---|---|---|
| N-FY001003M26D2 | ascscauaGfaCfuAfuUfugcuuu | 188 | asAfsagcAfaauagucUfaUfggusgsu | 38 |
| N-FY001003M27 | ascsCfauaGfAfCfuauuugcuuuscsa | 189 | asAfsagcAfaauagucUfaUfggusgsu | 38 |
| N-FY001003M27D2 | ascsCfauaGfAfCfuauuugcuuu | 190 | asAfsagcAfaauagucUfaUfggusgsu | 38 |
| N-FY001003M28 | ascsCfauagaCfuAfuUfugcuuuscsa | 191 | asAfsagcAfaauagucUfaUfggusgsu | 38 |
| N-FY001003M29 | ascsCfauaGfaCfuAfuuugcuuuscsa | 192 | asAfsagcAfaauagucUfaUfggusgsu | 38 |
| N-FY001003M29D2 | ascsCfauaGfaCfuAfuuugcuuu | 193 | asAfsagcAfaauagucUfaUfggusgsu | 38 |
| N-FY001003M30 | ascscauaGfacuAfuUfugcuuuscsa | 194 | asAfsagcAfaauagucUfaUfggusgsu | 38 |
| N-FY001003M31 | ascscauaGfacuAfUfUfugcuuuscsa | 195 | asAfsagcAfaauagucUfaUfggusgsu | 38 |
| N-FY001003M32 | ascsCfauaGfacuAfuuugCfuuuscsa | 196 | asAfsagcAfaauagucUfaUfggusgsu | 38 |
| N-FY001003M33 | ascsCfauaGfacuAfuuugcUfuuscsa | 197 | asAfsagcAfaauagucUfaUfggusgsu | 38 |
| N-FY001003M34 | ascsCfauaGfacuAfuuugCfUfuuscsa | 198 | asAfsagcAfaauagucUfaUfggusgsu | 38 |
| N-FY001003M35 | ascscauaGfaCfUfAfuuugcuuuscsa | 17 | asAfsAfGfCf***A***AfauAfgucUfauggusgsu | 199 |
| N-FY001003M37 | ascscauaGfaCfUfAfuuugcuuu | 19 | asAfsagcaAfauAfgucUfauggusgsu | 200 |
| N-FY001003M40 | ascscauaGfaCfUfAfuuugcuuu | 19 | asAfsagCfaAfauagucUfauggusgsu | 201 |
| N-FY001003M44 | ascsCfauaGfAfCfuauuugcuuu | 190 | asAfsagcaAfauAfgucUfauggusgsu | 200 |
| N-FY001003M45 | ascsCfauaGfaCfuAfuuugcuuu | 193 | asAfsagcaAfauAfgucUfauggusgsu | 200 |
| N-FY001002M10 | gscsuacuUfuCfGfAfgacuacuusuсc | 10 | asAfsguaGfucucgaaAfguagesgsc | 202 |
| N-FY001002M11 | gscsuaCfuUfUfCfgagacuacuususc | 203 | asAfsguaGfucucgaaAfgUfagcsgsc | 204 |
| N-FY001002M12 | gscsuacuuuCfGfAfgacuacususc | 205 | asAfsguaGfucucgaaAfgUfagcsgsc | 204 |

(continued)

| siRNA ID | Sense strand: 5'-3' | SEQ ID NO | Antisense strand: 5'-3' | SEQ ID NO |
|---|---|---|---|---|
| N-FY001002M13 | gscsuacuuUfCfGfagacuacuususc | 206 | asAfsguaGfucucgaaAfgUfagcsgsc | 204 |
| N-FY001002M 14 | gscsuacuUfuCfGfAfgacuacuususc | 10 | asAfsGfuAfguCfuCfgaaAfgUfaGfcsgsc | 207 |
| N-FY001002M15 | gscsuacuUfuCfGfAfgacuacuususc | 10 | asAfsGfUfAfgUfcuCfgaaAfguagcsgsc | 208 |
| N-FY001002M16 | gscsuacuuUfCfGfAfgacuacuususc | 209 | asAfsGfUfAfgUfcuCfgaaAfguagcsgsc | 208 |
| N-FY001002M17 | gscsuacuUfuCfGfAfgacuacuususc | 10 | asAfsgUfaGfucucgAfaAfgUfaGfcsGfsc | 210 |
| N-FY001002M18 | gscsuacuUfuCfGfAfgacuacuususc | 10 | asAfsguagucucgaaAfguagcsgsc | 211 |
| N-FY001002M19 | gscsuacuUfuCfgdAgacuacuususc | 212 | asAfsgUfAfGfuCfuCfgAfaAfgUfaGfcsGfsc | 213 |
| N-FY001002M20 | gscsuacuUfuCfgdAgacuacuususc | 212 | asAfsguaGfucucgaaAfgUfagcsgsc | 204 |
| N-FY001002M21 | gscsuacuUfuCfgAfgacuacuususc | 214 | asAfsguaGfucucgaaAfgUfagcsgsc | 204 |
| N-FY001002M22 | gscsuacuUfucgAfgAfcUfacuususc | 215 | asAfsguaGfucucgaaAfgUfagcsgsc | 204 |
| N-FY001002M23 | gscsuacuuUfCfGfaGfacuacuususc | 216 | asAfsguaGfucucgaaAfgUfagcsgsc | 204 |
| N-FY001002M24 | gscsuacuUfuCfgAfgAfcUfacuususc | 217 | asAfsguaGfucucgaaAfgUfagcsgsc | 204 |
| N-FY001002M25 | gscsuacuuuCfgAfgAfcuacuususc | 218 | asAfsguaGfucucgaaAfgUfagcsgsc | 204 |
| N-FY001002M26 | gscsuacuUfuCfgAfgAfcuacuususc | 219 | asAfsguaGfucucgaaAfgUfagcsgsc | 204 |
| N-FY001021M3 | csusacuuUfcGfAfGfacuacuuusTsT | 77 | P1asAfsaguAfgUfCfucgaAfaGfuagsTsT | 220 |
| N-FY001021M6 | csusacuuUfcGfAfGfacuacuuusTsT | 77 | asAfsaguAfgucucgaAfaGfuagsTsT | 221 |
| N-FY001021M6D2 | csusacuuUfcGfAfGfacuacuuu | 222 | asAfsaguAfgucucgaAfaGfuagsTsT | 221 |
| N-FY001021M7 | csusacuuUfcGfAfGfacuacuuusTsT | 77 | asAfsaguAfgUfCfucgaAfaGfuagsTsT | 223 |
| N-FY001021M7D2 | csusacuuUfcGfAfGfacuacuuu | 222 | asAfsaguAfgUfCfucgaAfaGfuagsTsT | 223 |

(continued)

| siRNA ID | Sense strand: 5'-3' | SEQ ID NO | Antisense strand: 5'-3' | SEQ ID NO |
|---|---|---|---|---|
| N-FY001021M8 | csusacuuUfcGfAfGfacuacuuusTsT | 77 | EVPasAfsaguAfgucucgaAfaGfuagsTsT | 224 |
| N-FY001021M9 | csusacuuUfcGfAfGfacuacuuusTsT | 77 | EVPasAfsaguAfgUfCfucgaAfaGfuagsTsT | 225 |
| N-FY001021M10 | csusacuuUfcGfAfGfacuacuuusTsT | 77 | asAfsaguAfgucucgaAfaguagsTsT | 226 |
| N-FY001021M11 | csusacUfuUfCfGfagacuacuuusTsT | 227 | asAfsaguAfgucucgaAfaGfuagsTsT | 221 |
| N-FY001021M12 | csusacuuucGfAfGfacuacuuusTsT | 228 | asAfsaguAfgucucgaAfaGfuagsTsT | 221 |
| N-FY001021M13 | csusacuuuCfGfAfgacuacuuusTsT | 229 | asAfsaguAfgucucgaAfaGfuagsTsT | 221 |
| N-FY001021M14 | csusacuuUfcGfAfGfacuacuuusTsT | 77 | asAfsAfgUfagUfcUfcgaAfaGfuAfgsTsT | 230 |
| N-FY001021M15 | csusacuuUfcGfAfGfacuacuuusTsT | 77 | asAfsAfGfUfaGfucUfcgaAfaguagsTsT | 231 |
| N-FY001021M16 | csusacuuuCfGfAfGfacuacuuusTsT | 232 | asAfsAfGfUfaGfucUfcgaAfaguagsTsT | 231 |
| N-FY001021M17 | csusacuuUfcGfAfGfacuacuuusTsT | 77 | asAfsaGfuAfgucucGfaAfaGfuAfgsTsT | 233 |
| N-FY001021M18 | csusacuuUfcGfAfGfacuacuuusTsT | 77 | asAfsaguagucucgaAfaguagsTsT | 361 |
| N-FY001021M19 | csusacuuUfcGfadGacuacuuusTsT | 234 | asAfsaGfUfAfgUfcUfcGfaAfaGfuAfgsTsT | 235 |
| N-FY001021M20 | csusacuuUfcGfadGacuacuuusTsT | 234 | asAfsaguAfgucucgaAfaGfuagsTsT | 221 |
| N-FY001021M21 | csusacuuUfcGfaGfacuacuuusTsT | 236 | asAfsaguAfgucucgaAfaGfuagsTsT | 221 |
| N-FY001021M22 | csusacuuUfcgaGfaCfuAfcuuusTsT | 237 | asAfsaguAfgucucgaAfaGfuagsTsT | 221 |
| N-FY001021M23 | csusacuuuCfGfAfgAfcuacuuusTsT | 238 | asAfsaguAfgucucgaAfaGfuagsTsT | 221 |
| N-FY001021M24 | csusacuuUfcGfaGfaCfuAfcuuusTsT | 239 | asAfsaguAfgucucgaAfaGfuagsTsT | 221 |
| N-FY001021M25 | csusacuuucGfaGfaCfuacuuusTsT | 240 | asAfsaguAfgucucgaAfaGfuagsTsT | 221 |

(continued)

| siRNA ID | Sense strand: 5'-3' | SEQ ID NO | Antisense strand: 5'-3' | SEQ ID NO |
|---|---|---|---|---|
| N-FY001021M26 | csusacuuUfcGfaGfaCfuacuuusTsT | 241 | asAfsaguAfgucucgaAfaGfuagsTsT | 221 |
| N-FY001021M27 | csusAfcuuUfCfGfagacuacuuusTsT | 242 | asAfsaguAfgucucgaAfaGfuagsTsT | 221 |
| N-FY001021M28 | csusAfcuuucGfaGfaCfuacuuusTsT | 243 | asAfsaguAfgucucgaAfaGfuagsTsT | 221 |
| N-FY001021M29 | csusAfcuuUfcGfaGfacuacuuusTsT | 244 | asAfsaguAfgucucgaAfaGfuagsTsT | 221 |
| N-FY001021M30 | csusacuuUfcgaGfaCfuacuuusTsT | 245 | asAfsaguAfgucucgaAfaGfuagsTsT | 221 |
| N-FY001021M31 | csusacuuUfcgaGfAfCfuacuuusTsT | 246 | asAfsaguAfgucucgaAfaGfuagsTsT | 221 |
| N-FY001021M32 | csusAfcuuUfcgaGfacuaCfuuusTsT | 247 | asAfsaguAfgucucgaAfaGfuagsTsT | 221 |
| N-FY001021M33 | csusAfcuuUfcgaGfacuacUfuusTsT | 248 | asAfsaguAfgucucgaAfaGfuagsTsT | 221 |
| N-FY001021M34 | csusAfcuuUfcgaGfacuaCfUfuusTsT | 249 | asAfsaguAfgucucgaAfaGfuagsTsT | 221 |
| N-FY001021M35 | csusacuuUfcGfAfGfacuacuuusTsT | 77 | asAfsAfGfUf***A***GfucUfcgaAfaguagsTsT | 250 |

Table 1-1

| siRNA ID | Sense strand: 5'-3' | SEQ ID NO | Antisense strand: 5'-3' | SEQ ID NO |
|---|---|---|---|---|
| siRNA1 | GACUAUUUGCUUUCAAAGA | 251 | UCUUUGAAAGCAAAUAGUC | 252 |
| siRNA2 | GCUACUUUCGAGACUACUU | 253 | AAGUAGUCUCGAAAGUAGC | 254 |
| siRNA3 | ACCAUAGACUAUUUGCUUU | 42 | AAAGCAAAUAGUCUAUGGU | 128 |
| siRNA4 | CCAUCCUCAUGUACAUAUU | 23 | AAUAUGUACAUGAGGAUGG | 24 |
| siRNA5 | CCAUCCUCAUGUACAUAUA | 31 | UAUAUGUACAUGAGGAUGG | 32 |
| siRNA6 | UGCCAUCCUCAUGUACAUA | 47 | UAUGUACAUGAGGAUGGCA | 48 |
| siRNA7 | GCCAUCCUCAUGUACAUAU | 255 | AUAUGUACAUGAGGAUGGC | 256 |
| siRNA8 | CAUCCUCAUGUACAUAUUA | 49 | UAAUAUGUACAUGAGGAUG | 50 |
| siRNA9 | CCUCAUGUACAUAUUCUGA | 55 | UCAGAAUAUGUACAUGAGG | 56 |
| siRNA10 | CUCAUGUACAUAUUCUGCACU | 257 | AGUGCAGAAUAUGUACAUG AG | 258 |
| siRNA11 | CUCAUGUACAUAUUCUGCA | 57 | UGCAGAAUAUGUACAUGAG | 58 |
| siRNA12 | UGUGGCGCUACUUUCGAGA | 59 | UCUCGAAAGUAGCGCCACA | 60 |
| siRNA13 | GUGGCGCUACUUUCGAGAA | 61 | UUCUCGAAAGUAGCGCCAC | 62 |
| siRNA14 | GCGCUACUUUCGAGACUAA | 259 | UUAGUCUCGAAAGUAGCGC | 260 |
| siRNA15 | CGCUACUUUCGAGACUACU | 261 | AGUAGUCUCGAAAGUAGCG | 262 |

(continued)

| siRNA ID | Sense strand: 5'-3' | SEQ ID NO | Antisense strand: 5'-3' | SEQ ID NO |
|---|---|---|---|---|
| siRNA16 | CUACUUUCGAGACUACUUU | 75 | AAAGUAGUCUCGAAAGUAG | 76 |
| siRNA17 | UACUUUCGAGACUACUUUA | 263 | UAAAGUAGUCUCGAAAGUA | 264 |
| siRNA18 | CUUUCGAGACUACUUUCCCAU | 265 | AUGGGAAAGUAGUCUCGAAAG | 266 |
| siRNA19 | ACACCAUAGACUAUUUGCU | 267 | AGCAAAUAGUCUAUGGUGU | 268 |
| siRNA20 | CACCAUAGACUAUUUGCUU | 269 | AAGCAAAUAGUCUAUGGUG | 270 |
| siRNA21 | ACCAUAGACUAUUUGCUUUCA | 15 | UGAAAGCAAAUAGUCUAUGGU | 271 |
| siRNA22 | CCAUAGACUAUUUGCUUUCAA | 272 | UUGAAAGCAAAUAGUCUAUGG | 273 |
| siRNA23 | CCAUAGACUAUUUGCUUUA | 131 | UAAAGCAAAUAGUCUAUGG | 132 |
| siRNA24 | UAGACUAUUUGCUUUCAAA | 274 | UUUGAAAGCAAAUAGUCUA | 275 |
| siRNA25 | AGACUAUUUGCUUUCAAAA | 276 | UUUUGAAAGCAAAUAGUCU | 277 |
| siRNA26 | ACUAUUUGCUUUCAAAGAA | 163 | UUCUUUGAAAGCAAAUAGU | 164 |
| siRNA27 | CUAUUUGCUUUCAAAGAAU | 165 | AUUCUUUGAAAGCAAAUAG | 166 |
| siRNA28 | UUUGCUUUCAAAGAAUGGGAA | 278 | UUCCCAUUCUUUGAAAGCAAA | 279 |

The uppercase letters "G", "C", "A", "T", and "U" each generally represent nucleotides containing guanine, cytosine, adenine, thymine, and uracil as a base, respectively; the lowercase letters a, u, c, and g: represent 2'-methoxy-modified nucleotides; Af, Gf, Cf, and Uf: represent 2'-fluoro-modified nucleotides; d: indicates that the nucleotide adjacent to the right side of the letter d is a 2'-deoxyribonucleotide; the lowercase letter s indicates that the two nucleotides adjacent to the left and right sides of the letter s are linked by a phosphorothioate group; P1: indicates that the nucleotide adjacent to the right side of P1 is a 5'-phosphate nucleotide; EVP: indicates that the nucleotide adjacent to the right side of EVP is a 5'-(E)-vinylphosphonate nucleotide; ***A***, ***U***, ***C,*** and ***G*** (underlined + bold + italic) represent GNA-modified nucleotides.

siRNA conjugates having the following sequences were synthesized by Shanghai Talen-bio Scientific Co.,Ltd:

Table 2. siRNA conjugates and sequences thereof:

| siRNA ID | Sense strand: 5'→3' | Antisense strand: 5'→3' |
|---|---|---|
| N-FY001001M2L96 | gsascuauUfuGfCfUfuucaaagaL96(SEQ ID NO: 280) | P1usCfsuuuGfaaagcaaAfuAfgucsusa(SEQ ID NO: 4) |
| N-FY001001M3L96 | gsascuauUfuGfCfUfuucaaagaL96(SEQ ID NO: 280) | P1usCfsuuuGfaAfAfgcaaAfuAfgucsusa(SEQ ID NO: 5) |
| N-FY001002M2L96 | gscsuacuUfuCfGfAfgacuacuuL96(SEQ ID NO: 281) | P1asAfsguaGfucucgaaAfgUfagcsgsc(SEQ ID NO: 11) |
| N-FY001002M3L96 | gscsuacuUfuCfGfAfgacuacuuL96(SEQ ID NO: 281) | P1asAfsguaGfuCfUfcgaaAfgUfagcsgsc(SEQ ID NO: 12) |
| N-FY001003M2L96 | ascscauaGfaCfUfAfuuugcuuuL96(SEQ ID NO: 282) | P1asAfsagcAfaauagucUfaUfggusgsu(SEQ ID NO: 18) |
| N-FY001003M3L96 | ascscauaGfaCfUfAfuuugcuuuL96(SEQ ID NO: 282) | P1asAfsagcAfaAfUfagucUfaUfggusgsu(SEQ ID NO: 20) |
| N-FY001004M2L96 | cscsauccUfcAfUfGfuacauauuL96(SEQ ID NO: 283) | P1asAfsuauGfuacaugaGfgAfuggsTsT(SEQ ID NO: 26) |
| N-FY001004M3L96 | cscsauccUfcAfUfGfuacauauuL96(SEQ ID NO: 283) | P1asAfsuauGfuAfCfaugaGfgAfuggsTsT(SE Q ID NO: 28) |
| N-FY001005M2L96 | cscsauccUfcAfUfGfuacauauaL96(SEQ ID NO: 284) | P1usAfsuauGfuacaugaGfgAfuggsTsT(SEQ ID NO: 34) |
| N-FY001005M3L96 | cscsauccUfcAfUfGfuacauauaL96(SEQ ID NO: 284) | P1usAfsuauGfuAfCfaugaGfgAfuggsTsT(SE Q ID NO: 35) |
| N-FY001003M6L96 | ascscauaGfaCfUfAfuuugcuuuL96(SEQ ID NO: 282) | asAfsagcAfaauagucUfaUfggusgsu(SEQ ID NO: 38) |
| N-FY001003M7L96 | ascscauaGfaCfUfAfuuugcuuuL96(SEQ ID NO: 282) | asAfsagcAfaAfUfagucUfaUfggusgsu(SEQ ID NO: 39) |
| N-FY001003M8L96 | ascscauaGfaCfUfAfuuugcuuuL96(SEQ ID NO: 282) | EVPasAfsagcAfaauagucUfaUfggusgsu(SEQ ID NO: 40) |
| N-FY001003M9L96 | ascscauaGfaCfUfAfuuugcuuuL96(SEQ ID NO: 282) | EVPasAfsagcAfaAfUfagucUfaUfggusgsu(S EQ ID NO: 41) |
| N-FY001021M2L96 | csusacuuUfcGfAfGfacuacuuuL96(SEQ ID NO: 285) | P1asAfsaguAfgucucgaAfaGfuagsTsT(SEQ ID NO: 78) |
| N-FY001021M4L96 | csusacuuUfcGfAfGfacuacuuuL96(SEQ ID NO: 285) | P1asAfsagu***A***gucucgaAfaGfuagsTsT(SEQ ID NO: 79) |
| N-FY001021M5L96 | csusacuuUfcGfAfGfacuacuuuL96(SEQ ID NO: 285) | P1asAfsaguAf***G***ucucgaAfaGfuagsTsT(SEQ ID NO: 80) |
| N-FY001022M2L96 | csusuucgAfgAfCfUfacuuucccauL96(SE Q ID NO: 286) | P1asUfsgggAfaaguaguCfuCfgaaagsusa(SEQ ID NO: 84) |
| N-FY001022M4L96 | csusuucgAfgAfCfUfacuuucccauL96(SE Q ID NO: 286) | P1asUfsggg***A***aaguaguCfuCfgaaagsusa(SEQ ID NO: 85) |
| N-FY001022M5L96 | csusuucgAfgAfCfUfacuuucccauL96(SE Q ID NO: 286) | P1asUfsgggAf***A***aguaguCfuCfgaaagsusa(SEQ ID NO: 86) |
| N-FY001025M2L96 | ascsaccaUfaGfAfCfuauuugcuL96(SEQ ID NO: 287) | P1asGfscaaAfuagucuaUfgGfuguscsc(SEQ ID NO: 94) |
| N-FY001025M3L96 | ascsaccaUfaGfAfCfuauuugcuL96(SEQ ID NO: 287) | P1asGfscaaAfuAfGfucuaUfgGfuguscsc(SEQ ID NO: 95) |
| N-FY001025M4L96 | ascsaccaUfaGfAfCfuauuugcuL96(SEQ ID NO: 287) | P1asGfscaa***A***uagucuaUfgGfuguscsc(SEQ ID NO: 96) |
| N-FY001025M5L96 | ascsaccaUfaGfAfCfuauuugcuL96(SEQ ID NO: 287) | P1asGfscaaAf***U***agucuaUfgGfuguscsc(SEQ ID NO: 97) |
| N-FY001025M6L96 | ascsaccaUfaGfAfCfuauuugcuL96(SEQ ID NO: 287) | asGfscaaAfuagucuaUfgGfuguscsc(SEQ ID NO: 98) |
| N-FY001025M7L96 | ascsaccaUfaGfAfCfuauuugcuL96(SEQ ID NO: 287) | asGfscaaAfuAfGfucuaUfgGfuguscsc(SEQ ID NO: 99) |

(continued)

| siRNA ID | Sense strand: 5'→3' | Antisense strand: 5'→3' |
|---|---|---|
| N-FY001025M8L96 | ascsaccaUfaGfAfCfuauuugcuL96(SEQ ID NO: 287) | EVPasGfscaaAfuagucuaUfgGfuguscsc(SEQ ID NO: 100) |
| N-FY001025M9L96 | ascsaccaUfaGfAfCfuauuugcuL96(SEQ ID NO: 287) | EVPasGfscaaAfuAfGfucuaUfgGfuguscsc(S EQ ID NO: 101) |
| N-FY001026M2L96 | csasccauAfgAfCfUfauuugcuuL96(SEQ ID NO: 288) | P1asAfsgcaAfauagucuAfuGfgugsusc(SEQ ID NO: 105) |
| N-FY001026M3L96 | csasccauAfgAfCfUfauuugcuuL96(SEQ ID NO: 288) | P1asAfsgcaAfaUfAfgucuAfuGfgugsusc(SE Q ID NO: 106) |
| N-FY001026M4L96 | csasccauAfgAfCfUfauuugcuuL96(SEQ ID NO: 288) | P1asAfsgca***A***auagucuAfuGfgugsusc(SEQ ID NO: 107) |
| N-FY001026M5L96 | csasccauAfgAfCfUfauuugcuuL96(SEQ ID NO: 288) | P1asAfsgca***A***fAuagucuAfuGfgugsusc(SEQ ID NO: 108) |
| N-FY001026M6L96 | csasccauAfgAfCfUfauuugcuuL96(SEQ ID NO: 288) | asAfsgcaAfauagucuAfuGfgugsusc(SEQ ID NO: 109) |
| N-FY001026M7L96 | csasccauAfgAfCfUfauuugcuuL96(SEQ ID NO: 288) | asAfsgcaAfaUfAfgucuAfuGfgugsusc(SEQ ID NO: 110) |
| N-FY001026M8L96 | csasccauAfgAfCfUfauuugcuuL96(SEQ ID NO: 288) | EVPasAfsgcaAfauagucuAfuGfgugsusc(SEQ ID NO: 111) |
| N-FY001026M9L96 | csasccauAfgAfCfUfauuugcuuL96(SEQ ID NO: 288) | EVPasAfsgcaAfaUfAfgucuAfuGfgugsusc(S EQ ID NO: 112) |
| N-FY001027M2L96 | ascscauaGfaCfUfAfuuugcuuucaL96(SE Q ID NO: 289) | P1usGfsaaaGfcaaauagUfcUfauggusgsu(SEQ ID NO: 116) |
| N-FY001027M3L96 | ascscauaGfaCfUfAfuuugcuuucaL96(SEQ ID NO: 289) | P1usGfsaaaGfcAfAfauagUfcUfauggusgsu(SEQ ID NO: 117) |
| N-FY001027M4L96 | ascscauaGfaCfUfAfuuugcuuucaL96( SEQ ID NO: 289) | P1usGfsaaa***G***caaauagUfcUfauggusgsu(SEQ ID NO: 118) |
| N-FY001027M5L96 | ascscauaGfaCfUfAfuuugcuuucaL96( SEQ ID NO: 289) | P1usGfsaaaGf***C***aaauagUfcUfauggusgsu(SEQ ID NO: 119) |
| N-FY001027M6L96 | ascscauaGfaCfUfAfuuugcuuucaL96( SEQ ID NO: 289) | usGfsaaaGfcaaauagUfcUfauggusgsu(SEQ ID NO: 120) |
| N-FY001027M7L96 | ascscauaGfaCfUfAfuuugcuuucaL96( SEQ ID NO: 289) | usGfsaaaGfcAfAfauagUfcUfauggusgsu(SEQ ID NO: 121) |
| N-FY001027M8L96 | ascscauaGfaCfUfAfuuugcuuucaL96( SEQ ID NO: 289) | EVPusGfsaaaGfcaaauagUfcUfauggusgsu( SEQ ID NO: 122) |
| N-FY001027M9L96 | ascscauaGfaCfUfAfuuugcuuucaL96( SEQ ID NO: 289) | EVPusGfsaaaGfcAfAfauagUfcUfauggusgsu( SEQ ID NO: 123) |
| N-FY001031M2L96 | usasgacuAfuUfUfGfcuuucaaaL96(SEQ ID NO: 290) | P1usUfsugaAfagcaaauAfgUfcuasusg(SEQ ID NO: 138) |
| N-FY001031M3L96 | usasgacuAfuUfUfGfcuuucaaaL96(SEQ ID NO: 290) | P1usUfsugaAfaGfCfaaauAfgUfcuasusg(SEQ ID NO: 139) |
| N-FY001031M4L96 | usasgacuAfuUfUfGfcuuucaaaL96(SEQ ID NO: 290) | P1usUfsuga***A***agcaaauAfgUfcuasusg(SEQ ID NO: 140) |
| N-FY001031M5L96 | usasgacuAfuUfUfGfcuuucaaaL96(SEQ ID NO: 290) | P1usUfsugaAf***A***gcaaauAfgUfcuasusg(SEQ ID NO: 141) |
| N-FY001031M6L96 | usasgacuAfuUfUfGfcuuucaaaL96(SEQ ID NO: 290) | usUfsugaAfagcaaauAfgUfcuasusg(SEQ ID NO: 142) |
| N-FY001031M7L96 | usasgacuAfuUfUfGfcuuucaaaL96(SEQ ID NO: 290) | usUfsugaAfaGfCfaaauAfgUfcuasusg(SEQ ID NO: 143) |
| N-FY001031M8L96 | usasgacuAfuUfUfGfcuuucaaaL96(SEQ ID NO: 290) | EVPusUfsugaAfagcaaauAfgUfcuasusg(SEQ ID NO: 144) |
| N-FY001031M9L96 | usasgacuAfuUfUfGfcuuucaaaL96(SEQ ID NO: 290) | EVPusUfsugaAfaGfCfaaauAfgUfcuasusg( SEQ ID NO: 145) |

(continued)

| siRNA ID | Sense strand: 5'→3' | Antisense strand: 5'→3' |
|---|---|---|
| N-FY001033M2L96 | ascsuauuUfgCfUfUfucaaagaaL96(SEQ ID NO: 291) | P1usUfscuuUfgaaagcaAfaUfaguscsu(SEQ ID NO: 153) |
| N-FY001033M3L96 | ascsuauuUfgCfUfUfucaaagaaL96(SEQ ID NO: 291) | P1usUfscuuUfgAfAfagcaAfaUfaguscsu(SEQ ID NO: 154) |
| N-FY001033M4L96 | ascsuauuUfgCfUfUfucaaagaaL96(SEQ ID NO: 291) | P1usUfscuu***U***gaaagcaAfaUfaguscsu(SEQ ID NO: 155) |
| N-FY001033M5L96 | ascsuauuUfgCfUfUfucaaagaaL96(SEQ ID NO: 291) | P1usUfscuuUf***G***aaagcaAfaUfaguscsu(SEQ ID NO: 156) |
| N-FY001033M6L96 | ascsuauuUfgCfUfUfucaaagaaL96(SEQ ID NO: 291) | usUfscuuUfgaaagcaAfaUfaguscsu(SEQ ID NO: 157) |
| N-FY001033M7L96 | ascsuauuUfgCfUfUfucaaagaaL96(SEQ ID NO: 291) | usUfscuuUfgAfAfagcaAfaUfaguscsu(SEQ ID NO: 158) |
| N-FY001033M8L96 | ascsuauuUfgCfUfUfucaaagaaL96(SEQ ID NO: 291) | EVPusUfscuuUfgaaagcaAfaUfaguscsu(SEQ ID NO: 159) |
| N-FY001033M9L96 | ascsuauuUfgCfUfUfucaaagaaL96(SEQ ID NO: 291) | EVPusUfscuuUfgAfAfagcaAfaUfaguscsu(SEQ ID NO: 160) |
| N-FY001003M10L96 | ascscauaGfaCfUfAfuuugcuuuL96(SEQ ID NO: 282) | asAfsagcAfaauagucUfauggusgsu(SEQ ID NO: 169) |
| N-FY001003M11L96 | ascscaUfaGfAfCfuauuugcuuuL96(SEQ ID NO: 292) | asAfsagcAfaauagucUfaUfggusgsu(SEQ ID NO: 38) |
| N-FY001003M12L96 | ascscauagaCfUfAfuuugcuuuL96(SEQ ID NO: 293) | asAfsagcAfaauagucUfaUfggusgsu(SEQ ID NO: 38) |
| N-FY001003M13L96 | ascscauagAfCfUfauuugcuuuL96(SEQ ID NO: 294) | asAfsagcAfaauagucUfaUfggusgsu(SEQ ID NO: 38) |
| N-FY001003M14L96 | ascscauaGfaCfUfAfuuugcuuuL96(SEQ ID NO: 282) | asAfsAfgCfaaAfuAfgucUfaUfgGfusgsu( SEQ ID NO: 173) |
| N-FY001003M15L96 | ascscauaGfaCfUfAfuuugcuuuL96(SEQ ID NO: 282) | asAfsAfGfCfaAfauAfgucUfauggusgsu(SEQ ID NO: 174) |
| N-FY001003M16L96 | ascscauagAfCfUfAfuuugcuuuL96(SEQ ID NO: 295) | asAfsAfGfCfaAfauAfgucUfauggusgsu(SEQ ID NO: 174) |
| N-FY001003M17L96 | ascscauaGfaCfUfAfuuugcuuuL96(SEQ ID NO: 282) | asAfsaGfcAfaauagUfcUfaUfgGfusGfsu(SEQ ID NO: 176) |
| N-FY001003M18L96 | ascscauaGfaCfUfAfuuugcuuuL96(SEQ ID NO: 282) | asAfsagcaaauagucUfauggusgsu(SEQ ID NO: 177) |
| N-FY001003M19L96 | ascscauaGfaCfudAuuugcuuuL96(SEQ ID NO: 296) | asAfsaGfCfAfaAfuAfgUfcUfaUfgGfusGfsu( SEQ ID NO: 179) |
| N-FY001003M20L96 | ascscauaGfaCfudAuuugcuuuL96(SEQ ID NO: 296) | asAfsagcAfaauagucUfaUfggusgsu(SEQ ID NO: 38) |
| N-FY001003M21L96 | ascscauaGfaCfuAfuuugcuuuL96(SEQ ID NO: 297) | asAfsagcAfaauagucUfaUfggusgsu(SEQ ID NO: 38) |
| N-FY001003M22L96 | ascscauaGfacuAfuUfuGfcuuuL96(SEQ ID NO: 298) | asAfsagcAfaauagucUfaUfggusgsu(SEQ ID NO: 38) |
| N-FY001003M23L96 | ascscauagAfCfUfaUfuugcuuuL96(SEQ ID NO: 299) | asAfsagcAfaauagucUfaUfggusgsu(SEQ ID NO: 38) |
| N-FY001003M24L96 | ascscauaGfaCfuAfuUfuGfcuuuL96( SEQ ID NO: 300) | asAfsagcAfaauagucUfaUfggusgsu(SEQ ID NO: 38) |
| N-FY001003M25L96 | ascscauagaCfuAfuUfugcuuuL96(SEQ ID NO: 301) | asAfsagcAfaauagucUfaUfggusgsu(SEQ ID NO: 38) |
| N-FY001003M26L96 | ascscauaGfaCfuAfuUfugcuuuL96(SEQ ID NO: 302) | asAfsagcAfaauagucUfaUfggusgsu(SEQ ID NO: 38) |
| N-FY001003M27L96 | ascsCfauaGfAfCfuauuugcuuuL96(SEQ ID NO: 303) | asAfsagcAfaauagucUfaUfggusgsu(SEQ ID NO: 38) |

(continued)

| siRNA ID | Sense strand: 5'→3' | Antisense strand: 5'→3' |
|---|---|---|
| N-FY001003M28L96 | ascsCfauagaCfuAfuUfugcuuuL96(SEQ ID NO: 304) | asAfsagcAfaauagucUfaUfggusgsu(SEQ ID NO: 38) |
| N-FY001003M29L96 | ascsCfauaGfaCfuAfuuugcuuuL96(SEQ ID NO: 305) | asAfsagcAfaauagucUfaUfggusgsu(SEQ ID NO: 38) |
| N-FY001003M30L96 | ascscauaGfacuAfuUfugcuuuL96(SEQ ID NO: 306) | asAfsagcAfaauagucUfaUfggusgsu(SEQ ID NO: 38) |
| N-FY001003M31L96 | ascscauaGfacuAfUfUfugcuuuL96(SEQ ID NO: 307) | asAfsagcAfaauagucUfaUfggusgsu(SEQ ID NO: 38) |
| N-FY001003M32L96 | ascsCfauaGfacuAfuuugCfuuuL96(SEQ ID NO: 308) | asAfsagcAfaauagucUfaUfggusgsu(SEQ ID NO: 38) |
| N-FY001003M33L96 | ascsCfauaGfacuAfuuugcUfuuL96(SEQ ID NO: 309) | asAfsagcAfaauagucUfaUfggusgsu(SEQ ID NO: 38) |
| N-FY001003M34L96 | ascsCfauaGfacuAfuuugCfUfuuL96( SEQ ID NO: 310) | asAfsagcAfaauagucUfaUfggusgsu(SEQ ID NO: 38) |
| N-FY001003M35L96 | ascscauaGfaCfUfAfuuugcuuuL96(SEQ ID NO: 282) | asAfsAfGfCf***A***AfauAfgucUfauggusgsu(SEQ ID NO: 199) |
| N-FY001003M37L96 | ascscauaGfaCfUfAfuuugcuuuL96(SEQ ID NO: 282) | asAfsagcaAfauAfgucUfauggusgsu(SEQ ID NO: 200) |
| N-FY001003M40L96 | ascscauaGfaCfUfAfuuugcuuuL96(SEQ ID NO: 282) | asAfsagCfaAfauagucUfauggusgsu(SEQ ID NO: 201) |
| N-FY001003M44L96 | ascsCfauaGfAfCfuauuugcuuuL96(SEQ ID NO: 303) | asAfsagcaAfauAfgucUfauggusgsu(SEQ ID NO: 200) |
| N-FY001003M45L96 | ascsCfauaGfaCfuAfuuugcuuuL96(SEQ ID NO: 305) | asAfsagcaAfauAfgucUfauggusgsu(SEQ ID NO: 200) |
| N-FY001002M10L96 | gscsuacuUfuCfGfAfgacuacuuL96(SEQ ID NO: 281) | asAfsguaGfucucgaaAfguagcsgsc(SEQ ID NO: 202) |
| N-FY001002M11L96 | gscsuaCfuUfUfCfgagacuacuuL96(SEQ ID NO: 311) | asAfsguaGfucucgaaAfgUfagcsgsc(SEQ ID NO: 204) |
| N-FY001002M12L96 | gscsuacuuuCfGfAfgacuacuuL96(SEQ ID NO: 312) | asAfsguaGfucucgaaAfgUfagcsgsc(SEQ ID NO: 204) |
| N-FY001002M13L96 | gscsuacuuUfCfGfagacuacuuL96(SEQ ID NO: 360) | asAfsguaGfucucgaaAfgUfagcsgsc(SEQ ID NO: 204) |
| N-FY001002M14L96 | gscsuacuUfuCfGfAfgacuacuuL96(SEQ ID NO: 281) | asAfsGfuAfguCfuCfgaaAfgUfaGfesgsc(SEQ ID NO: 207) |
| N-FY001002M15L96 | gscsuacuUfuCfGfAfgacuacuuL96(SEQ ID NO: 281) | asAfsGfUfAfgUfcuCfgaaAfguagcsgsc(SEQ ID NO: 208) |
| N-FY001002M16L96 | gscsuacuuUfCfGfAfgacuacuuL96(SEQ ID NO: 313) | asAfsGfUfAfgUfcuCfgaaAfguagcsgsc(SEQ ID NO: 208) |
| N-FY001002M17L96 | gscsuacuUfuCfGfAfgacuacuuL96(SEQ ID NO: 281) | asAfsgUfaGfucucgAfaAfgUfaGfcsGfsc(SEQ ID NO: 210) |
| N-FY001002M18L96 | gscsuacuUfuCfGfAfgacuacuuL96(SEQ ID NO: 281) | asAfsguagucucgaaAfguagcsgsc(SEQ ID NO: 211) |
| N-FY001002M19L96 | gscsuacuUfuCfgdAgacuacuuL96(SEQ ID NO: 314) | asAfsgUfAfGfuCfuCfgAfaAfgUfaGfcsGfsc( SEQ ID NO: 213) |
| N-FY001002M20L96 | gscsuacuUfuCfgdAgacuacuuL96(SEQ ID NO: 314) | asAfsguaGfucucgaaAfgUfagcsgsc(SEQ ID NO: 204) |
| N-FY001002M21L96 | gscsuacuUfuCfgAfgacuacuuL96(SEQ ID NO: 315) | asAfsguaGfucucgaaAfgUfagcsgsc(SEQ ID NO: 204) |
| N-FY001002M22L96 | gscsuacuUfucgAfgAfcUfacuuL96(SEQ ID NO: 316) | asAfsguaGfucucgaaAfgUfagcsgsc(SEQ ID NO: 204) |
| N-FY001002M23L96 | gscsuacuuUfCfGfaGfacuacuuL96(SEQ ID NO: 317) | asAfsguaGfucucgaaAfgUfagcsgsc(SEQ ID NO: 204) |

(continued)

| siRNA ID | Sense strand: 5'→3' | Antisense strand: 5'→3' |
|---|---|---|
| N-FY001002M24L96 | gscsuacuUfuCfgAfgAfcUfacuuL96( SEQ ID NO: 318) | asAfsguaGfucucgaaAfgUfagcsgsc(SEQ ID NO: 204) |
| N-FY001002M25L96 | gscsuacuuuCfgAfgAfcuacuuL96(SEQ ID NO: 319) | asAfsguaGfucucgaaAfgUfagcsgsc(SEQ ID NO: 204) |
| N-FY001002M26L96 | gscsuacuUfuCfgAfgAfcuacuuL96(SEQ ID NO: 320) | asAfsguaGfucucgaaAfgUfagcsgsc(SEQ ID NO: 204) |
| N-FY001021M3L96 | csusacuuUfcGfAfGfacuacuuuL96(SEQ ID NO: 285) | P1asAfsaguAfgUfCfucgaAfaGfuagsTsT( SEQ ID NO: 220) |
| N-FY001021M6L96 | csusacuuUfcGfAfGfacuacuuuL96(SEQ ID NO: 285) | asAfsaguAfgucucgaAfaGfuagsTsT(SEQ ID NO: 221) |
| N-FY001021M7L96 | csusacuuUfcGfAfGfacuacuuuL96(SEQ ID NO: 285) | asAfsaguAfgUfCfucgaAfaGfuagsTsT(SEQ ID NO: 223) |
| N-FY001021M8L96 | csusacuuUfcGfAfGfacuacuuuL96(SEQ ID NO: 285) | EVPasAfsaguAfgucucgaAfaGfuagsTsT(SEQ ID NO: 224) |
| N-FY001021M9L96 | csusacuuUfcGfAfGfacuacuuuL96(SEQ ID NO: 285) | EVPasAfsaguAfgUfCfucgaAfaGfuagsTsT( SEQ ID NO: 225) |
| N-FY001021M10L96 | csusacuuUfcGfAfGfacuacuuuL96(SEQ ID NO: 285) | asAfsaguAfgucucgaAfaguagsTsT(SEQ ID NO: 226) |
| N-FY001021M11L96 | csusacUfuUfCfGfagacuacuuuL96(SEQ ID NO: 321) | asAfsaguAfgucucgaAfaGfuagsTsT(SEQ ID NO: 221) |
| N-FY001021M12L96 | csusacuuucGfAfGfacuacuuuL96(SEQ ID NO: 322) | asAfsaguAfgucucgaAfaGfuagsTsT(SEQ ID NO: 221) |
| N-FY001021M13L96 | csusacuuuCfGfAfgacuacuuuL96(SEQ ID NO: 323) | asAfsaguAfgucucgaAfaGfuagsTsT(SEQ ID NO: 221) |
| N-FY001021M14L96 | csusacuuUfcGfAfGfacuacuuuL96(SEQ ID NO: 285) | asAfsAfgUfagUfcUfcgaAfaGfuAfgsTsT(SE Q ID NO: 230) |
| N-FY001021M15L96 | csusacuuUfcGfAfGfacuacuuuL96(SEQ ID NO: 285) | asAfsAfGfUfaGfucUfcgaAfaguagsTsT(SEQ ID NO: 231) |
| N-FY001021M16L96 | csusacuuuCfGfAfGfacuacuuuL96(SEQ ID NO: 324) | asAfsAfGfUfaGfucUfcgaAfaguagsTsT(SEQ ID NO: 231) |
| N-FY001021M17L96 | csusacuuUfcGfAfGfacuacuuuL96(SEQ ID NO: 285) | asAfsaGfuAfgucucGfaAfaGfuAfgsTsT(SEQ ID NO: 233) |
| N-FY001021M18L96 | csusacuuUfcGfAfGfacuacuuuL96(SEQ ID NO: 285) | asAfsaguagucucgaAfaguagsTsT(SEQ ID NO: 361) |
| N-FY001021M19L96 | csusacuuUfcGfadGacuacuuuL96(SEQ ID NO: 325) | asAfsaGfUfAfgUfcUfcGfaAfaGfuAfgsTsT(S EQ ID NO: 235) |
| N-FY001021M20L96 | csusacuuUfcGfadGacuacuuuL96(SEQ ID NO: 325) | asAfsaguAfgucucgaAfaGfuagsTsT(SEQ ID NO: 221) |
| N-FY001021M21L96 | csusacuuUfcGfaGfacuacuuuL96(SEQ ID NO: 326) | asAfsaguAfgucucgaAfaGfuagsTsT(SEQ ID NO: 221) |
| N-FY001021M22L96 | csusacuuUfcgaGfaCfuAfcuuuL96(SEQ ID NO: 327) | asAfsaguAfgucucgaAfaGfuagsTsT(SEQ ID NO: 221) |
| N-FY001021M23L96 | csusacuuuCfGfAfgAfcuacuuuL96(SEQ ID NO: 328) | asAfsaguAfgucucgaAfaGfuagsTsT(SEQ ID NO: 221) |
| N-FY001021M24L96 | csusacuuUfcGfaGfaCfuAfcuuuL96(SEQ ID NO: 329) | asAfsaguAfgucucgaAfaGfuagsTsT(SEQ ID NO: 221) |
| N-FY001021M25L96 | csusacuuucGfaGfaCfuacuuuL96(SEQ ID NO: 330) | asAfsaguAfgucucgaAfaGfuagsTsT(SEQ ID NO: 221) |
| N-FY001021M26L96 | csusacuuUfcGfaGfaCfuacuuuL96(SEQ ID NO: 331) | asAfsaguAfgucucgaAfaGfuagsTsT(SEQ ID NO: 221) |
| N-FY001021M27L96 | csusAfcuuUfCfGfagacuacuuuL96(SEQ ID NO: 332) | asAfsaguAfgucucgaAfaGfuagsTsT(SEQ ID NO: 221) |

(continued)

| siRNA ID | Sense strand: 5'→3' | Antisense strand: 5'→3' |
|---|---|---|
| N-FY001021M28L96 | csusAfcuuucGfaGfaCfuacuuuL96(SEQ ID NO: 333) | asAfsaguAfgucucgaAfaGfuagsTsT(SEQ ID NO: 221) |
| N-FY001021M29L96 | csusAfcuuUfcGfaGfacuacuuuL96(SEQ ID NO: 334) | asAfsaguAfgucucgaAfaGfuagsTsT(SEQ ID NO: 221) |
| N-FY001021M30L96 | csusacuuUfcgaGfaCfuacuuuL96(SEQ ID NO: 335) | asAfsaguAfgucucgaAfaGfuagsTsT(SEQ ID NO: 221) |
| N-FY001021M31L96 | csusacuuUfcgaGfAfCfuacuuuL96(SEQ ID NO: 336) | asAfsaguAfgucucgaAfaGfuagsTsT(SEQ ID NO: 221) |
| N-FY001021M32L96 | csusAfcuuUfcgaGfacuaCfuuuL96(SEQ ID NO: 337) | asAfsaguAfgucucgaAfaGfuagsTsT(SEQ ID NO: 221) |
| N-FY001021M33L96 | csusAfcuuUfcgaGfacuacUfuuL96(SEQ ID NO: 338) | asAfsaguAfgucucgaAfaGfuagsTsT(SEQ ID NO: 221) |
| N-FY001021M34L96 | csusAfcuuUfcgaGfacuaCfUfuuL96( SEQ ID NO: 339) | asAfsaguAfgucucgaAfaGfuagsTsT(SEQ ID NO: 221) |
| N-FY001021M35L96 | csusacuuUfcGfAfGfacuacuuuL96(SEQ ID NO: 285) | asAfsAfGfUf***A***GfucUfcgaAfaguagsTsT(SEQ ID NO: 250) |

Table 2-1

| siRNA ID | Sense strand: 5'→3' | Antisense strand: 5'→3' |
|---|---|---|
| GalXC-DGAT2-0898 | csauagacUfAfUfUfugcuuucaagcagccg[ademA-GalNAc][ademA-GalNAc][ademA-GalNAc]ggcugc (SEQ ID NO: 340) | MePhosphonate-4O-usUfsGfAfAfaGfcaAfauaGfucuaugsgsg (SEQ ID NO: 221) |

The structural formula of L96 is as shown in formula (I) below, and L96 is linked to the 3' end of the sense strand or the blunt end formed at the 3' end of the sense strand in Table 1 via a phosphodiester bond:

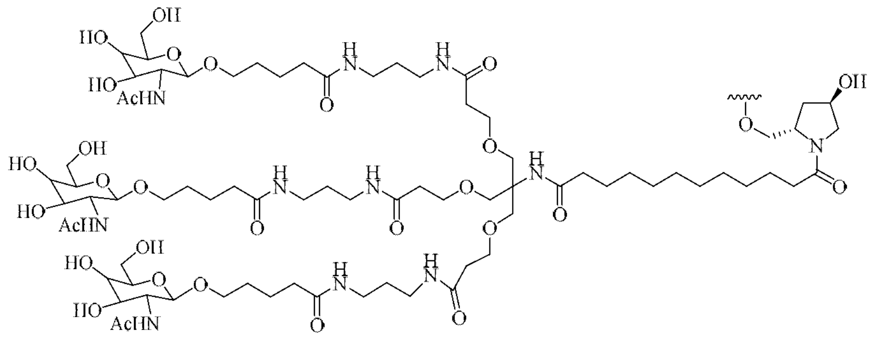

formula (I).

GalXC-DGAT2-0898 was used as a comparative conjugate, and it is disclosed in WO2022031850A2. The structural formula of [ademA-GalNAc] is as shown in formula (IX) below:

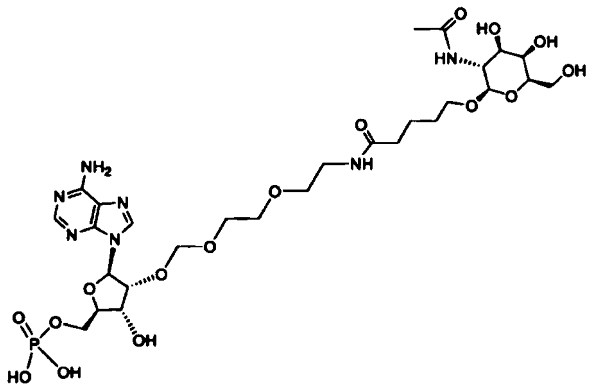

formula (IX).

The structural formula of MePhosphonate-4O-us is as shown in formula (XI) below:

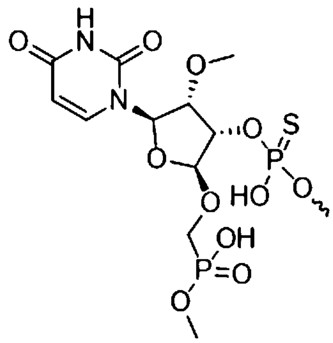

formula (XI).

In Table 1, Table 1-1, and Table 2, if the 5' end nucleotides of the sense strand, the modified sense strand, and the modified sense strand linked to a conjugated group are not labeled with P1 or EVP on their left sides, it indicates that the 5' end nucleotides are not linked to a 5' phosphate group or a 5' phosphate-derived group, and their structures are as shown in formula X.

In Table 1, Table 1-1, and Table 2, if the 5' end nucleotides of the antisense strand and the modified antisense strand are not labeled with P1 or EVP on their left sides, it indicates that the 5' end nucleotides are not linked to a 5' phosphate group or a 5' phosphate-derived group, and their structures are also as shown in formula X.

In Table 1, Table 1-1, and Table 2, the 3' positions of the 3' end nucleotides of the sense strand, the modified sense strand, the antisense strand, and the modified antisense strand are hydroxyl groups.

## Example 2. Inhibition of DGAT2 gene expression by siRNAs

2.1. Experimental materials

HepG2 cells, purchased from the Cell Bank of the Committee on Culture Collections, Chinese Academy of Sciences, Cat. No.: SCSP-510.

RNA extraction kit, Cat. No.: QIAGEN-74106.
Lipo®RNAiMAX transfection reagent, purchased from Invitrogen, Cat. No.: 13778-150.
DMEM culture medium, purchased from Gibco, Cat. No.: 11965118.
Reverse transcription kit (HiScript®III 1st Strand cDNA Synthesis Kit (+gDNA wiper)), purchased from Vazyme, Cat. No.: R312-02.
TaqMan™ gene expression premix, purchased from Applied Biosystems, Cat. No.: 4369016.
Opti-medium: purchased from Gibco, Cat. No.: 31985070.
FBS, purchased from Gibco, Cat. No.: 10099141.
PBS, purchased from MACGENE, Cat. No.: CC006.
Pancreatin, purchased from Gibco, Cat. No.: 15400054.
Target DGAT2 primer set, purchased from Shanghai Talen-bio Scientific Co.,Ltd, forward primer: GGCCTCCCG GAGACTGA (SEQ ID NO: 341); reverse primer: AAGTGATTTGCAGCTGGTTCCT (SEQ ID NO: 342).
TaqMan Gene Expression Assay (GAPDH), purchased from Shanghai Talen-bio Scientific Co.,Ltd, forward primer: GCACCGTCAAGGCTGAGAAC (SEQ ID NO: 343); reverse primer: TGGTGAAGACGCCAGTGGA (SEQ ID NO: 344).

2.2. Experimental method

2.2.1. HepG2 cells were plated in a 96-well plate and cultured in a fresh DMEM culture medium for 24 h. The cultured cells were resuspended in a PS (penicillin-streptomycin mixed solution)-free DMEM culture medium to prepare a cell suspension with a density of $1.11 \times 10^5$ cells/mL, which was then plated in a 96-well plate, with 90 μL of the cell suspension added to each well, i.e., 10000 cells/well.

2.2.2. The dry powder of the test siRNA (for convenience of description, the siRNAs and siRNA conjugates tested in this example are referred to as siRNA) was centrifuged at low temperature and high speed and then dissolved in ultrapure distilled water to prepare a 100 μM siRNA stock solution.

2.2.3. Preparation of 0.01 nM siRNA transfection dilution

(1) Preparation of 10 nM siRNA reserve solution:

a. 2 μL of the 100 μM siRNA stock solution prepared in step 2.2.2 above was taken, and 18 μL of ultrapure distilled water was added to obtain an siRNA dilution with a final concentration of 10 μM;
b. 2 μL of the 10 μM siRNA dilution prepared in step a was taken, and 18 μL of ultrapure distilled water was added to obtain an siRNA dilution with a final concentration of 1 μM;
c. 2 μL of the 1 μM siRNA dilution prepared in step b was taken, and 18 μL of ultrapure distilled water was added to obtain an siRNA reserve solution with a final concentration of 0.1 μM;
d. 2 μL of the 0.1 μM siRNA dilution prepared in step c was taken, and 18 μL of ultrapure distilled water was added to obtain an siRNA reserve solution with a final concentration of 10 nM;

(2) 2 μL of the 10 nM siRNA reserve solution prepared in step (1) was taken, and 98 μL of Opti-meditun was added to obtain a 0.2 nM siRNA dilution;
(3) 3 μL of Lipo®RNAiMAX transfection reagent was taken, and 97 μL of Opti-medium was added to obtain a Lipo®RNAiMAX transfection reagent dilution; the Lipo®RNAiMAX transfection reagent dilution and the siRNA dilution were mixed at a volume ratio of 1:1, and the mixture was left to stand for 5 min. 10 μL of the transfection mixture was added to the 96-well plate to transfect the cultured HepG2 cells (the final volume was 100 μL, and the siRNA concentration in this system was 0.01 nM).

A 1 nM siRNA transfection dilution could be prepared following procedures similar to the above.
SiRNA transfection dilutions of 2.5 nM, 0.04 nM, and 0.0025 nM could be prepared following procedures similar to the above.
2.2.4. After transfection, the cells were cultured for 24 h, with 2 replicates set for each concentration.
2.2.5. Total RNA was extracted according to the instructions of the RNA extraction kit.
2.2.6. Reverse transcription of the extracted total RNA to cDNA was performed using a reverse transcription kit according to the following steps:
a) gDNA was removed with gDNA enzyme according to the table below:

| | Volume/μL |
|---|---|
| 5×gDNA Buffer | 2 |
| Sample (RNA) | 8 |

The above reagents were reacted at 42°C for 2 min to obtain a mixed solution;
b) The reverse transcription program was performed according to the table below:

| | Volume/μL |
|---|---|
| Mixed solution obtained in step a) | 10 |
| 10×RT Mix | 2 |
| HiScript III Enzyme Mix | 2 |
| Oligo $(dT)_{20}VN$ | 1 |
| Random hexamers | 1 |
| RNase-free $ddH_2O$ | 4 |

50°C, 15 min, extension; 85°C, 5 s, inactivation.
c) The reverse transcription product was stored at 4°C for real-time PCR analysis.

2.2.7. Performing real-time PCR analysis

a) The qPCR reaction mixture was prepared as shown in the table below, all reagents were placed on ice throughout the operation;

| Reagent | Volume (μL) |
|---|---|
| GoTaq® qPCR Master Mix | 10 |
| DGAT2/GAPDH PF/PR(2 μM) | 2 |
| cDNA template | 8 |

b) The qPCR program was performed as shown in the table below:

| 50°C | 95°C | 95°C | 60°C | 95°C | 55°C | 95°C |
|---|---|---|---|---|---|---|
| 2 min | 10 min | 3 s | 30 s | 1 min | 30 s | 30 s |
| 1 cycle | | 40 cycles | | 1 cycle | | |

2.2.8. Results analysis

a) The Ct value was automatically calculated using the Quant Studio 6 Flex software with default settings;
b) The relative expression level of the gene was calculated using the following formula:

$$\Delta Ct = Ct\ (DGAT2\ gene) - Ct\ (GAPDH)$$

$$\Delta\Delta Ct = \Delta Ct\ (test\ sample\ group) - \Delta Ct\ (Mock\ group)$$

mRNA expression relative to Mock group = $2^{-\Delta\Delta Ct}$.
Mock group: a group to which no siRNA was added as compared with the test sample group.

Inhibition rate (%) = (relative expression level of mRNA in Mock group - relative expression level of mRNA in test sample group)/relative expression level of mRNA in Mock group × 100%

2.2.9. Experimental results

siRNA concentrations of 1 nM, 0.01 nM, 2.5 nM, 0.04 nM, and 0.0025 nM were selected for testing.

Table 3. Inhibition rate of siRNAs of the present disclosure

| siRNA ID | 1 nM-24 h (%) | 0.01 nM-24 h (%) | siRNA ID | 1 nM-24 h (%) | 0.01 nM-24 h (%) |
|---|---|---|---|---|---|
| N-FY001001M2 | 79.5 | 47.1 | N-FY001006 | 59.83 | -- |
| N-FY001001M3 | 79.8 | 46.3 | N-FY001007 | 51.89 | -- |
| N-FY001001M4 | 74.5 | 36.8 | N-FY001008 | 59.84 | -- |
| N-FY001001M5 | 59.5 | - | N-FY001010 | 85.14 | -- |
| N-FY001002 | 88.5 | 57.1 | N-FY001011 | 63.88 | -- |
| N-FY001002M2 | 77.8 | 41.1 | N-FY001012 | 79.42 | -- |
| N-FY001002M3 | 75.8 | 39.3 | N-FY001013 | 79.81 | -- |
| N-FY001003 | 90.6 | 67.1 | N-FY001014 | 64.67 | -- |
| N-FY001003M2 | 84.8 | 51.1 | N-FY001015 | 81.64 | -- |
| N-FY001003M3 | 85.8 | 49.3 | N-FY001016 | 74.42 | -- |

(continued)

| siRNA ID | 1 nM-24 h (%) | 0.01 nM-24 h (%) | siRNA ID | 1 nM-24 h (%) | 0.01 nM-24 h (%) |
|---|---|---|---|---|---|
| N-FY001003M4 | 76.5 | 46.8 | N-FY001017 | 82.29 | 45.28 |
| N-FY001003M5 | 69.7 | 34.9 | N-FY001017M2 | 59.78 | -- |
| N-FY001004M2 | 74.8 | 41.0 | N-FY001018 | 77.95 | -- |
| N-FY001004M3 | 65.5 | 39.3 | N-FY001019 | 77.69 | 51.42 |
| N-FY001005 | 84.8 | 51.0 | N-FY001019M2 | 88.59 | -- |
| N-FY001005M2 | 78.8 | 42.0 | N-FY001020 | 82.21 | 36.76 |
| N-FY001005M3 | 63.9 | 39.3 | N-FY001021 | 90.53 | 72.91 |
| N-FY001003M6 | 83.7 | 50.1 | N-FY001021M2 | 89.56 | 59.06 |
| N-FY001003M7 | 84.6 | 49.9 | N-FY001022 | 79.30 | 47.64 |
| N-FY001003M8 | 86.1 | 51.8 | N-FY001022M2 | 89.56 | 59.06 |
| N-FY001003M9 | 87.5 | 52.1 | N-FY001024 | 84.86 | 52.90 |
| N-FY001026 | 85.97 | 54.04 | N-FY001024M2 | 91.58 | 38.45 |
| N-FY001026M2 | 93.54 | 53.88 | N-FY001025 | 84.07 | 48.03 |
| N-FY001027 | 87.45 | 46.35 | N-FY001025M2 | 94.61 | 72.90 |
| N-FY001027M2 | 93.88 | 62.03 | N-FY001028 | 83.06 | 49.01 |
| N-FY001030 | 85.26 | 71.90 | N-FY001028M2 | 89.64 | 43.84 |
| N-FY001030M2 | 91.84 | 36.00 | N-FY001029 | 92.46 | 83.65 |
| N-FY001032 | 83.03 | 44.08 | N-FY001029M2 | 91.59 | 35.79 |
| N-FY001032M2 | 87.71 | -- | N-FY001031 | 86.39 | 44.68 |
| N-FY001033 | 84.87 | 48.60 | N-FY001031M2 | 94.48 | 67.56 |
| N-FY001033M2 | 94.45 | 53.83 | N-FY001034 | 81.06 | -- |
| N-FY001035 | 82.00 | -- | N-FY001036 | 81.02 | 32.78 |
| N-FY001037 | 71.24 | -- | N-FY001003M6D2 | 81.99 | 52.69 |
| N-FY001003D2 | 91.28 | 68.81 | N-FY001003M29D2 | 94.59 | 41.09 |
| N-FY001003M21D2 | 89.77 | 24.03 | N-FY001003M37 | 95.24 | 54.86 |
| N-FY001003M24D2 | 93.96 | 46.07 | N-FY001003M40 | 94.23 | 59.66 |
| N-FY001003M26D2 | 94.60 | 40.72 | N-FY001003M44 | 95.17 | 50.87 |
| N-FY001003M27D2 | 94.01 | 44.31 | N-FY001003M45 | 92.48 | 38.62 |
| Note: "--" results are not shown. | | | | | |

As can be seen from Table 3, the siRNA of the present disclosure can significantly inhibit DGAT2 gene expression at both 1 nM and 0.01 nM, with an inhibition rate of up to 95.24 % at 1 nM after 24 h and an inhibition rate of up to 83.65% at 0.01 nM after 24 h.

Table 4. Inhibition rates of siRNA conjugates of the present disclosure

| siRNA ID | 1 nM-24 h (%) | 0.01 nM-24 h (%) | siRNA ID | 1 nM-24 h (%) | 0.01 nM-24 h (%) |
|---|---|---|---|---|---|
| N-FY001003M6L96 | 90.48 | 66.33 | N-FY001003M11L96 | 87.05 | 33.83 |
| N-FY001003M12L96 | 92.19 | 36.73 | N-FY001003M13L96 | 90.84 | 59.91 |
| N-FY001003M14L96 | 87.92 | 55.91 | N-FY001003M15L96 | 91.25 | 64.52 |
| N-FY001003M19L96 | 80.59 | 75.34 | N-FY001003M20L96 | 90.08 | 58.52 |

(continued)

| siRNA ID | 1 nM-24 h (%) | 0.01 nM-24 h (%) | siRNA ID | 1 nM-24 h (%) | 0.01 nM-24 h (%) |
|---|---|---|---|---|---|
| N-FY001003M21L96 | 84.68 | 46.32 | N-FY001003M22L96 | 84.03 | 52.09 |
| N-FY001003M23L96 | 82.02 | 34.59 | N-FY001003M24L96 | 87.61 | 49.55 |
| N-FY001003M25L96 | 86.20 | 48.24 | N-FY001003M26L96 | 87.86 | 60.07 |
| N-FY001003M27L96 | 84.86 | -- | N-FY001003M28L96 | 85.25 | 38.17 |
| N-FY001003M29L96 | 92.05 | 44.77 | N-FY001003M30L96 | 81.02 | 35.64 |
| N-FY001003M31L96 | 80.54 | -- | N-FY001003M32L96 | 88.01 | -- |
| N-FY001003M33L96 | 89.01 | -- | N-FY001003M34L96 | 79.75 | -- |
| N-FY001021M6L96 | 86.35 | 53.66 | N-FY001021M10L96 | 87.08 | 45.52 |
| N-FY001021M11L96 | 85.68 | -- | N-FY001021M12L96 | 29.97 | -- |
| N-FY001021M13L96 | 87.52 | -- | N-FY001021M14L96 | 89.42 | 57.70 |
| N-FY001021M15L96 | 87.18 | 56.86 | N-FY001021M16L96 | 89.58 | 78.08 |
| N-FY001021M17L96 | 89.57 | 53.16 | N-FY001021M18L96 | 87.46 | 54.29 |
| N-FY001021M19L96 | 89.75 | 51.60 | N-FY001021M20L96 | 85.74 | 40.11 |
| N-FY001021M21L96 | 85.69 | 39.74 | N-FY001021M22L96 | 43.79 | -- |
| N-FY001021M23L96 | 87.84 | 41.23 | N-FY001021M24L96 | 85.34 | 38.85 |
| N-FY001021M25L96 | 33.95 | 32.77 | N-FY001021M26L96 | 81.58 | -- |
| N-FY001021M27L96 | 83.15 | 35.45 | N-FY001021M28L96 | 54.71 | -- |
| N-FY001021M29L96 | 82.83 | 38.70 | N-FY001021M30L96 | 78.46 | -- |
| N-FY001021M31L96 | 13.59 | -- | N-FY001021M32L96 | 46.83 | -- |
| N-FY001021M33L96 | 13.77 | -- | N-FY001021M34L96 | 65.83 | -- |
| N-FY001002M6L96 | 77.74 | -- | N-FY001002M10L96 | 59.15 | -- |
| N-FY001002M12L96 | 61.82 | -- | N-FY001002M14L96 | 71.62 | -- |
| N-FY001002M17L96 | 79.71 | 42.86 | N-FY001002M18L96 | 28.88 | -- |
| N-FY001002M20L96 | 83.20 | -- | N-FY001002M21L96 | 67.82 | -- |
| N-FY001002M22L96 | 60.44 | -- | N-FY001002M23L96 | 82.64 | -- |
| N-FY001002M24L96 | 63.20 | -- | N-FY001002M25L96 | 32.48 | -- |
| N-FY001002M26L96 | 53.05 | -- | N-FY001003M35L96 | 88.92 | 53.78 |
| N-FY001003M37L96 | 94.46 | 60.22 | N-FY001003M44L96 | 95.43 | 60.05 |
| N-FY001003M40L96 | 95.24 | 58.31 | N-FY001003M45L96 | 94.13 | 61.81 |
| Note: "--" results are not shown. | | | | | |

**Example 3. Determination of inhibition rates of siRNA conjugates against DGAT2 gene expression**

3.1. Test materials

Primary human hepatocyte (PHH) cells, provided by WuXi AppTec (Shanghai) Co., Ltd.
PHH culture medium: invitroGRO CP Meduim serum free BIOVIT, Cat. No.: S03316.
Lipo®RNAiMAX transfection reagent, purchased from Invitrogen, Cat. No.: 13778-150.
RNeasy® 96 Kit, purchased from QIAGEN, Cat. No.: QIAGEN-74182.
FastKing RT Kit (containing gDNase), purchased from TianGen, Cat. No.: KR116-02.

FastStart Universal Probe master, purchased from Roche, Cat. No.: 4913918001.
Primers for DGAT2 and GAPDH were provided by WuXi AppTec (Shanghai) Co., Ltd.

3.2. Test method

siRNA conjugates (final concentrations of siRNA conjugates were 5 nM and 0.2 nM, with replicates) were introduced into PHH cells via transfection, and the procedures were as described below: frozen PHH cells were taken, thawed, counted, and adjusted to $6 \times 10^5$ cells/mL. Simultaneously, the siRNA conjugates were introduced into the cells using the Lipo®RNAiMax transfection reagent, and the cells were seeded into a 96-well plate at a density of 54,000 cells per well, with 100 μL of PHH culture medium added to each well. The cells were cultured in an incubator at 37°C with 5% $CO_2$. After 48 h, the culture medium was removed, and the cells were collected for total RNA extraction. The total RNA was extracted using the RNeasy® 96 Kit according to the kit instructions.

SiRNA conjugates (final concentrations of siRNA conjugates were 200 nM and 10 nM, with replicates) were introduced into PHH cells via free uptake, and the procedures were as described below: frozen PHH cells were taken, thawed, counted, and adjusted to $6 \times 10^5$ cells/mL. Simultaneously, the siRNA conjugates were added, and the cells were seeded into a 96-well plate at a density of 54,000 cells per well, with 100 μL of culture solution added to each well. The cells were cultured in an incubator at 37°C with 5% $CO_2$. After 48 h, the culture medium was removed, and the cells were collected for total RNA extraction. The total RNA was extracted using the RNeasy® 96 Kit according to the kit instructions.

Reverse transcription of the extracted total RNA to cDNA was performed using a reverse transcription kit according to the following steps:

a) gDNA was removed with gDNA enzyme according to the table below:

| | Volume/μL |
|---|---|
| 5×gDNA Buffer | 2 |
| Sample (RNA) | 8 |

The above reagents were reacted at 42°C for 2 min to obtain a mixed solution.

b) The reverse transcription program was performed according to the table below:

| | Volume/μL |
|---|---|
| Mixed solution obtained in step a) | 10 |
| FQ-RT Primer Mix | 2 |
| FastKing RT Enzyme Mix | 1 |
| 10×King RT Buffer | 1 |
| RNase-Free Water | 5 |

42°C, 15 min, extension; 95°C, 3 min, inactivation.

c) The reverse transcription product was stored at 4°C for real-time PCR analysis.

3.2.7. Performing real-time PCR analysis

a) The qPCR reaction mixture was prepared as shown in the table below, all reagents were placed on ice hroughout the operation;

| Reagent | Volume (μL) |
|---|---|
| FastStart Universal Probe Master | 5 |
| target gene TaqMan® Gene Expression Assays(60×) | 1/6 |
| cDNA | 2 |
| RNase-Free Water | Top up to 10 μL |

(continued)

| Reagent | Volume (μL) |
|---|---|
| FastStart Universal Probe Master | 5 |
| GAPDH gene TaqMan® Gene Expression Assays(60×) | 1/6 |
| cDNA | 2 |
| RNase-Free Water | Top up to 10 μL |

b) The qPCR program was performed as shown in the table below:

| | | |
|---|---|---|
| 95°C | 95°C | 60°C |
| 10 min | 15 s | 1 min |
| 1 cycle | 40 cycles | |

Results analysis

a) The Ct value was automatically calculated using the Quant Studio 7 software with default settings.
b) The relative expression level of the gene was calculated using the following formulas:

$$\Delta Ct = Ct\ (DGAT2\ gene) - Ct\ (GAPDH)$$

$\Delta\Delta Ct = \Delta Ct$ (test sample group) - $\Delta Ct$ (Mock group), wherein the Mock group represents a group to which no siRNA conjugate was added as compared with the test sample group;
mRNA expression relative to Mock group = $2^{-\Delta\Delta Ct}$.

Inhibition rate (%) = (relative expression level of mRNA in Mock group - relative expression level of mRNA in test sample group)/relative expression level of mRNA in Mock group × 100%.

The experimental results are shown in Table 5.

Table 5. Inhibition rates of siRNA conjugates against DGAT2 gene expression

| siRNA conjugate ID | Inhibition rate (%) (siRNA conjugate introduced into PHH via transfection) | | Inhibition rate (%) (siRNA conjugate introduced into PHH via free uptake) | |
|---|---|---|---|---|
| | 5 nM | 0.2 nM | 200 nM | 10 nM |
| N-FY001001M2L96 | 84.95 | 68.79 | 77.32 | 67.63 |
| N-FY001001M3L96 | 86.15 | 73.10 | 82.71 | 77.66 |
| N-FY001002M2L96 | 76.87 | 70.18 | 73.07 | 61.80 |
| N-FY001002M3L96 | 82.25 | 71.88 | 75.39 | 63.81 |
| N-FY001003M2L96 | 93.72 | 81.22 | 92.75 | 90.55 |
| N-FY001003M3L96 | 95.30 | 84.25 | 93.87 | 90.91 |
| N-FY001004M2L96 | 79.32 | 54.41 | 74.08 | 55.50 |
| N-FY001004M3L96 | 78.48 | 57.83 | 75.88 | 66.11 |
| N-FY001005M2L96 | 80.05 | 64.32 | 78.15 | 61.54 |
| N-FY001005M3L96 | 80.68 | 58.21 | 79.31 | 67.51 |
| N-FY001003M6L96 | 95.97 | 89.22 | 94.36 | 93.73 |

(continued)

| siRNA conjugate ID | Inhibition rate (%) (siRNA conjugate introduced into PHH via transfection) | | Inhibition rate (%) (siRNA conjugate introduced into PHH via free uptake) | |
|---|---|---|---|---|
| | 5 nM | 0.2 nM | 200 nM | 10 nM |
| N-FY001003M7L96 | 96.18 | 89.87 | 93.84 | 93.15 |
| N-FY001003M8L96 | 96.82 | 90.05 | 94.31 | 93.89 |
| N-FY001003M9L96 | 97.01 | 90.56 | 95.32 | 94.13 |
| N-FY001021M2L96 | 94.87 | 88.85 | 87.54 | 86.68 |
| N-FY001021M4L96 | 94.02 | 88.07 | 86.61 | 85.36 |
| N-FY001021M5L96 | 91.79 | 83.28 | 72.59 | 70.57 |
| N-FY001022M2L96 | 90.08 | 82.41 | 79.33 | 74.42 |
| N-FY001022M4L96 | 73.36 | 59.47 | -- | -- |
| N-FY001022M5L96 | 66.02 | 52.42 | -- | -- |
| N-FY001025M2L96 | 94.35 | 86.52 | 92.50 | 91.56 |
| N-FY001025M3L96 | -- | -- | 90.52 | 90.04 |
| N-FY001025M4L96 | 94.13 | 87.76 | 85.54 | 84.26 |
| N-FY001025M5L96 | 86.57 | 79.54 | 66.59 | 65.72 |
| N-FY001025M6L96 | -- | -- | 87.15 | 86.44 |
| N-FY001025M7L96 | -- | -- | 87.97 | 86.57 |
| N-FY001025M8L96 | -- | -- | 91.09 | 90.12 |
| N-FY001025M9L96 | -- | -- | 92.51 | 90.43 |
| N-FY001026M2L96 | 96.16 | 90.55 | 92.40 | 92.19 |
| N-FY001026M3L96 | -- | -- | 92.87 | 92.06 |
| N-FY001026M4L96 | 87.03 | 78.21 | 67.03 | 66.93 |
| N-FY001026M5L96 | 74.78 | 44.03 | -- | -- |
| N-FY001026M6L96 | -- | -- | 91.03 | 89.57 |
| N-FY001026M7L96 | -- | -- | 91.75 | 89.64 |
| N-FY001026M8L96 | -- | -- | 93.21 | 92.18 |
| N-FY001026M9L96 | -- | -- | 93.73 | 92.65 |
| N-FY001027M2L96 | 95.38 | 86.89 | 91.14 | 91.07 |
| N-FY001027M3L96 | -- | -- | 92.15 | 91.73 |
| N-FY001027M4L96 | 93.67 | 85.35 | 88.09 | 88.03 |
| N-FY001027M5L96 | 87.74 | 75.27 | 67.95 | 66.32 |
| N-FY001027M6L96 | -- | -- | 89.72 | 89.05 |
| N-FY001027M7L96 | -- | -- | 90.10 | 89.52 |
| N-FY001027M8L96 | -- | -- | 92.53 | 91.74 |
| N-FY001027M9L96 | -- | -- | 93.07 | 91.55 |
| N-FY001031M2L96 | 94.36 | 87.39 | 93.67 | 92.10 |
| N-FY001031M3L96 | -- | -- | 94.63 | 92.15 |
| N-FY001031M4L96 | 84.76 | 75.44 | 66.26 | 65.18 |
| N-FY001031M5L96 | 65.05 | 53.74 | -- | -- |
| N-FY001031M6L96 | -- | -- | 91.05 | 90.87 |

(continued)

| siRNA conjugate ID | Inhibition rate (%) (siRNA conjugate introduced into PHH via transfection) | | Inhibition rate (%) (siRNA conjugate introduced into PHH via free uptake) | |
|---|---|---|---|---|
| | 5 nM | 0.2 nM | 200 nM | 10 nM |
| N-FY001031M7L96 | -- | -- | 92.13 | 91.53 |
| N-FY001031M8L96 | -- | -- | 93.88 | 92.69 |
| N-FY001031M9L96 | -- | -- | 94.05 | 92.76 |
| N-FY001033M2L96 | 95.09 | 87.97 | 89.07 | 88.42 |
| N-FY001033M3L96 | -- | -- | 90.12 | 90.07 |
| N-FY001033M4L96 | 92.98 | 82.82 | 83.46 | 82.96 |
| N-FY001033M5L96 | 90.64 | 79.93 | 77.41 | 76.91 |
| N-FY001033M6L96 | -- | -- | 88.71 | 87.94 |
| N-FY001033M7L96 | -- | -- | 89.05 | 88.12 |
| N-FY001033M8L96 | -- | -- | 91.05 | 89.34 |
| N-FY001033M9L96 | -- | -- | 92.63 | 91.06 |
| Note: "--" results are not shown. | | | | |

As can be seen from Table 5, the siRNA conjugates of the present disclosure exhibited relatively high DGAT2 gene inhibition activity in PHH cells. When the siRNA conjugates were introduced into PHH via transfection, the inhibition rate at 5 nM could be up to 97.01%; when the siRNA conjugates were introduced into PHH via free uptake, the inhibition rate at 200 nM could be up to 95.32%.

**Example 4. Determination of inhibition rates of siRNAs against DGAT2 gene expression**

4.1. Test materials

Primary human hepatocyte (PHH) cells, provided by IPHASE Biosciences (Suzhou) Co., Ltd..
PHH culture medium: purchased from IPHASE Biosciences (Suzhou) Co., Ltd., Cat. No.: 0193301.32.
RNA extraction kit, Cat. No.: QIAGEN-74106.
Lipo®RNAiMAX transfection reagent, purchased from Invitrogen, Cat. No.: 13778-150.
DMEM culture medium, purchased from Gibco, Cat. No.: 11965118.
Reverse transcription kit (HiScript®III 1st Strand cDNA Synthesis Kit (+gDNA wiper)), purchased from Vazyme, Cat. No.: R312-02.
TaqMan™ gene expression premix, purchased from Applied Biosystems, Cat. No.: 4369016.
Opti-medium: purchased from Gibco, Cat. No.: 31985070.
FBS, purchased from Gibco, Cat. No.: 10099141.
PBS, purchased from MACGENE, Cat. No.: CC006.
Pancreatin, purchased from Gibco, Cat. No.: 15400054.
Target DGAT2 primer set, purchased from Shanghai Talen-bio Scientific Co.,Ltd forward primer: GGCCTCCCGG AGACTGA (SEQ ID NO: 341); reverse primer: AAGTGATTTGCAGCTGGTTCCT (SEQ ID NO: 342).
GAPDH primer set, purchased from Shanghai Talen-bio Scientific Co.,Ltd forward primer: GCACCGTCAAGGCT GAGAAC (SEQ ID NO: 343); reverse primer: TGGTGAAGACGCCAGTGGA (SEQ ID NO: 344).

4.2. Test method

siRNA conjugates (final concentrations of siRNA conjugates were 2.5 nM and 0.625 nM, with replicates) were introduced into PHH cells via transfection, and the procedures were as described below: frozen PHH cells were taken, thawed, counted, and adjusted to $6 \times 10^5$ cells/mL. Simultaneously, the siRNA conjugates were introduced into the cells using the Lipo®RNAiMax transfection reagent, and the cells were seeded into a 96-well plate at a density of 54,000 cells

per well, with 100 μL of PHH culture medium added to each well. The cells were cultured in an incubator at 37°C with 5% $CO_2$. After 48 h, the culture medium was removed, and the cells were collected for total RNA extraction. The total RNA was extracted using the RNeasy® 96 Kit according to the kit instructions.

Using a method similar to that in Example 2, the extracted total RNA was reverse transcribed to cDNA by a reverse transcription reaction, and the DGAT2 cDNA obtained by reverse transcription was quantitatively amplified by qPCR. GAPDH cDNA was amplified in parallel as an internal control. The PCR reaction program was: 95°C for 10 min, followed by a cycling mode of 95°C for 15 s and then 60°C for 60 s, for a total of 40 cycles.

Results analysis

a) The Ct value was automatically calculated using the Quant Studio 7 software with default settings.
b) The relative expression level of the gene was calculated using the following formulas:

$$\Delta Ct = Ct\ (DGAT2\ gene) - Ct\ (GAPDH)$$

$$\Delta\Delta Ct = \Delta Ct\ (test\ sample\ group) - \Delta Ct\ (Mock\ group),$$

wherein the Mock group represents a group to which no siRNA conjugate was added as compared with the test sample group;

mRNA expression relative to Mock group = $2^{-\Delta\Delta Ct}$.

Inhibition rate (%) = (relative expression level of mRNA in Mock group - relative expression level of mRNA in test sample group)/relative expression level of mRNA in Mock group × 100%.

As can be seen from the experimental results, the siRNA conjugates of the present disclosure all exhibited relatively high DGAT2 gene inhibition activity in PHH cells.

**Example 5. *In vivo* silencing effects of siRNA conjugates in mice expressing human DGAT2 (hDGAT2) gene**

(1) AAV construction of mouse model overexpressing hDGAT2 gene

C57BL/6 mice aged 6-8 weeks (provided by Jiangsu GemPharmatech Co., Ltd.) entered the facility. After 3-5 days of adaptive feeding, a single tail vein injection of the adeno-associated virus (AAV) carrying the hDGAT2 gene (pAAV [Exp]-CBh>{hDGAT2 CDS}:T2A:SEAP(ns):WPRE, the virus provided by VectorBuilder (Guangzhou) Co., Ltd.) was administered to establish the target gene overexpression model, with the administration volume being 100 μL ($3 \times 10^{11}$ vg)/mouse, followed by feeding with common feed.

(2) Investigation of *in vivo* silencing efficacy of siRNAs in hDGAT2 mouse model

14 days after AAV virus injection, the mice were grouped (6 mice per group) and subcutaneously administered a single 3 mg/kg dose of N-FY001001M2L96, N-FY001002M2L96, N-FY001002M3L96, N-FY001003M2L96, and N-FY001004M2L96. The expression level of the SEAP protein (i.e., the expression level of the hDGAT2 protein) was measured on day 7, day 14, day 21, day 28, day 35, day 42, day 49, and day 56 after the administration.

Table 6. Inhibition rates of siRNA conjugates against hDGAT2

| siRNA conjugate ID | Inhibition rate (%) against hDGAT2 | | | | | | | |
|---|---|---|---|---|---|---|---|---|
| | Day 7 | Day 14 | Day 21 | Day 28 | Day 35 | Day 42 | Day 49 | Day 56 |
| N-FY001001M2L96 | 40.03 | 47.33 | 57.72 | 59.00 | 58.13 | 57.80 | 41.27 | 31.25 |
| N-FY001002M2L96 | 46.53 | 66.68 | 66.21 | 58.48 | 48.60 | 44.05 | -- | -- |
| N-FY001002M3L96 | 40.32 | 57.13 | 53.70 | 50.15 | 45.84 | 43.84 | -- | -- |
| N-FY001003M2L96 | 71.62 | 75.16 | 77.16 | 74.73 | 70.40 | 65.90 | 53.12 | 50.46 |

(continued)

| siRNA conjugate ID | Inhibition rate (%) against hDGAT2 | | | | | | | |
|---|---|---|---|---|---|---|---|---|
| | Day 7 | Day 14 | Day 21 | Day 28 | Day 35 | Day 42 | Day 49 | Day 56 |
| N-FY001004M2L96 | 58.30 | 63.99 | 63.67 | 64.58 | 61.78 | 63.16 | 45.53 | 36.77 |
| Note: "--" results are not shown. | | | | | | | | |

As can be seen from Table 6, the siRNA conjugates of the present disclosure exhibited relatively high inhibition activity against the hDGAT2 gene *in vivo* and were able to reduce the hDGAT2 protein level for a long duration. 21 days after administration of a single 3 mg/kg dose, the inhibition rate of siRNA conjugate N-FY001003M2L96 against hDGAT2 could be up to 77.16%.

**Example 6. *In vivo* silencing effects of siRNA conjugates in mice expressing human DGAT2 (hDGAT2) gene**

(1) AAV construction of mouse model overexpressing hDGAT2 gene

C57BL/6 mice aged 6-8 weeks (provided by Jiangsu GemPharmatech Co., Ltd.) entered the facility. After 3-5 days of adaptive feeding, a single tail vein injection of the adeno-associated virus (AAV) carrying the hDGAT2 gene (pAAV [Exp]-CBh>{hDGAT2 CDS}:T2A:SEAP(ns):WPRE, the virus was provided by VectorBuilder (Guangzhou) Co., Ltd.) was administered to establish the target gene overexpression model, with the administration volume being 100 $\mu$L ($3 \times 10^{11}$ vg)/mouse, followed by feeding with common feed.

(2) Investigation of *in vivo* silencing efficacy of siRNAs in hDGAT2 mouse model

14 days after AAV virus injection, the mice were grouped (6 mice per group) and subcutaneously administered a single 3 mg/kg dose of N-FY001003M6L96 and N-FY001003M8L96. The expression level of the SEAP protein (i.e., the expression level of the hDGAT2 protein) was measured on day 7, day 14, and day 21 after the administration.

Table 7. Inhibition rates of siRNA conjugates against hDGAT2

| siRNA conjugate ID | Inhibition rate (%) against hDGAT2 | | |
|---|---|---|---|
| | Day 7 | Day 14 | Day 21 |
| N-FY001003M6L96(3 mpk) | 82.31 | 82.75 | 81.90 |
| N-FY001003M8L96(3 mpk) | 84.29 | 87.07 | 84.22 |

As can be seen from Table 7, the siRNA conjugates of the present disclosure exhibited relatively high inhibition activity against the hDGAT2 gene *in vivo* and were able to reduce the hDGAT2 protein level for a long duration. 21 days after administration of a single 3 mg/kg dose, the inhibition rates of the siRNA conjugates against hDGAT2 could still be up to 80% or higher.

**Example 7. *In vivo* silencing effects of siRNA conjugates in mice expressing human DGAT2 (hDGAT2) gene**

(1) AAV construction of mouse model overexpressing hDGAT2 gene

C57BL/6 mice aged 6-8 weeks (provided by Jiangsu GemPharmatech Co., Ltd.) entered the facility. After 3-5 days of adaptive feeding, a single tail vein injection of the adeno-associated virus (AAV) carrying the hDGAT2 gene (pAAV [Exp]-CBh>{hDGAT2 CDS}:T2A:SEAP(ns):WPRE, the virus provided by VectorBuilder (Guangzhou) Co., Ltd.) was administered to establish the target gene overexpression model, with the administration volume being 100 $\mu$L ($3 \times 10^{11}$ vg)/mouse, followed by feeding with common feed.

(2) Investigation of *in vivo* silencing efficacy of siRNAs in hDGAT2 mouse model

14 days after AAV virus injection, the mice were grouped (6 mice per group) and subcutaneously administered a single 3 mg/kg dose of N-FY001003M6L96, N-FY001003M11L96, N-FY001003M14L96, N-FY001003M15L96, GalXC-DGAT2-0898, N-FY001021M11L96, N-FY001021M13L96, N-FY001021M15L96, and N-FY001021M19L96. The expression level of the SEAP protein (i.e., the expression level of the hDGAT2 protein) was measured on day 7 and day 14 after the administration.

Table 8. Inhibition rates of siRNA conjugates against hDGAT2

| siRNA conjugate ID | Inhibition rate (%) against hDGAT2 | | | |
|---|---|---|---|---|
| | Day 7 | Day 14 | Day 21 | Day 28 |
| N-FY001003M6L96 (3 mpk) | 84.06 | 86.27 | 85.54 | 80.02 |
| N-FY001003M11L96 (3 mpk) | 76.07 | 85.14 | 80.74 | 75.10 |
| N-FY001003M14L96 (3 mpk) | 75.63 | 74.86 | 48.18 | 21.43 |
| N-FY001003M15L96 (3 mpk) | 83.81 | 84.42 | 74.72 | 60.28 |
| GalXC-DGAT2-0898 (3 mpk) | 76.47 | 77.89 | 72.28 | 67.14 |
| N-FY001021M11L96 (3 mpk) | 66.29 | 63.00 | 53.35 | 40.46 |
| N-FY001021M13L96 (3 mpk) | 50.18 | 62.59 | 49.27 | 33.53 |
| N-FY001021M15L96 (3 mpk) | 73.47 | 74.70 | 64.28 | 49.88 |
| N-FY001021M19L96 (3 mpk) | 71.58 | 71.43 | 62.46 | 51.58 |

As can be seen from Table 8, the siRNA conjugates of the present disclosure exhibited relatively high inhibition activity against the hDGAT2 gene *in vivo.* 28 days after administration of a single 3 mg/kg dose of the siRNA conjugates, N-FY001003M6L96 could still achieve an inhibition rate of up to 80.02% against hDGAT2, demonstrating superior efficacy compared with both N-FY001003M11L96 (ESC-modified) and GalXC-DGAT2-0898 (comparative conjugate).

**Example 8. *In vivo* silencing effects of siRNA conjugates in mice expressing human DGAT2 (hDGAT2) gene**

(1) AAV construction of mouse model overexpressing hDGAT2 gene

C57BL/6 mice aged 6-8 weeks (provided by Jiangsu GemPharmatech Co., Ltd.) entered the facility. After 3-5 days of adaptive feeding, a single tail vein injection of the adeno-associated virus (AAV) carrying the hDGAT2 gene (pAAV [Exp]-CBh>{hDGAT2 CDS}:T2A:SEAP(ns):WPRE, the virus provided by VectorBuilder (Guangzhou) Co., Ltd.) was administered to establish the target gene overexpression model, with the administration volume being 100 μL ($3 \times 10^{11}$ vg)/mouse, followed by feeding with common feed.

(2) Investigation of *in vivo* silencing efficacy of siRNAs in hDGAT2 mouse model

14 days after AAV virus injection, the mice were grouped (6 mice per group) and subcutaneously administered a single 3 mg/kg dose of N-FY001003M6L96, N-FY001003M21L96, N-FY001003M22L96, N-FY001003M24L96, N-FY001003M25L96, N-FY001003M26L96, N-FY001003M27L96, N-FY001003M28L96, N-FY001003M29L96, and N-FY001021M27L96. The expression level of the SEAP protein (i.e., the expression level of the hDGAT2 protein) was measured on day 7, day 14, and day 21 after the administration.

Table 9. Inhibition rates of siRNA conjugates against hDGAT2

| siRNA conjugate ID | Inhibition rate (%) against hDGAT2 | | |
|---|---|---|---|
| | Day 7 | Day 14 | Day 21 |
| N-FY001003M6L96 (3 mpk) | 87.87 | 91.54 | 86.96 |
| N-FY001003M21L96 (3 mpk) | 88.87 | 93.25 | 89.61 |
| N-FY001003M22L96 (3 mpk) | 72.93 | 80.32 | 72.57 |
| N-FY001003M24L96 (3 mpk) | 85.23 | 90.88 | 85.89 |
| N-FY001003M25L96 (3 mpk) | 68.85 | 78.24 | 67.78 |
| N-FY001003M26L96 (3 mpk) | 86.09 | 90.10 | 84.20 |
| N-FY001003M27L96 (3 mpk) | 85.35 | 89.70 | 86.10 |

(continued)

| siRNA conjugate ID | Inhibition rate (%) against hDGAT2 | | |
|---|---|---|---|
| | Day 7 | Day 14 | Day 21 |
| N-FY001003M28L96 (3 mpk) | 61.17 | 66.59 | 56.52 |
| N-FY001003M29L96 (3 mpk) | 84.25 | 90.30 | 87.22 |
| N-FY001021M27L96 (3 mpk) | 78.82 | 84.12 | 80.55 |

As can be seen from Table 9, the siRNA conjugates of the present disclosure exhibited relatively high inhibition activity against the hDGAT2 gene *in vivo.* 14 days after administration of a single 3 mg/kg dose of the siRNA conjugates, N-FY001003M22L96, N-FY001003M25L96, and N-FY001021M27L96 all could achieve an inhibition rate of around 80%; N-FY001003M6L96, N-FY001003M21L96, N-FY001003M24L96, N-FY001003M26L96, N-FY001003M27L96, and N-FY001003M29L96 all could achieve an inhibition rate of about 90% or higher.

**Example 9. Stability experiment**

Table 10. Antisense strands of parent drugs and antisense strands of major metabolites

| Parent drug ID | Code for antisense strand of parent drug and antisense strand of main metabolite | Sequence for antisense strand of parent drug and antisense strand of main metabolite |
|---|---|---|
| N-FY001003M6L96 | B (i.e., antisense strand of N-FY001003M6L96) | asAfsagcAfaauagucUfaUfggusgsu (SEQ ID NO: 38) |
| | B-1 | asAfsagcAfaauagucUfaUfggusg (SEQ ID NO: 345) |
| N-FY001003M11L96 | C (i.e., antisense strand of N-FY001003M11L96) | asAfsagcAfaauagucUfaUfggusgsu (SEQ ID NO: 38) |
| | C-1 | asAfsagcAfaauagucUfaUfggusg (SEQ ID NO: 345) |

9.1. *In vitro* liver homogenate stability testing

Tris-HCl, purchased from Beyotime, Cat. No.: ST790-500 mL.

MgCl2, purchased from Macklin, Cat. No.: C11362865.

Sterile PBS, purchased from Solarbio, model: 0.01 M, pH 7.2-7.4.

Ammonium acetate, purchased from Tianjin Fengchuan Chemical Reagent Co., Ltd., Cat. No.: 202101052.

Ethylenediaminetetraacetic acid (EDTA) diammonium salt, purchased from ALDRICH, Cat. No.: MKCK0920.

Internal standard, provided by Sangon Biotech.

(1) Preparation of liver homogenate

The homogenate (mouse liver homogenate, rat liver homogenate, or human liver homogenate) was prepared with 100 mM Tris and 1 mM MgCl2 (pH = 6) at a ratio of 1 g:10 mL.

(2) Sample incubation

Preparation of working solutions: N-FY001003M6L96 and N-FY001003M11L96 were separately formulated into

40 μM test substances using sterile PBS.

975 μL of liver homogenate (N = 2) was pre-incubated at 37°C for 5 min, and 25 μL of the 40 μM test substance was added. The mixture was well mixed by vortex and aliquoted by time point, with 100 μL per aliquot. The sample at 48 h was incubated at 37°C. Samples at the remaining time points were frozen in liquid nitrogen, stored in a freezer at -60°C to -90°C, and were taken out for incubation according to the times shown in Table 10.

Table 11

| Time (h) | Operation |
|---|---|
| 0 | Incubate sample for 48 h |
| 24 | Incubate sample for 24 h |
| 48 | Take out sample for 0 h, and uniformly treat samples for 0-48 h |

Sample treatment

200 μL of 100 mM ammonium acetate-EDTA solution (pH = 10) and 100 μL of internal standard working solution were added to 100 μL of the incubated sample, then subjected to vortexing for 3 min, followed by the addition of 200 μL of DNA extraction solution and 400 μL of dichloromethane. The mixture was vortexed for 3 min and centrifuged at 12000 rpm at room temperature for 10 min, and the supernatant was taken for injection analysis.

Sample detection and analysis

The proportions of the antisense strands of the parent drugs and the antisense strands of major metabolites were semi-quantitatively detected using the LC-MS/MS method. The results are shown in Table 12.

Table 12. Percentage (%) of antisense strands of parent drugs N-FY001003M6L96 and N-FY001003M11L96 and major metabolites thereof in rat liver homogenate

| Parent drug ID | Antisense strand code | Antisense strand percentage (%) | | Sum of antisense strand percentage (%) | |
|---|---|---|---|---|---|
| | | 24 h | 48 h | 24 h | 48 h |
| N-FY001003M6L96 | B | 75.28 | 71.44 | 97.74 | 95.09 |
| | B-1 | 22.46 | 23.65 | | |
| N-FY001003M11L96 | C | 72.80 | 36.16 | 90.43 | 61.40 |
| | C-1 | 17.63 | 25.24 | | |

As can be seen from the experimental data in Table 12, the stability of parent drug N-FY001003M6L96 was better than that of N-FY001003M11L96 after 48 h in rat liver homogenate. In addition, although N-FY001003M6L96 and N-FY001003M11L96 only differed in their sense strands, there was a significant difference in the remaining amounts of their metabolites, indicating that the metabolic outcome is a result of the synergistic effect of the siRNA double strands.

9.2. *In vivo* stability testing in cynomolgus monkeys

1. Via biochemical testing and liver biopsy pathological analysis for screening, 6 animals meeting the requirements were selected from a group of 8- to 9-year-old cynomolgus monkeys with high-fat and high-fructose induction (cynomolgus monkeys were sourced from WuXi AppTec (Chengdu) Co., Ltd.). 2 animals meeting the experimental requirements for NASH (defined as hepatic steatosis histopathology score ≥ 2, inflammatory response ≥ 2, ballooning ≥ 1 (if present), and hepatic fibrosis histopathology score ≥ 2 simultaneously) were enrolled in the experiment; additionally, 2 healthy animals were selected as healthy control.
2. Administration observation period

The animals underwent a 12-week administration observation period. The day of administration was defined as Day 0 (D0). The specific administration design is summarized in the table below:

Table 13

| Test substance | Administration dose | Administration concentration | Administration volume (mL/kg) | Administration mode | Administration frequency |
|---|---|---|---|---|---|
| Normal saline | ~ | NA | 0.5 | Subcutaneous injection | Once on Day0 |
| N-FY001003M2L96 | 9 mg/kg | 18 mg/mL | 0.5 | | |

The injection site was the lateral scapular/neck area, where the skin was loose. The hair around the injection site was shaved clean, the skin was disinfected with iodine and alcohol, and then subcutaneous administration was performed. On D21 after the administration, liver puncture biopsy was performed to detect the drug metabolism within the liver.

Table 14

| Name | Antisense strand A | A-1 | A-3 |
|---|---|---|---|
| Content (μg/g) | < LLOQ | 7.64 | 18.55 |

As can be seen from the experimental data in Table 14, antisense strand A mainly existed in two metabolite forms in cynomolgus monkey liver, among which metabolite antisense strand A-3 (i.e., the form with the phosphorylation modification removed from the 5' end and one nucleotide removed from the 3' end of the AS strand) had the highest content, followed by metabolite antisense strand A-1 (i.e., the form with the phosphorylation modification removed from the 5' end of the AS strand).

9.3. *In vitro* PHH experiment with conjugates composed of antisense strand of major metabolite of parent drug N-FY001003M6L96 and sense strand of N-FY001003M6L96

Table 15

| siRNA conjugate ID | SS (5'→3') | AS (5'→3') |
|---|---|---|
| N-FY001003M6L96 | ascscauaGfaCfUfAfuuugcuuuL96 (SEQ ID NO: 282) | asAfsagcAfaauagucUfaUfggusgsu (SEQ ID NO: 38) |
| N-FY001003M6L96B-1 | ascscauaGfaCfUfAfuuugcuuuL96 (SEQ ID NO: 282) | asAfsagcAfaauagucUfaUfggusg (SEQ ID NO: 345) |

The exact same experimental method as in Example 3 was employed.

Table 16. Inhibition rates of siRNA conjugates against DGAT2 gene expression

| siRNA conjugate ID | Inhibition rate (%) (siRNA conjugate introduced into PHH via transfection) | | Inhibition rate (%) (siRNA conjugate introduced into PHH via free uptake) | |
|---|---|---|---|---|
| | 5 nM | 0.2 nM | 200 nM | 10 nM |
| N-FY001003M6L96 | 95.97 | 89.22 | 93.36 | 93.73 |
| N-FY001003M6L96B-1 | 96.26 | 89.57 | 93.41 | 93.38 |

As can be seen from Table 16, metabolite N-FY001003M6L96B-1 of N-FY001003M6L96 exhibited *in vivo* activity comparable to that of its parent drug, showing an excellent inhibition effect on the DGAT2 gene.

**Example 10. Plasma kinetic study of siRNA conjugates in CD-1 mice**

Test animals: CD-1 mice, SPF grade, male, around 30 g, purchased from SPF (Beijing) Biotechnology Co., Ltd.

Administration dose and mode: siRNA conjugates were administered at a dose of 3 mg/kg (10 mL/kg). After random grouping, a single subcutaneous injection was administered, with 6 mice per group.

Sample collection: whole blood samples were collected at 0.0833 h, 0.25 h, 0.5 h, 1 h, 2 h, 4 h, 8 h, 24 h, 36 h, and

48 h after administration for a total of 10 time points. For each group, the first 3 mice were sampled at 0.0833 h, 0.5 h, 2 h, 8 h, and 36 h; the latter 3 mice were sampled at 0.25 h, 1 h, 4 h, 24 h, and 48 h. Whole blood samples were immediately placed on ice after collection. Within 0.5 h of collection, the samples were centrifuged at 4°C and 2000 g for 5 min to separate plasma. The upper plasma samples were collected into sample tubes. Plasma samples were frozen and stored in a freezer at -10°C to -30°C within 0.5 h and transferred to a freezer at -60°C to -90°C within 24 h.

Sample detection and analysis: the concentration of the parent drug in plasma samples at each time point was detected by the LC-MS/MS method. PK parameters ($C_{max}$, $T_{max}$, AUC, MRT, $t_{1/2}$) were calculated using WinNonlin software. The results are shown in Table 17.

Table 17. Plasma kinetic parameters of siRNA conjugates in mice

| Conjugate | Analysis object | $C_{max}$ (h) | $T_{max}$ (ng/mL) | $t_{1/2}$ (h) | $AUC_{0-t}$ (ng·h/mL) | $MRT_{0-t}$ (h) |
|---|---|---|---|---|---|---|
| N-FY001003M2L96 | Sense strand | 0.5 h | 409 | 0.593 | 605 | 1.11 |
| | Antisense strand | 0.5 h | 463 | 0.624 | 714 | 1.13 |
| N-FY001004M2L96 | Sense strand | 0.5 h | 483 | 0.767 | 759 | 1.18 |
| | Antisense strand | 0.5 h | 393 | 0.778 | 583 | 1.13 |

As can be seen from Table 17, the siRNA conjugates of the present disclosure had a short half-life and relatively fast clearance in plasma.

**Example 11. Tissue distribution test of siRNA conjugates in CD-1 mice**

Test animals: CD-1 mice, SPF grade, male, around 30 g, purchased from SPF (Beijing) Biotechnology Co., Ltd.

Administration dose and mode: siRNA conjugates were administered at a dose of 3 mg/kg (10 mL/kg). After random grouping, a single subcutaneous injection was administered, with 3 animals per time point, totaling 24 mice.

Sample collection:

24 h after administration: plasma, liver, kidney, and spleen were collected;
72 h after administration: plasma, liver, kidney, and spleen were collected;
168 h (1 week) after administration: plasma, liver, kidney, spleen, brain, heart, lung, stomach, small intestine, muscle, and testis were collected;
336 h (2 weeks) after administration: plasma, liver, kidney, and spleen were collected;
672 h (4 weeks) after administration: plasma, liver, kidney, spleen, brain, heart, lung, stomach, small intestine, muscle, and testis were collected;
1008 h (6 weeks) after administration: plasma, liver, kidney, and spleen were collected;
1344 h (8 weeks) after administration: plasma, liver, kidney, and spleen were collected;
1680 h (10 weeks) after administration: plasma, liver, kidney, spleen, brain, heart, lung, stomach, small intestine, muscle, and testis were collected.

Sample detection and analysis: the concentration of the parent drug in plasma and tissue samples at each time point was detected by the LC-MS/MS method. AUC in plasma and tissues was calculated using the trapezoidal area method.

It can be concluded from this experiment that the siRNA conjugates of the present disclosure primarily accumulate in the liver, have a long retention time in tissues, and exhibit very good stability.

**Example 12. MTD test of siRNA conjugates administered by single subcutaneous injection to C57 mice**

Test animals: C57 mice, SPF grade, male, around 25 g, purchased from SPF (Beijing) Biotechnology Co., Ltd. Based on the body weight on the last day of the acclimatization period, the animals were randomly grouped. The specific dose design and grouping are shown in the table below:

| Group code | Group name | Dose (mg/kg) | Number of animals (main test + satellite group) | |
|---|---|---|---|---|
| | | | Female | Male |
| G1 | Vehicle control group | 0 | 3+3 | 3+3 |

(continued)

| Group code | Group name | Dose (mg/kg) | Number of animals (main test + satellite group) | |
|---|---|---|---|---|
| | | | Female | Male |
| G2 | Low dose group | 50 | 3 | 3 |
| G3 | Medium dose group | 100 | 3 | 3 |
| G4 | High dose group | 200 | 3 | 3 |
| G5 | MTD dose group | MTD | 12 | 12 |

Detection indicators

Clinical observation: 4 h of continuous observation was performed on the day of administration, and at least one clinical observation was performed daily during the recovery period.

Body weight: all surviving animals were weighed twice weekly.

Immunotoxicity: for the MTD dose group, blood was collected alternately at 1 h ± 2 min, 4 h ± 5 min, 8 h ± 10 min, and 24 h ± 20 min after administration on D1, with 3 animals/gender/group per time point, to detect cytokines (IFN-y, TNF-$\alpha$, IL-2/6/8).

Toxicokinetics: for the MTD dose group, blood was collected alternately before administration and at 30 min ± 2 min, 1 h ± 2 min, 4 h ± 5 min, 8 h ± 10 min, and 24 h ± 20 min after administration on D1, with 3 animals/gender/group per time point, to detect plasma concentration.

Blood biochemistry: animals in the main test groups were necropsied on D28, and animals in the satellite group were necropsied in batches on D7, D14, D21, and D28. The blood biochemistry was examined.

Tissue distribution: animals in the main test groups were necropsied on D28, and animals in the satellite group were necropsied in batches on D7, D14, D21, and D28. Blood and liver were collected to detect tissue drug concentration.

Histopathological examination: animals in the main test groups were necropsied on D28. Major organs (heart, liver, spleen, lung, kidney, brain, adrenal gland, thymus, stomach, uterus/testis, ovary/epididymis) as well as abnormal tissues or organs were collected, fixed, and subjected to histopathological examination.

It can be concluded from this experiment that the siRNA conjugates of the present disclosure have low toxicity and an excellent medication safety window.

**Example 13. Efficacy experiment of siRNA conjugates in cynomolgus monkeys with NASH model**

1. 6 male cynomolgus monkeys (weight > 8 kg) aged 10 years or more with a NASH model (hepatic histopathology scores required to meet steatosis ≥ 2, inflammatory response ≥ 1, ballooning ≥ 1, 2 ≥ hepatic fibrosis score ≥ 1) (provided by Kunming Biomed International Co., Ltd.) were divided into two groups, entered the housing facility for feeding, and underwent MRI liver fat scanning 1 week before administration (D-7).
2. Administration observation period

The animals underwent a 12-week administration observation period. The day of administration was defined as Day 0 (D0). The specific administration design is summarized in the table below:

| Compound | Route of administration | Dose (mg/kg) | Concentratio n (mg/mL) | Administrati on volume (mL/kg) | Administration frequency |
|---|---|---|---|---|---|
| Normal saline | Subcutaneous administration | - | - | 0.5 | D0, D29, and D64 |
| N-FY001003M6L96 | Subcutaneous administration | 9 | 18 | 0.5 | Three administrations in total |

The injection site was the lateral scapular/neck area, where the skin was loose. The hair around the injection site was shaved clean, the skin was disinfected with iodine and alcohol, and then subcutaneous administration was performed. Liver fat scanning was performed through MRI on D28, D56, and D84 after administration; liver puncture biopsy was performed on D56 for mRNA expression analysis. The results are shown in Table 18 and Table 19.

Table 18. Percentage changes in liver fat content

| Compound | Percentage change in liver fat content | | | |
|---|---|---|---|---|
| | D-7 | D28 | D56 | D84 |
| Normal saline | 0.00 | -1.01 | 3.28 | 0.10 |
| N-FY001003M6L96 | 0.00 | -30.49 | -36.93 | -50.60 |

As can be seen from Table 18, the siRNA conjugates of the present disclosure had significant improvement effects on the change in liver fat content in cynomolgus monkeys with the NASH model. On D28, the conjugates reduced liver fat content by 30.49%; on D56, they reduced liver fat content by 36.93%; and on D84, they reduced liver fat content by 50.60%.

Table 19. Remaining percentage of liver mRNA

| Group | Remaining percentage of liver mRNA |
|---|---|
| | D84 |
| Normal saline | 96.43 |
| N-FY001003M6L96 | 2.33 |

As can be seen from Table 19, the siRNA conjugates of the present disclosure could significantly reduce the expression level of DGAT2 mRNA in the liver of cynomolgus monkeys with the NASH model, and on D84, the remaining liver mRNA was only 2.33%.

## Claims

**1.** An siRNA for inhibiting DGAT2 gene expression, comprising a sense strand and an antisense strand, wherein each nucleotide in the siRNA is independently a modified or unmodified nucleotide; the sense strand comprises a nucleotide sequence I, the antisense strand comprises a nucleotide sequence II, and the nucleotide sequence I is at least partially reversely complementary to the nucleotide sequence II to form a double-stranded region, wherein the nucleotide sequence I and the nucleotide sequence II are selected from the following sequences:

(1) the nucleotide sequence I comprising the nucleotide sequence shown in SEQ ID NO: 346, and the nucleotide sequence II comprising the nucleotide sequence shown in SEQ ID NO: 347:

5'-GACUAUUUGCUUU-3' (SEQ ID NO: 346)
5'-AAAGCAAAUAGUC-3' (SEQ ID NO: 347);

(2) the nucleotide sequence I comprising the nucleotide sequence shown in SEQ ID NO: 348, and the nucleotide sequence II comprising the nucleotide sequence shown in SEQ ID NO: 349:

5'-CCAUCCUCAUGUACAUAU-3' (SEQ ID NO: 348)
5'-AUAUGUACAUGAGGAUGG-3' (SEQ ID NO: 349);

(3) the nucleotide sequence I comprising the nucleotide sequence shown in SEQ ID NO: 350, and the nucleotide sequence II comprising the nucleotide sequence shown in SEQ ID NO: 351:

5'-CACCAUAGACUAUU-3' (SEQ ID NO: 350)
5'-AAUAGUCUAUGGUG-3' (SEQ ID NO: 351);

(4) the nucleotide sequence I comprising the nucleotide sequence shown in SEQ ID NO: 352, and the nucleotide sequence II comprising the nucleotide sequence shown in SEQ ID NO: 353:

5'-UUUGCUUUCAAAGAA-3' (SEQ ID NO: 352)
5'-UUCUUUGAAAGCAAAUA-3' (SEQ ID NO: 353);

(5) the nucleotide sequence I comprising the nucleotide sequence shown in SEQ ID NO: 354, and the nucleotide sequence II comprising the nucleotide sequence shown in SEQ ID NO: 355:

5'-CUUUCGAGACUA-3' (SEQ ID NO: 354)
5'-UAGUCUCGAAAGUA-3' (SEQ ID NO: 355);

(6) the nucleotide sequence I comprising the nucleotide sequence shown in SEQ ID NO: 356, and the nucleotide sequence II comprising the nucleotide sequence shown in SEQ ID NO: 357:

5'-CUCAUGUACAUA-3' (SEQ ID NO: 356)
5'-UAUGUACAUGAG-3' (SEQ ID NO: 357);

(7) the nucleotide sequence I comprising the nucleotide sequence shown in SEQ ID NO: 358, and the nucleotide sequence II comprising the nucleotide sequence shown in SEQ ID NO: 359:

5'-GUGGCGCUACUUUCGAGA-3' (SEQ ID NO: 358)
5'-UCUCGAAAGUAGCGCCAC-3' (SEQ ID NO: 359).

**2.** The siRNA according to claim 1, wherein the nucleotide sequence I is substantially reversely complementary, virtually reversely complementary, or completely reversely complementary to the nucleotide sequence II; the substantially reversely complementary means that there are no more than 3 base mismatches between the two nucleotide sequences; the virtually reversely complementary means that there is no more than 1 base mismatch between the two nucleotide sequences; the completely reversely complementary means that there is no mismatch between the two nucleotide sequences.

**3.** The siRNA according to claim 1 or 2, wherein the sense strand further comprises a nucleotide sequence III, and the antisense strand further comprises a nucleotide sequence IV, wherein the nucleotide sequence III and the nucleotide sequence IV each independently have a length of 0-11 nucleotides; the nucleotide sequence III is linked to the 5' end of the nucleotide sequence I, the nucleotide sequence IV is linked to the 3' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences; and/or
the nucleotide sequence III is linked to the 3' end of the nucleotide sequence I, the nucleotide sequence IV is linked to the 5' end of the nucleotide sequence II, and the nucleotide sequence III and the nucleotide sequence IV are equal in length and virtually reversely complementary or completely reversely complementary; the virtually reversely complementary means that there is no more than 1 base mismatch between the two nucleotide sequences; the completely reversely complementary means that there is no mismatch between the two nucleotide sequences.

**4.** The siRNA according to any one of claims 1-3, wherein the siRNA comprises a sense strand and an antisense strand, wherein each nucleotide in the siRNA is independently a modified or unmodified nucleotide; the sense strand comprises nucleotide sequences I and III, the antisense strand comprises nucleotide sequences II and IV, and the nucleotide sequences I and III are at least partially reversely complementary to the nucleotide sequences II and IV to form a double-stranded region, wherein the nucleotide sequences I and III and the nucleotide sequences II and IV are selected from the following sequences:

(1) the nucleotide sequences I and III comprising or consisting of the nucleotide sequence shown in SEQ ID NO: 251, and the nucleotide sequences II and IV comprising or consisting of the nucleotide sequence shown in SEQ ID NO: 252;
(2) the nucleotide sequences I and III comprising or consisting of the nucleotide sequence shown in SEQ ID NO: 253, and the nucleotide sequences II and IV comprising or consisting of the nucleotide sequence shown in SEQ ID NO: 254;
(3) the nucleotide sequences I and III comprising or consisting of the nucleotide sequence shown in SEQ ID NO: 42, and the nucleotide sequences II and IV comprising or consisting of the nucleotide sequence shown in SEQ ID NO: 128;
(4) the nucleotide sequences I and III comprising or consisting of the nucleotide sequence shown in SEQ ID NO: 23, and the nucleotide sequences II and IV comprising or consisting of the nucleotide sequence shown in SEQ ID NO: 24;

(5) the nucleotide sequences I and III comprising or consisting of the nucleotide sequence shown in SEQ ID NO: 31, and the nucleotide sequences II and IV comprising or consisting of the nucleotide sequence shown in SEQ ID NO: 32;
(6) the nucleotide sequences I and III comprising or consisting of the nucleotide sequence shown in SEQ ID NO: 47, and the nucleotide sequences II and IV comprising or consisting of the nucleotide sequence shown in SEQ ID NO: 48;
(7) the nucleotide sequences I and III comprising or consisting of the nucleotide sequence shown in SEQ ID NO: 255, and the nucleotide sequences II and IV comprising or consisting of the nucleotide sequence shown in SEQ ID NO: 256;
(8) the nucleotide sequences I and III comprising or consisting of the nucleotide sequence shown in SEQ ID NO: 49, and the nucleotide sequences II and IV comprising or consisting of the nucleotide sequence shown in SEQ ID NO: 50;
(9) the nucleotide sequences I and III comprising or consisting of the nucleotide sequence shown in SEQ ID NO: 55, and the nucleotide sequences II and IV comprising or consisting of the nucleotide sequence shown in SEQ ID NO: 56;
(10) the nucleotide sequences I and III comprising or consisting of the nucleotide sequence shown in SEQ ID NO: 257, and the nucleotide sequences II and IV comprising or consisting of the nucleotide sequence shown in SEQ ID NO: 258;
(11) the nucleotide sequences I and III comprising or consisting of the nucleotide sequence shown in SEQ ID NO: 57, and the nucleotide sequences II and IV comprising or consisting of the nucleotide sequence shown in SEQ ID NO: 58;
(12) the nucleotide sequences I and III comprising or consisting of the nucleotide sequence shown in SEQ ID NO: 59, and the nucleotide sequences II and IV comprising or consisting of the nucleotide sequence shown in SEQ ID NO: 60;
(13) the nucleotide sequences I and III comprising or consisting of the nucleotide sequence shown in SEQ ID NO: 61, and the nucleotide sequences II and IV comprising or consisting of the nucleotide sequence shown in SEQ ID NO: 62;
(14) the nucleotide sequences I and III comprising or consisting of the nucleotide sequence shown in SEQ ID NO: 259, and the nucleotide sequences II and IV comprising or consisting of the nucleotide sequence shown in SEQ ID NO: 260;
(15) the nucleotide sequences I and III comprising or consisting of the nucleotide sequence shown in SEQ ID NO: 261, and the nucleotide sequences II and IV comprising or consisting of the nucleotide sequence shown in SEQ ID NO: 262;
(16) the nucleotide sequences I and III comprising or consisting of the nucleotide sequence shown in SEQ ID NO: 75, and the nucleotide sequences II and IV comprising or consisting of the nucleotide sequence shown in SEQ ID NO: 76;
(17) the nucleotide sequences I and III comprising or consisting of the nucleotide sequence shown in SEQ ID NO: 263, and the nucleotide sequences II and IV comprising or consisting of the nucleotide sequence shown in SEQ ID NO: 264;
(18) the nucleotide sequences I and III comprising or consisting of the nucleotide sequence shown in SEQ ID NO: 265, and the nucleotide sequences II and IV comprising or consisting of the nucleotide sequence shown in SEQ ID NO: 266;
(19) the nucleotide sequences I and III comprising or consisting of the nucleotide sequence shown in SEQ ID NO: 267, and the nucleotide sequences II and IV comprising or consisting of the nucleotide sequence shown in SEQ ID NO: 268;
(20) the nucleotide sequences I and III comprising or consisting of the nucleotide sequence shown in SEQ ID NO: 269, and the nucleotide sequences II and IV comprising or consisting of the nucleotide sequence shown in SEQ ID NO: 270;
(21) the nucleotide sequences I and III comprising or consisting of the nucleotide sequence shown in SEQ ID NO: 15, and the nucleotide sequences II and IV comprising or consisting of the nucleotide sequence shown in SEQ ID NO: 271;
(22) the nucleotide sequences I and III comprising or consisting of the nucleotide sequence shown in SEQ ID NO: 272, and the nucleotide sequences II and IV comprising or consisting of the nucleotide sequence shown in SEQ ID NO: 273;
(23) the nucleotide sequences I and III comprising or consisting of the nucleotide sequence shown in SEQ ID NO: 131, and the nucleotide sequences II and IV comprising or consisting of the nucleotide sequence shown in SEQ ID NO: 132;
(24) the nucleotide sequences I and III comprising or consisting of the nucleotide sequence shown in SEQ ID NO:

274, and the nucleotide sequences II and IV comprising or consisting of the nucleotide sequence shown in SEQ ID NO: 275;
(25) the nucleotide sequences I and III comprising or consisting of the nucleotide sequence shown in SEQ ID NO: 276, and the nucleotide sequences II and IV comprising or consisting of the nucleotide sequence shown in SEQ ID NO: 277;
(26) the nucleotide sequences I and III comprising or consisting of the nucleotide sequence shown in SEQ ID NO: 163, and the nucleotide sequences II and IV comprising or consisting of the nucleotide sequence shown in SEQ ID NO: 164;
(27) the nucleotide sequences I and III comprising or consisting of the nucleotide sequence shown in SEQ ID NO: 165, and the nucleotide sequences II and IV comprising or consisting of the nucleotide sequence shown in SEQ ID NO: 166; or
(28) the nucleotide sequences I and III comprising or consisting of the nucleotide sequence shown in SEQ ID NO: 278, and the nucleotide sequences II and IV comprising or consisting of the nucleotide sequence shown in SEQ ID NO: 279.

**5.** The siRNA according to any one of claims 1-4, wherein the sense strand further comprises a nucleotide sequence V, and/or the antisense strand further comprises a nucleotide sequence VI, wherein the nucleotide sequence V and the nucleotide sequence VI have a length of 0-3 nucleotides; the nucleotide sequence V is linked to the 3' end of the sense strand to form a 3' overhang of the sense strand, and/or the nucleotide sequence VI is linked to the 3' end of the antisense strand to form a 3' overhang of the antisense strand; preferably, the nucleotide sequence V or the nucleotide sequence VI has a length of 2 nucleotides; more preferably, the nucleotide sequence V or the nucleotide sequence VI is two consecutive thymine deoxyribonucleotides, two consecutive uracil ribonucleotides, or one cytosine deoxyribonucleotide and one adenine deoxyribonucleotide in the 5' to 3' direction;
or, the nucleotide sequence V or the nucleotide sequence VI is mismatched or complementary to a nucleotide at a corresponding position of a target mRNA.

**6.** The siRNA according to any one of claims 1-5, wherein the double-stranded region has a length of 15-30 nucleotide pairs; preferably, the double-stranded region has a length of 17-23 nucleotide pairs; more preferably, the double-stranded region has a length of 19-21 nucleotide pairs.

**7.** The siRNA according to any one of claims 1-6, wherein the sense strand or the antisense strand has 15-30 nucleotides; preferably, the sense strand or the antisense strand has 19-25 nucleotides; more preferably, the sense strand or the antisense strand has 19-23 nucleotides.

**8.** The siRNA according to any one of claims 1-7, wherein at least one nucleotide in the sense strand or the antisense strand is a modified nucleotide, and/or at least one phosphoester group is a phosphoester group with a modification group; preferably, the phosphoester group with a modification group is a phosphorothioate group formed by substituting one oxygen atom in a phosphodiester bond of the phosphoester group with a sulfur atom; and/or, the siRNA comprises a sense strand without a 3' overhang nucleotide.

**9.** The siRNA according to any one of claims 1-8, wherein the 5' end nucleotide of the antisense strand is linked to a 5' phosphate group or a 5' phosphate-derived group, or the 5' end nucleotide of the antisense strand is not linked to a 5' phosphate group or a 5' phosphate-derived group.

**10.** The siRNA according to any one of claims 1-9, wherein the modified nucleotide is selected from a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, a 2'-substituted amino-modified nucleotide, a nucleotide analog, and a combination of any two or more thereof.

**11.** The siRNA according to any one of claims 1-10, wherein the modified nucleotide is selected from a 2'-fluoro-modified nucleotide, a 2'-methoxy-modified nucleotide, a 2'-O-$CH_2$-$CH_2$-O-$CH_3$ modified nucleotide, a 2'-O-$CH_2$-CH=$CH_2$ modified nucleotide, a 2'-$CH_2$-$CH_2$-CH=$CH_2$ modified nucleotide, a 2'-deoxynucleotide, a nucleotide analog, and a combination of any two or more thereof.

**12.** The siRNA according to any one of claims 1-11, wherein each nucleotide in the sense strand and the antisense strand is independently a 2'-fluoro-modified nucleotide or a non-fluoro-modified nucleotide;

preferably, in the sense strand, in the 5' to 3' direction, the 2'-fluoro-modified nucleotides are located at positions 7,

9, 10, and 11, and the remaining positions are the non-fluoro-modified nucleotides; or, the 2'-fluoro-modified nucleotides are located at positions 5, 7, 8, and 9, and the remaining positions are the non-fluoro-modified nucleotides; or, the 2'-fluoro-modified nucleotides are located at positions 9, 10, and 11, and the remaining positions are the non-fluoro-modified nucleotides; or, the 2'-fluoro-modified nucleotides are located at positions 8, 9, and 10, and the remaining positions are the non-fluoro-modified nucleotides; or, the 2'-fluoro-modified nucleotides are located at positions 8, 9, 10, and 11, and the remaining positions are the non-fluoro-modified nucleotides; or, the 2'-fluoro-modified nucleotides are located at positions 7 and 9, and the remaining positions are the non-fluoro-modified nucleotides; or, the 2'-fluoro-modified nucleotides are located at positions 7, 9, and 11, and the remaining positions are the non-fluoro-modified nucleotides; or, the 2'-fluoro-modified nucleotides are located at positions 7, 11, 13, and 15, and the remaining positions are the non-fluoro-modified nucleotides; or, the 2'-fluoro-modified nucleotides are located at positions 8, 9, 10, and 12, and the remaining positions are the non-fluoro-modified nucleotides; or, the 2'-fluoro-modified nucleotides are located at positions 7, 9, 11, 13, and 15, and the remaining positions are the non-fluoro-modified nucleotides; or, the 2'-fluoro-modified nucleotides are located at positions 9, 11, and 13, and the remaining positions are the non-fluoro-modified nucleotides; or, the 2'-fluoro-modified nucleotides are located at positions 7, 9, 11, and 13, and the remaining positions are the non-fluoro-modified nucleotides; or, the 2'-fluoro-modified nucleotides are located at positions 3, 7, 8, and 9, and the remaining positions are the non-fluoro-modified nucleotides; or, the 2'-fluoro-modified nucleotides are located at positions 3, 9, 11, and 13, and the remaining positions are the non-fluoro-modified nucleotides; or, the 2'-fluoro-modified nucleotides are located at positions 3, 7, 9, and 11, and the remaining positions are the non-fluoro-modified nucleotides; or, the 2'-fluoro-modified nucleotides are located at positions 7, 11, and 13, and the remaining positions are the non-fluoro-modified nucleotides; or, the 2'-fluoro-modified nucleotides are located at positions 7, 11, 12, and 13, and the remaining positions are the non-fluoro-modified nucleotides; or, the 2'-fluoro-modified nucleotides are located at positions 3, 7, 11, and 16, and the remaining positions are the non-fluoro-modified nucleotides; or, the 2'-fluoro-modified nucleotides are located at positions 3, 7, 11, and 17, and the remaining positions are the non-fluoro-modified nucleotides; or, the 2'-fluoro-modified nucleotides are located at positions 3, 7, 11, 16, and 17, and the remaining positions are the non-fluoro-modified nucleotides; and/or

in the antisense strand, in the 5' to 3' direction, the 2'-fluoro-modified nucleotides are located at positions 2, 6, 14, and 16, and the remaining positions are the non-fluoro-modified nucleotides; or, the 2'-fluoro-modified nucleotides are located at positions 2, 6, 8, 9, 14, and 16, and the remaining positions are the non-fluoro-modified nucleotides; or, the 2'-fluoro-modified nucleotides are located at positions 2, 14, and 16, and the remaining positions are the non-fluoro-modified nucleotides; or, the 2'-fluoro-modified nucleotides are located at positions 2, 6, and 14, and the remaining positions are the non-fluoro-modified nucleotides; or, the 2'-fluoro-modified nucleotides are located at positions 2, 3, 5, 8, 10, 14, 16, and 18, and the remaining positions are the non-fluoro-modified nucleotides; or, the 2'-fluoro-modified nucleotides are located at positions 2, 3, 4, 5, 7, 10, and 14, and the remaining positions are the non-fluoro-modified nucleotides; or, the 2'-fluoro-modified nucleotides are located at positions 2, 4, 6, 12, 14, 16, 18, and 20, and the remaining positions are the non-fluoro-modified nucleotides; or, the 2'-fluoro-modified nucleotides are located at positions 2 and 14, and the remaining positions are the non-fluoro-modified nucleotides; or, the 2'-fluoro-modified nucleotides are located at positions 2, 4, 5, 6, 8, 10, 12, 14, 16, 18, and 20, and the remaining positions are the non-fluoro-modified nucleotides; or, the 2'-fluoro-modified nucleotides are located at positions 2, 7, 10, and 14, and the remaining positions are the non-fluoro-modified nucleotides; or, the 2'-fluoro-modified nucleotides are located at positions 2, 5, 7, and 14, and the remaining positions are the non-fluoro-modified nucleotides.

**13.** The siRNA according to any one of claims 1-12, wherein each nucleotide in the sense strand and the antisense strand is independently a 2'-fluoro-modified nucleotide or a non-fluoro-modified nucleotide;
each of the non-fluoro-modified nucleotides is a 2'-methoxy-modified nucleotide, and the 2'-methoxy-modified nucleotide refers to a nucleotide formed by substituting 2'-hydroxyl of a ribosyl group with methoxy.

**14.** The siRNA according to claim 12 or 13, wherein each of the non-fluoro-modified nucleotides is independently selected from one of a nucleotide formed by substituting hydroxyl at the 2' position of a ribosyl group of the nucleotide with a non-fluorine group and a nucleotide analog, and the nucleotide analog is selected from one of isonucleotide, LNA, ENA, cET-BNA, UNA, and GNA.

**15.** The siRNA according to claim 14, wherein any one nucleotide in the sense strand and the antisense strand is independently a 2'-fluoro-modified nucleotide, a 2'-methoxy-modified nucleotide, a GNA-modified nucleotide, or a combination of any two or more thereof;

preferably, in the sense strand, in the 5' to 3' direction, the 2'-fluoro-modified nucleotides are located at positions 7,

9, 10, and 11, and the remaining positions are the 2'-methoxy-modified nucleotides; or, the 2'-fluoro-modified nucleotides are located at positions 5, 7, 8, and 9, and the remaining positions are the 2'-methoxy-modified nucleotides; or, the 2'-fluoro-modified nucleotides are located at positions 9, 10, and 11, and the remaining positions are the 2'-methoxy-modified nucleotides; or, the 2'-fluoro-modified nucleotides are located at positions 8, 9, and 10, and the remaining positions are the 2'-methoxy-modified nucleotides; or, the 2'-fluoro-modified nucleotides are located at positions 8, 9, 10, and 11, and the remaining positions are the 2'-methoxy-modified nucleotides; or, the 2'-fluoro-modified nucleotides are located at positions 7 and 9, position 11 is a 2'-deoxynucleotide, and the remaining positions are the 2'-methoxy-modified nucleotides; or, the 2'-fluoro-modified nucleotides are located at positions 7, 9, and 11, and the remaining positions are the 2'-methoxy-modified nucleotides; or, the 2'-fluoro-modified nucleotides are located at positions 7, 11, 13, and 15, and the remaining positions are the 2'-methoxy-modified nucleotides; or, the 2'-fluoro-modified nucleotides are located at positions 8, 9, 10, and 12, and the remaining positions are the 2'-methoxy-modified nucleotides; or, the 2'-fluoro-modified nucleotides are located at positions 7, 9, 11, 13, and 15, and the remaining positions are the 2'-methoxy-modified nucleotides; or, the 2'-fluoro-modified nucleotides are located at positions 9, 11, and 13, and the remaining positions are the 2'-methoxy-modified nucleotides; or, the 2'-fluoro-modified nucleotides are located at positions 7, 9, 11, and 13, and the remaining positions are the 2'-methoxy-modified nucleotides; or, the 2'-fluoro-modified nucleotides are located at positions 3, 7, 8, and 9, and the remaining positions are the 2'-methoxy-modified nucleotides; or, the 2'-fluoro-modified nucleotides are located at positions 3, 9, 11, and 13, and the remaining positions are the 2'-methoxy-modified nucleotides; or, the 2'-fluoro-modified nucleotides are located at positions 3, 7, 9, and 11, and the remaining positions are the 2'-methoxy-modified nucleotides; or, the 2'-fluoro-modified nucleotides are located at positions 7, 11, and 13, and the remaining positions are the 2'-methoxy-modified nucleotides; or, the 2'-fluoro-modified nucleotides are located at positions 7, 11, 12, and 13, and the remaining positions are the 2'-methoxy-modified nucleotides; or, the 2'-fluoro-modified nucleotides are located at positions 3, 7, 11, and 16, and the remaining positions are the 2'-methoxy-modified nucleotides; or, the 2'-fluoro-modified nucleotides are located at positions 3, 7, 11, and 17, and the remaining positions are the 2'-methoxy-modified nucleotides; or, the 2'-fluoro-modified nucleotides are located at positions 3, 7, 11, 16, and 17, and the remaining positions are the 2'-methoxy-modified nucleotides; and/or

in the antisense strand, in the 5' to 3' direction, the 2'-fluoro-modified nucleotides are located at positions 2, 6, 14, and 16, and the remaining positions are the 2'-methoxy-modified nucleotides; or, the 2'-fluoro-modified nucleotides are located at positions 2, 6, 14, and 16, position 7 is the GNA-modified nucleotide, and the remaining positions are the 2'-methoxy-modified nucleotides; or, the 2'-fluoro-modified nucleotides are located at positions 2, 6, 8, 9, 14, and 16, and the remaining positions are the 2'-methoxy-modified nucleotides; or, the 2'-fluoro-modified nucleotides are located at positions 2, 14, and 16, and the remaining positions are the 2'-methoxy-modified nucleotides; or, the 2'-fluoro-modified nucleotides are located at positions 2, 14, and 16, position 6 is the GNA-modified nucleotide, and the remaining positions are the 2'-methoxy-modified nucleotides; or, the 2'-fluoro-modified nucleotides are located at positions 2, 6, and 14, and the remaining positions are the 2'-methoxy-modified nucleotides; or, the 2'-fluoro-modified nucleotides are located at positions 2, 3, 5, 8, 10, 14, 16, and 18, and the remaining positions are the 2'-methoxy-modified nucleotides; or, the 2'-fluoro-modified nucleotides are located at positions 2, 3, 4, 5, 7, 10, and 14, and the remaining positions are the 2'-methoxy-modified nucleotides; or, the 2'-fluoro-modified nucleotides are located at positions 2, 3, 4, 5, 7, 10, and 14, position 6 is the GNA-modified nucleotide, and the remaining positions are the 2'-methoxy-modified nucleotides; or, the 2'-fluoro-modified nucleotides are located at positions 2, 4, 6, 12, 14, 16, 18, and 20, and the remaining positions are the 2'-methoxy-modified nucleotides; or, the 2'-fluoro-modified nucleotides are located at positions 2 and 14, and the remaining positions are the 2'-methoxy-modified nucleotides; or, the 2'-fluoro-modified nucleotides are located at positions 2, 4, 5, 6, 8, 10, 12, 14, 16, 18, and 20, and the remaining positions are the 2'-methoxy-modified nucleotides; or, the 2'-fluoro-modified nucleotides are located at positions 2, 7, 10, and 14, and the remaining positions are the 2'-methoxy-modified nucleotides; or, the 2'-fluoro-modified nucleotides are located at positions 2, 5, 7, and 14, and the remaining positions are the 2'-methoxy-modified nucleotides.

**16.** The siRNA according to any one of claims 1-15, wherein at least one of the following linkages between nucleotides in the siRNA is a phosphorothioate group linkage:

a linkage between the first nucleotide and the second nucleotide from the 5' end of the sense strand;
a linkage between the second nucleotide and the third nucleotide from the 5' end of the sense strand;
a linkage between the first nucleotide and the second nucleotide from the 3' end of the sense strand;
a linkage between the second nucleotide and the third nucleotide from the 3' end of the sense strand;
a linkage between the first nucleotide and the second nucleotide from the 5' end of the antisense strand;
a linkage between the second nucleotide and the third nucleotide from the 5' end of the antisense strand;

a linkage between the first nucleotide and the second nucleotide from the 3' end of the antisense strand;
a linkage between the second nucleotide and the third nucleotide from the 3' end of the antisense strand.

**17.** The siRNA according to any one of claims 1-16, wherein in the 5' to 3' direction, the sense strand comprises phosphorothioate groups located at the positions shown below:

a position between the first nucleotide and the second nucleotide from the 5' end of the sense strand;
a position between the second nucleotide and the third nucleotide from the 5' end of the sense strand;
a position between the first nucleotide and the second nucleotide from the 3' end of the sense strand;
a position between the second nucleotide and the third nucleotide from the 3' end of the sense strand; or,
the sense strand comprises phosphorothioate groups located at the positions shown below:

a position between the first nucleotide and the second nucleotide from the 5' end of the sense strand;
a position between the second nucleotide and the third nucleotide from the 5' end of the sense strand.

**18.** The siRNA according to any one of claims 1-17, wherein in the 5' to 3' direction, the antisense strand comprises phosphorothioate groups located at the positions shown below:

a position between the first nucleotide and the second nucleotide from the 5' end of the antisense strand;
a position between the second nucleotide and the third nucleotide from the 5' end of the antisense strand;
a position between the first nucleotide and the second nucleotide from the 3' end of the antisense strand;
a position between the second nucleotide and the third nucleotide from the 3' end of the antisense strand.

**19.** The siRNA according to any one of claims 1-18, wherein any one nucleotide in the sense strand and the antisense strand is independently a 2'-fluoro-modified nucleotide, a 2'-methoxy-modified nucleotide, a GNA-modified nucleotide, or a combination of any two or more thereof;

preferably, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, and the remaining positions are the 2'-methoxy-modified nucleotides, with the 5' end nucleotide of the antisense strand linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, and the remaining positions are the 2'-methoxy-modified nucleotides, with the 5' end nucleotide of the antisense strand linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 8, 9, 14, and 16 of the antisense strand, and the remaining positions are the 2'-methoxy-modified nucleotides, with the 5' end nucleotide of the antisense strand linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the GNA-modified nucleotide is located at position 7 of the antisense strand, and the remaining positions are the 2'-methoxy-modified nucleotides, with the 5' end nucleotide of the antisense strand linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 14, and 16 of the antisense strand, the GNA-modified nucleotide is located at position 6 of the antisense strand, and the remaining positions are the 2'-methoxy-modified nucleotides, with the 5' end nucleotide of the antisense strand linked to a 5' phosphate group;
or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;
or, in the 5' to 3' direction, the 2'-fluoro-modified nucleotides are located at positions 7, 9, 10, and 11 of the sense

strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 8, 9, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is linked to a 5'-(E)-vinylphosphonate group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, 8, 9, 14, and 16 of the antisense strand, the remaining positions are the 2'-methoxy-modified nucleotides, and the 5' end nucleotide of the antisense strand is linked to a 5'-(E)-vinylphosphonate group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 6, and 14 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 5, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 9, 10, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 8, 9, and 10 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 5, 8, 10, 14, 16, and 18 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 8, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, the remaining positions are the 2'-methoxy-modified nucleotides, and the 5' end nucleotide of the antisense strand is not linked

to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 4, 6, 12, 14, 16, 18, and 20 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2 and 14 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7 and 9 of the sense strand, position 11 is a 2'-deoxynucleotide, and the remaining positions are the 2'-methoxy-modified nucleotides; in the 5' to 3' direction, the 2'-fluoro-modified nucleotides are located at positions 2, 4, 5, 6, 8, 10, 12, 14, 16, 18, and 20 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7 and 9 of the sense strand, position 11 is a 2'-deoxynucleotide, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 11, 13, and 15 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 8, 9, 10, and 12 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 11, 13, and 15 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 11, 13, and 15 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 11, and 13 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 7, 8, and 9 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 7, 8, and 9 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 9, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 7, 9, and 11 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 7, 9, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 11, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 11, 12, and 13 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 7, 11, and 16 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 7, 11, and 17 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 3, 7, 11, 16, and 17 of the sense strand, and the remaining positions are the 2'-methoxy-modified nucleotides; in the 5' to 3' direction, the 2'-fluoro-modified nucleotides are located at positions 2, 6, 14, and 16 of the antisense strand, the remaining positions are the 2'-methoxy-modified nucleotides, and the 5' end nucleotide of the antisense strand is not linked to a 5' phosphate group or a 5' phosphate-derived group;

or, in the 5' to 3' direction, the 2'-fluoro-modified nucleotides are located at positions 7, 9, 10, and 11 of the sense strand, and the remaining positions are the 2'-methoxy-modified nucleotides; in the 5' to 3' direction, the 2'-fluoro-modified nucleotides are located at positions 2, 3, 4, 5, 7, 10, and 14 of the antisense strand, position 6 is the GNA-modified nucleotide, the remaining positions are the 2'-methoxy-modified nucleotides, and the 5' end nucleotide of the antisense strand is not linked to a 5' phosphate group or a 5' phosphate-derived group;
or, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, and the 5' end nucleotide of the antisense strand is not linked to a 5' phosphate group or a 5' phosphate-derived group;
or, in the 5' to 3' direction, the 2'-fluoro-modified nucleotides are located at positions 7, 9, 10, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 5, 7, and 14 of the antisense strand, the remaining positions are the 2'-methoxy-modified nucleotides, and the 5' end nucleotide of the antisense strand is not linked to a 5' phosphate group or a 5' phosphate-derived group;
or, in the 5' to 3' direction, the 2'-fluoro-modified nucleotides are located at positions 3, 7, 8, and 9 of the sense strand, and the remaining positions are the 2'-methoxy-modified nucleotides, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, and the 5' end nucleotide of the antisense strand is not linked to a 5' phosphate group or a 5' phosphate-derived group;
or, in the 5' to 3' direction, the 2'-fluoro-modified nucleotides are located at positions 3, 7, 9, and 11 of the sense strand, and the remaining positions are the 2'-methoxy-modified nucleotides, with the overhang removed from the 3' end; in the 5' to 3' direction, the 2'-fluoro-modified nucleotides are located at positions 2, 7, 10, and 14 of the antisense strand, the remaining positions are the 2'-methoxy-modified nucleotides, and the 5' end nucleotide of the antisense strand is not linked to a 5' phosphate group or a 5' phosphate-derived group.

**20.** The siRNA according to claim 1, wherein the siRNA comprises or is selected from the group consisting of at least one of the siRNAs in Table 1; preferably, the siRNA is selected from N-FY001003, N-FY001003D2, N-FY001003M2, N-FY001003M2D2, N-FY001003M3, N-FY001003M4, N-FY001003M5, N-FY001003M6, N-FY001003M6D2, N-FY001003M7, N-FY001003M7D2, N-FY001003M8, N-FY001003M9, N-FY001003M10, N-FY001003M11, N-FY001003M12, N-FY001003M13, N-FY001003M14, N-FY001003M15, N-FY001003M16, N-FY001003M17, N-FY001003M18, N-FY001003M19, N-FY001003M20, N-FY001003M21, N-FY001003M21D2, N-FY001003M22, N-FY001003M23, N-FY001003M24, N-FY001003M24D2, N-FY001003M25, N-FY001003M26, N-FY001003M26D2, N-FY001003M27, N-FY001003M27D2, N-FY001003M28, N-FY001003M29, N-FY001003M29D2, N-FY001003M30, N-FY001003M31, N-FY001003M32, N-FY001003M33, N-FY001003M34, N-FY001003M35, N-FY001003M37, N-FY001003M40, N-FY001003M44, and N-FY001003M45.

**21.** An siRNA conjugate or a prodrug thereof, wherein the siRNA conjugate comprises the siRNA according to any one of claims 1-20 and a conjugated group conjugated to the siRNA.

**22.** The siRNA conjugate or the prodrug thereof according to claim 21, wherein the conjugated group comprises a pharmaceutically acceptable targeting group and a linker, and the siRNA, the linker, and the targeting group are sequentially linked covalently or non-covalently;

preferably, in the siRNA conjugate, the sense strand and the antisense strand of the siRNA are complementary to form a double-stranded region of the siRNA conjugate, the 3' end of the sense strand forms a blunt end, and the 3' end of the antisense strand has 1-3 overhanging nucleotides extending out of the double-stranded region;
or,
in the siRNA conjugate, the sense strand and the antisense strand of the siRNA are complementary to form a double-stranded region of the siRNA conjugate, the 3' end of the sense strand forms a blunt end, and the 3' end of the antisense strand forms a blunt end.

**23.** The siRNA conjugate or the prodrug thereof according to claim 22, wherein the conjugated group is selected from any one of the groups consisting of formula (I) to formula (VIII):

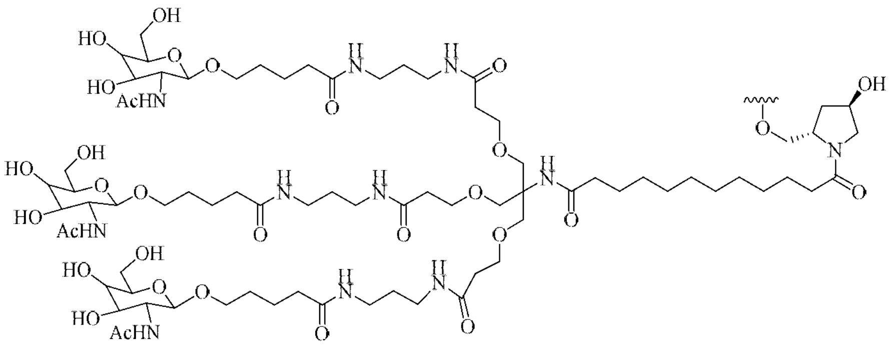

formula (I)

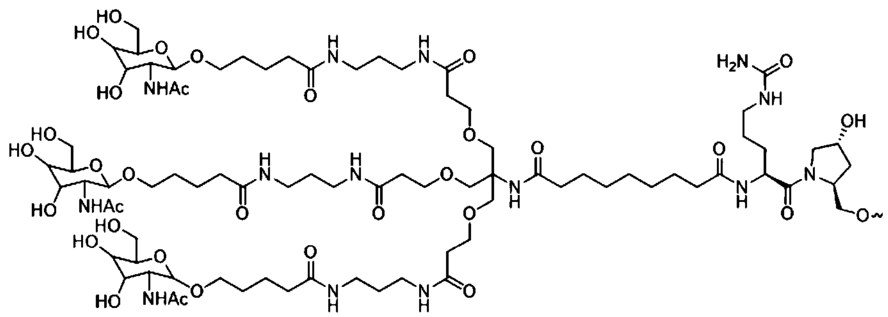

formula (II)

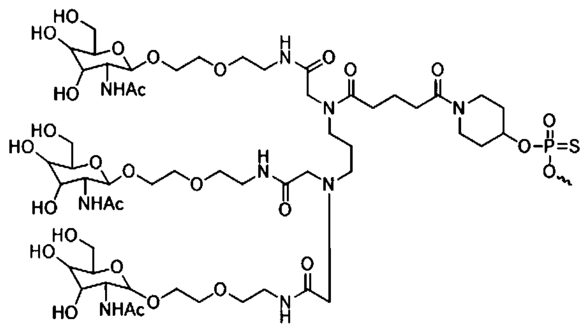

formula (III)

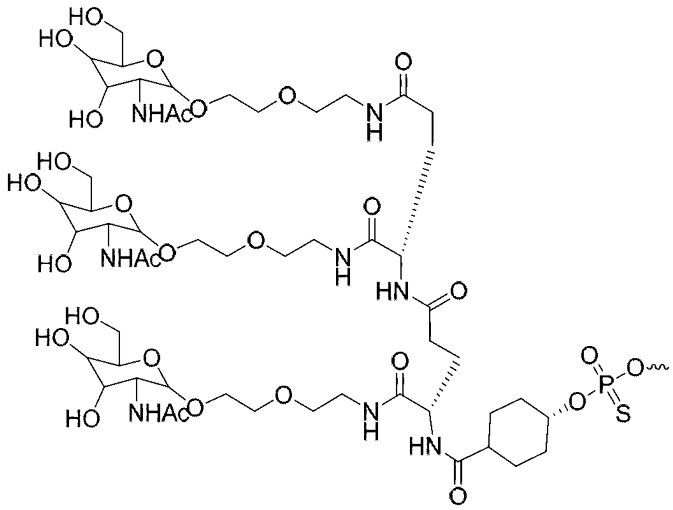

formula (IV)

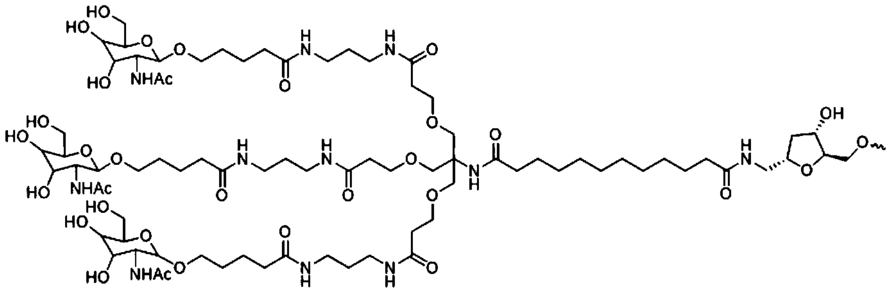

formula (V)

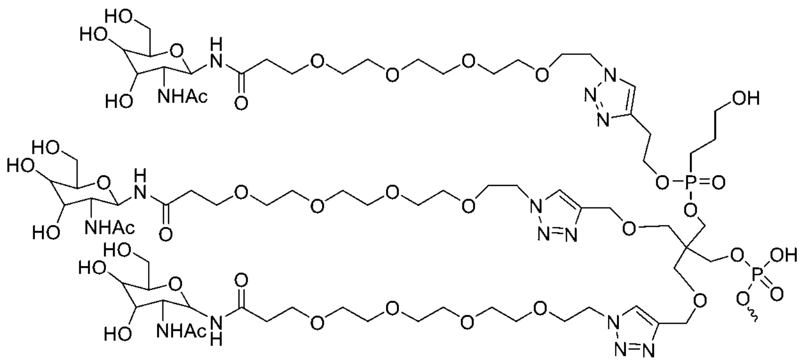

formula (VI)

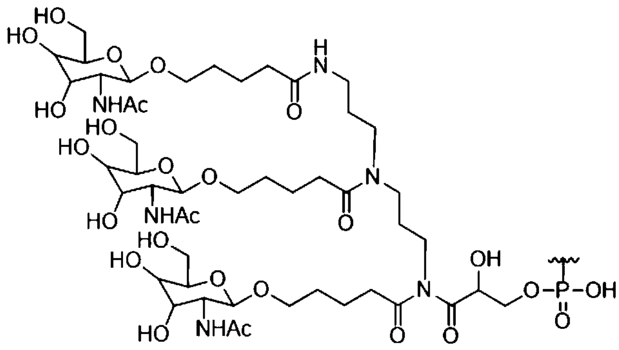

formula (VII)

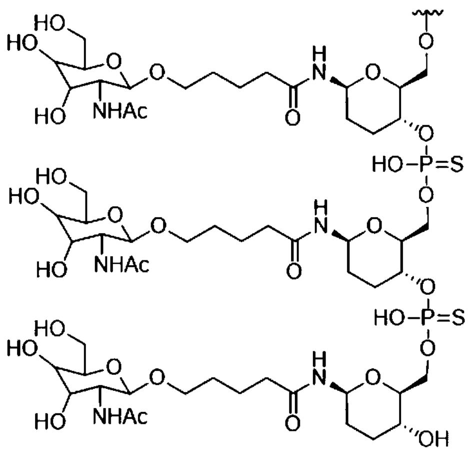

formula (VIII).

24. The siRNA conjugate or the prodrug thereof according to any one of claims 21-23, wherein the siRNA conjugate comprises or is selected from the group consisting of at least one of the siRNA conjugates in Table 2; preferably, the siRNA conjugate is selected from N-FY001003M2L96, N-FY001003M3L96, N-FY001003M6L96, N-FY001003M7L96, N-FY001003M8L96, N-FY001003M9L96, N-FY001003M10L96, N-FY001003M11L96, N-FY001003M12L96, N-FY001003M13L96, N-FY001003M14L96, N-FY001003M15L96, N-FY001003M16L96, N-FY001003M17L96, N-FY001003M18L96, N-FY001003M19L96, N-FY001003M20L96, N-FY001003M21L96, N-FY001003M22L96, N-FY001003M23L96, N-FY001003M24L96, N-FY001003M25L96, N-FY001003M26L96, N-FY001003M27L96, N-FY001003M28L96, N-FY001003M29L96, N-FY001003M30L96, N-FY001003M31L96, N-FY001003M32L96, N-FY001003M33L96, N-FY001003M34L96, N-FY001003M35L96, N-FY001003M37L96, N-FY001003M40L96, N-FY001003M44L96, N-FY001003M45L96, and N-FY001003M6L96B-1.

25. A pharmaceutical composition, comprising the siRNA according to any one of claims 1-20, or the siRNA conjugate or the prodrug thereof according to any one of claims 21-24, and a pharmaceutically acceptable carrier.

26. A kit, comprising the siRNA according to any one of claims 1-20, or the siRNA conjugate or the prodrug thereof according to any one of claims 21-24, or the pharmaceutical composition according to claim 25.

27. Use of the siRNA according to any one of claims 1-20, or the siRNA conjugate according to any one of claims 21-24, or the pharmaceutical composition or the prodrug thereof according to claim 25 in preparing a medicament for inhibiting DGAT2 gene expression.

28. Use of the siRNA according to any one of claims 1-20, or the siRNA conjugate according to any one of claims 21-24, or the pharmaceutical composition according to claim 25 in preparing a medicament for preventing and/or treating a disease associated with DGAT2 gene overexpression;
preferably, the disease is selected from the group consisting of: non-alcoholic fatty liver disease, non-alcoholic steatohepatitis, alcoholic fatty liver, obesity, hepatic steatosis, fatty liver, and hyperlipidemia.

**29.** A method for inhibiting DGAT2 gene expression, comprising contacting a therapeutically effective amount of the siRNA according to any one of claims 1-20, or the siRNA conjugate or the prodrug thereof according to any one of claims 21-24, or the pharmaceutical composition according to claim 25 with a cell expressing DGAT2; or administering to a subject in need a therapeutically effective amount of the siRNA according to any one of claims 1-20, or the siRNA conjugate or the prodrug thereof according to any one of claims 21-24, or the pharmaceutical composition according to claim 25.

**30.** A method for treating and/or preventing a disease associated with DGAT2 gene overexpression, comprising administering to a subject in need a therapeutically effective amount of the siRNA according to any one of claims 1-20, or the siRNA conjugate or the prodrug thereof according to any one of claims 21-24, or the pharmaceutical composition according to claim 25.

**INTERNATIONAL SEARCH REPORT**

International application No. **PCT/CN2024/110631**

**A. CLASSIFICATION OF SUBJECT MATTER**

C12N15/113(2010.01)i； A61K31/7088(2006.01)i； A61P1/16(2006.01)i； A61P3/00(2006.01)i

According to International Patent Classification (IPC) or to both national classification and IPC

**B. FIELDS SEARCHED**

Minimum documentation searched (classification system followed by classification symbols)

IPC： C12N; A61K; A61P

Documentation searched other than minimum documentation to the extent that such documents are included in the fields searched

Electronic data base consulted during the international search (name of data base and, where practicable, search terms used)

CNTXT, WPABSC, ENTXTC, ENTXT, USTXT, WOTXT, CJFD, VEN, CNKI, DWPI, 万方, WANFANG, 百度, Baidu, bing, PUBMED, ISI Web of Knowledge, NCBI GenBank, EBI-EMBL, STN, 读秀, DUXIU: SEQ ID Nos: 346-347, 抑制, DGAT2, 二酰甘油O-酰基转移酶, 表达, siRNA, dsRNA, RNAi, 缀合, 修饰, 乙酰半乳糖胺, GalNAc, 非酒精性脂肪性肝病, nash, inhibit, interfere, express, conjugat+, ligand, modif+.

**C. DOCUMENTS CONSIDERED TO BE RELEVANT**

| Category* | Citation of document, with indication, where appropriate, of the relevant passages | Relevant to claim No. |
|---|---|---|
| A | CN 116171160 A (DICERNA PHARMACEUTICALS, INC.) 26 May 2023 (2023-05-26) claims 1-35, and description, paragraphs 6-7 | 1-30 |
| A | US 2022228141 A1 (UNIVERSITY OF MASSACHUSETTS) 21 July 2022 (2022-07-21) claims 1-3, 6, 20, 32, 37-38, 69, 72-73 and 144 | 1-30 |
| A | CN 109477106 A (IONIS PHARMACEUTICALS, INC.) 15 March 2019 (2019-03-15) entire document | 1-30 |
| A | CN 110520531 A (SILENCE THERAPEUTICS GMBH) 29 November 2019 (2019-11-29) entire document | 1-30 |
| A | WO 2023041508 A2 (ARGONAUTE RNA LIMITED) 23 March 2023 (2023-03-23) entire document | 1-30 |
| A | YENILMEZ, B. et al. "An RNAi Therapeutic Targeting Hepatic DGAT2 in a Genetically Obese Mouse Model of Nonalcoholic Steatohepatitis" *Molecular Therapy*, Vol. 30, No. (3), 31 March 2022 (2022-03-31), pp. 1329-1342 | 1-30 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 November 2024** | **20 November 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/110631**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29 (in part), 30**
because they relate to subject matter not required to be searched by this Authority, namely:

The subject matter of claims 29 (the part involving inhibiting the expression of a DGAT2 gene in a subject) and 30 relates to a method for treating a human or animal body, and therefore no international search is performed according to PCT Rule 39.1(iv). The search is carried out on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Claims 1-30 relate to seven inventions:

Invention I: claims 1-30 (in part) relate to an siRNA for inhibiting DGAT2 gene expression, wherein the siRNA contains a sense strand and an antisense strand, the sense strand contains nucleotide sequence I, the antisense strand contains nucleotide sequence II, the nucleotide sequence I contains a nucleotide sequence as shown in SEQ ID NO: 346, the nucleotide sequence II contains a nucleotide sequence as shown in SEQ ID NO: 347, each nucleotide in the siRNA is independently a modified or unmodified nucleotide, and the nucleotide sequence I and the nucleotide sequence II are at least partially reversely complementary to form a double-stranded region; and a corresponding siRNA conjugate or a prodrug thereof, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

Invention II: claims 1-30 (in part) relate to an siRNA for inhibiting DGAT2 gene expression, wherein the siRNA contains a sense strand and an antisense strand, the sense strand contains nucleotide sequence I, the antisense strand contains nucleotide sequence II, the nucleotide sequence I contains a nucleotide sequence as shown in SEQ ID NO: 348, the nucleotide sequence II contains a nucleotide sequence as shown in SEQ ID NO: 349, each nucleotide in the siRNA is independently a modified or unmodified nucleotide, and the nucleotide sequence I and the nucleotide sequence II are at least partially reversely complementary to form a double-stranded region; and a corresponding siRNA conjugate or a prodrug thereof, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

Invention III: claims 1-30 (in part) relate to an siRNA for inhibiting DGAT2 gene expression, wherein the siRNA contains a sense strand and an antisense strand, the sense strand contains nucleotide sequence I, the antisense strand contains nucleotide sequence II, the nucleotide sequence I contains a nucleotide sequence as shown in SEQ ID NO: 350, the nucleotide sequence II contains a nucleotide sequence as shown in SEQ ID NO: 351, each nucleotide in the siRNA is independently a modified or unmodified nucleotide, and the nucleotide sequence I and the nucleotide sequence II are at least partially reversely complementary to form a double-stranded region; and a corresponding siRNA conjugate or a prodrug thereof, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

INTERNATIONAL SEARCH REPORT

International application No. PCT/CN2024/110631

**Box No. III Observations where unity of invention is lacking (Continuation of item 3 of first sheet)**

Invention IV: claims 1-30 (in part) relate to an siRNA for inhibiting DGAT2 gene expression, wherein the siRNA contains a sense strand and an antisense strand, the sense strand contains nucleotide sequence I, the antisense strand contains nucleotide sequence II, the nucleotide sequence I contains a nucleotide sequence as shown in SEQ ID NO: 352, the nucleotide sequence II contains a nucleotide sequence as shown in SEQ ID NO: 353, each nucleotide in the siRNA is independently a modified or unmodified nucleotide, and the nucleotide sequence I and the nucleotide sequence II are at least partially reversely complementary to form a double-stranded region; and a corresponding siRNA conjugate or a prodrug thereof, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

Invention V: claims 1-30 (in part) relate to an siRNA for inhibiting DGAT2 gene expression, wherein the siRNA contains a sense strand and an antisense strand, the sense strand contains nucleotide sequence I, the antisense strand contains nucleotide sequence II, the nucleotide sequence I contains a nucleotide sequence as shown in SEQ ID NO: 354, the nucleotide sequence II contains a nucleotide sequence as shown in SEQ ID NO: 355, each nucleotide in the siRNA is independently a modified or unmodified nucleotide, and the nucleotide sequence I and the nucleotide sequence II are at least partially reversely complementary to form a double-stranded region; and a corresponding siRNA conjugate or a prodrug thereof, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

Invention VI: claims 1-30 (in part) relate to an siRNA for inhibiting DGAT2 gene expression, wherein the siRNA contains a sense strand and an antisense strand, the sense strand contains nucleotide sequence I, the antisense strand contains nucleotide sequence II, the nucleotide sequence I contains a nucleotide sequence as shown in SEQ ID NO: 356, the nucleotide sequence II contains a nucleotide sequence as shown in SEQ ID NO: 357, each nucleotide in the siRNA is independently a modified or unmodified nucleotide, and the nucleotide sequence I and the nucleotide sequence II are at least partially reversely complementary to form a double-stranded region; and a corresponding siRNA conjugate or a prodrug thereof, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

Invention VII: claims 1-30 (in part) relate to an siRNA for inhibiting DGAT2 gene expression, wherein the siRNA contains a sense strand and an antisense strand, the sense strand contains nucleotide sequence I, the antisense strand contains nucleotide sequence II, the nucleotide sequence I contains a nucleotide sequence as shown in SEQ ID NO: 358, the nucleotide sequence II contains a nucleotide sequence as shown in SEQ ID NO: 359, each nucleotide in the siRNA is independently a modified or unmodified nucleotide, and the nucleotide sequence I and the nucleotide sequence II are at least partially reversely complementary to form a double-stranded region; and a corresponding siRNA conjugate or a prodrug thereof, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

The same or corresponding technical features of inventions I-VII described above are: an siRNA for inhibiting DGAT2 gene expression, a sense strand structure and antisense strand structure, modification and complementation thereof, an siRNA conjugate or a prodrug thereof corresponding to the siRNA, a pharmaceutical composition, a kit, the use in the preparation of a medicament for inhibiting DGAT2 gene expression, the use in the preparation of a medicament for preventing and/or treating diseases related to DGAT2 gene overexpression, a method for inhibiting DGAT2 gene expression, and a method for treating and/or preventing diseases related to DGAT2 gene overexpression.

D1 (CN 116171160 A, publication date: 26 May 2023) discloses the above technical features: an siRNA for targeted inhibition of DGAT2 gene expression, which contains an antisense strand and a sense strand that have a complementary region, wherein an siRNA sequence includes modification; and further relating to an siRNA conjugate, a pharmaceutical composition or a kit and a preparation method therefor, and a method for inhibiting DGAT2 expression and treating a metabolic liver disease, a non-alcoholic fatty liver disease (NAFLD), non-alcoholic steatohepatitis (NASH), etc. (see D1, claims 1-35). Therefore, the same or corresponding technical features of inventions I-VII described above are not special technical features as defined in PCT Rule 13.2. Therefore, inventions I-VII described above lack unity of invention (PCT Rule 13.1).

Form PCT/ISA/210 (continuation of first sheet) (July 2022)

INTERNATIONAL SEARCH REPORT

International application No.
**PCT/CN2024/110631**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-30 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/110631**

| Patent document cited in search report | Publication date (day/month/year) | Patent family member(s) | Publication date (day/month/year) |
|---|---|---|---|
| CN 116171160 A | 26 May 2023 | EP 4192953 A2 | 14 June 2023 |
| | | IL 300299 A | 01 April 2023 |
| | | TW 202221120 A | 01 June 2022 |
| | | CA 3190594 A1 | 10 February 2022 |
| | | CL 2023000367 A1 | 06 October 2023 |
| | | US 2023340487 A1 | 26 October 2023 |
| | | AU 2021320219 A1 | 02 March 2023 |
| | | JP 2023538283 A | 07 September 2023 |
| | | KR 20230047453 A | 07 April 2023 |
| | | MX 2023001451 A | 23 March 2023 |
| | | WO 2022031850 A2 | 10 February 2022 |
| | | WO 2022031850 A3 | 14 April 2022 |
| | | BR 112023001820 A2 | 28 March 2023 |
| US 2022228141 A1 | 21 July 2022 | AU 2021382621 A1 | 22 June 2023 |
| | | AU 2021382621 A9 | 08 February 2024 |
| | | EP 4247391 A1 | 27 September 2023 |
| | | WO 2022109398 A1 | 27 May 2022 |
| | | WO 2022109398 A9 | 29 September 2022 |
| | | JP 2023550485 A | 01 December 2023 |
| CN 109477106 A | 15 March 2019 | CL 2018000070 A1 | 27 July 2018 |
| | | AU 2016294347 A1 | 04 January 2018 |
| | | AU 2016294347 B2 | 28 July 2022 |
| | | BR 112017028194 A2 | 06 November 2018 |
| | | BR 112017028194 B1 | 14 March 2023 |
| | | MX 2018000412 A | 14 March 2018 |
| | | NZ 738254 A | 30 August 2024 |
| | | US 2020040341 A1 | 06 February 2020 |
| | | US 11312962 B2 | 26 April 2022 |
| | | MY 192997 A | 20 September 2022 |
| | | JP 2022106727 A | 20 July 2022 |
| | | KR 20180027522 A | 14 March 2018 |
| | | KR 102640776 B1 | 23 February 2024 |
| | | JP 2024084827 A | 25 June 2024 |
| | | DK 3320094 T3 | 23 May 2022 |
| | | ZA 201708453 B | 30 August 2023 |
| | | WO 2017011276 A1 | 19 January 2017 |
| | | JP 2018525030 A | 06 September 2018 |
| | | JP 7054672 B2 | 14 April 2022 |
| | | IL 256622 A | 28 February 2018 |
| | | IL 256622 B1 | 01 May 2023 |
| | | IL 256622 B2 | 01 September 2023 |
| | | ES 2917181 T3 | 07 July 2022 |
| | | EP 3320094 A1 | 16 May 2018 |
| | | EP 3320094 A4 | 10 April 2019 |
| | | EP 3320094 B1 | 13 April 2022 |
| | | US 2023047452 A1 | 16 February 2023 |
| | | SG 10202006563 PA | 28 August 2020 |
| | | PE 20180800 A1 | 09 May 2018 |
| | | US 2018312845 A1 | 01 November 2018 |
| | | CA 2991894 A1 | 19 January 2017 |

Form PCT/ISA/210 (patent family annex) (July 2022)

**INTERNATIONAL SEARCH REPORT**
**Information on patent family members**

International application No.
**PCT/CN2024/110631**

| Patent document cited in search report | Publication date (day/month/year) | Patent family member(s) | Publication date (day/month/year) |
|---|---|---|---|
| | | CO 2017013065 A2 | 28 March 2018 |
| | | EP 4092119 A2 | 23 November 2022 |
| | | EP 4092119 A3 | 22 March 2023 |
| CN 110520531 A | 29 November 2019 | US 2022090067 A1 | 24 March 2022 |
| | | US 12084656 B2 | 10 September 2024 |
| | | US 2020095580 A1 | 26 March 2020 |
| | | US 11015198 B2 | 25 May 2021 |
| | | AU 2018247925 A1 | 03 October 2019 |
| | | AU 2018247925 B2 | 06 June 2024 |
| | | SI 3607069 T1 | 28 February 2023 |
| | | JP 2022173554 A | 18 November 2022 |
| | | RS 63836 B1 | 31 January 2023 |
| | | CA 3057565 A1 | 11 October 2018 |
| | | EP 4219713 A2 | 02 August 2023 |
| | | EP 4219713 A3 | 16 August 2023 |
| | | HRP 20221400 T1 | 06 January 2023 |
| | | LT 3607069 T | 10 January 2023 |
| | | EP 3607069 A1 | 12 February 2020 |
| | | EP 3607069 B1 | 02 November 2022 |
| | | DK 3607069 T3 | 21 November 2022 |
| | | ES 2932831 T3 | 26 January 2023 |
| | | PL 3607069 T3 | 06 March 2023 |
| | | JP 2020516312 A | 11 June 2020 |
| | | JP 7155239 B2 | 18 October 2022 |
| | | IL 268828 A | 31 October 2019 |
| | | HUE 061247 T2 | 28 June 2023 |
| | | WO 2018185241 A1 | 11 October 2018 |
| | | FI 3607069 T3 | 13 January 2023 |
| WO 2023041508 A2 | 23 March 2023 | CA 3229020 A1 | 23 March 2023 |
| | | WO 2023041508 A3 | 08 June 2023 |
| | | JP 2024531728 A | 29 August 2024 |
| | | EP 4402263 A2 | 24 July 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102140458 B
- WO 2009082607 A2
- CN 105378082 A
- WO 2015006740 A2
- WO 2022031850 A2


### Non-patent literature cited in the description


- **AMIN N B** ; **CARVAJAL-GONZALEZ S** ; **PURKAL J et al.** Targeting diacylglycerol acyltransferase 2 for the treatment of nonalcoholic steatohepatitis [J. *Sci. Transl. Med*, 2019, vol. 11 (520), eaav9701
- **PRL A** ; **EMB B** ; **LW B et al.** *Novel antisense inhibition of diacylglycerol O-acyltransferase 2 for treatment of non-alcoholic fatty liver disease: a multicentre, double-blind, randomised, placebo-controlled phase 2 trial [J*, 2020
- **BATUHAN YENILMEZ et al.** An RNAi therapeutic targeting hepatic DGAT2 in a genetically obese mouse model of nonalcoholic steatohepatitis. *Mol Ther.*, 02 March 2022, vol. 30 (3), 1329-1342
- **XING XIAN YU et al.** Antisense oligonucleotide reduction of DGAT2 expression improves hepatic steatosis and hyperlipidemia in bbese mice. *Hepatology*, August 2005, vol. 42 (2), 362-71
- **BRANDON PABST et al.** Mechanistic characterization of long residence time inhibitors of diacylglycerol acyltransferase 2 (DGAT2). *Biochemistry*, 2018, vol. 57 (51), 6997-7010
- **TINGLU NING et al.** Genetic interaction of DGAT2 and FAAH in the development of human obesity. *Endocrine*, 2017, vol. 56, 366-378
- **J.K. WATTS** ; **G.F. DELEAVEY** ; **M.J. DAMHA**. Chemically modified siRNA: tools and applications. *Drug Discov Today*, 2008, vol. 13 (19-20), 842-55
- *ACS Chemical biology*, 2015, vol. 10 (5), 1181-7
- **SAMBROOK et al.** Molecular Cloning: A Laboratory Manual. Cold Spring Harbor Laboratory Press, 1989